# EUROPEAN PATENT SPECIFICATION

(11) **EP 1 891 215 B1**
(45) Date of publication and mention of the grant of the patent: **28.10.2009**
(21) Application number: 06732451.7
(22) Date of filing: 20.04.2006
(51) Int. Cl.: C12N 15/09

(54) **HIGHLY SAFE INTRANASALLY ADMINISTRABLE GENE VACCINES FOR TREATING ALZHEIMER'S DISEASE**
HOCHSICHERE INTRANASAL VERABREICHBARE GENIMPFSTOFFE ZUR BEHANDLUNG VON MORBUS ALZHEIMER
VACCINS GENIQUES SANS DANGER ADMINISTRABLES PAR VOIE INTRANASALE POUR LE TRAITEMENT DE LA MALADIE D'ALZHEIMER

(30) Priority: 20.04.2005 JP 2005123015; 18.10.2005 US 727690 P
(43) Date of publication of application: 27.02.2008
(73) Proprietor: Dnavec Corporation, Tsukuba-shi, Ibaraki 305-0856 (JP); Japan as Represented by President of National Center for Geriatrics and Gerontology, Aichi 4748511 (JP)
(72) Inventor: HARA, Hideo c/o National Center for Geriatrics & Gerontology, Obu-shi, Aichi 474-8511 (JP); TABIRA, Takeshi c/o National Center for Geriatrics & Gerontology, Obu-shi, Aichi 474-8511 (JP); YONEMITSU, Yoshikazu, Fukuoka-shi, Fukuoka, 8130043 (JP); INOUE, Makoto c/o DNAVEC CORPORATION, Tsukuba-shi, Ibaraki 305-0856 (JP); TOKUSUMI, Yumiko , c/o DNAVEC CORPORATION, Tsukuba-shi, Ibaraki 305-0856 (JP); HASEGAWA, Mamoru c/o DNAVEC CORPORATION, Tsukuba-shi, Ibaraki 305-0856 (JP)
(74) Representative: Vossius & Partner
(86) International application number: PCT/JP2006/308913
(87) International publication number: WO 2006/112553

(56) References cited:
- EP-A- 1 270 016
- WO-A-99/27944
- WO-A-2004/111250
- FURLAN ROBERTO ET AL: "Vaccination with amyloid-beta peptide induces autoimmune encephalomyelitis in C57/BL6 mice." BRAIN, vol. 126, no. 2, February 2003 (2003-02), pages 285-291, XP002402349 ISSN: 0006-8950
- HARA HIDEO ET AL: "Development of a safe oral Abeta vaccine using recombinant adeno-associated virus vector for Alzheimer's disease" JOURNAL OF ALZHEIMER'S DISEASE, vol. 6, no. 5, October 2004 (2004-10), pages 483-488, XP009073576 ISSN: 1387-2877
- NATH A ET AL: "AMYLOID BETA - PEPTIDE AUTOANTIBODIES ARE INCREASED IN ALZHEIMERS DISEASE AND ENHANCE PEPTIDE NEUROTOXICITY: IMPLICATIONS FOR DISEASE PATHOGENESIS AND VACCINE DEVELOPMENT." SOCIETY FOR NEUROSCIENCE ABSTRACT VIEWER AND ITINERARY PLANNER, vol. 2002, 2002, pages Abstract No. 686.17 URL-http://sf, XP008056287 & 32ND ANNUAL MEETING OF THE SOCIETY FOR NEUROSCIENCE; ORLANDO, FLORIDA, USA; NOVEMBER 02-07, 2002
- HANDISURYA A ET AL: "Papillomavirus-like particle amyloid beta vaccine induces strong antibody response" JOURNAL OF INVESTIGATIVE DERMATOLOGY, vol. 122, no. 3, March 2004 (2004-03), page A135,A134, XP009073547 & 65TH ANNUAL MEETING OF THE SOCIETY-FOR-INVESTIGATIVE-DERMATOLOGY; PROVIDENCE, RI, USA; APRIL 28 -MAY 01, 2004 ISSN: 0022-202X
- "0739 Mucosal immunotherapy for Alzheimer's disease by nasal administration of recombinant sendaivirus vector expressing Abeta 1-43/IL-10" JOURNAL OF NEUROLOGICAL SCIENCES, ELSEVIER SCIENTIFIC PUBLISHING CO, AMSTERDAM, NL, vol. 238, 2005, pages S288-S289, XP005546928 ISSN: 0022-510X

## Description

### TECHNICAL FIELD

The present invention relates to vaccines based on minus strand RNA virus vectors for treating Alzheimer's disease.

### BACKGROUND ART

In the rapidly aging society of Japan, senile dementia is becoming a serious social problem including the problem of nursing those affected. Indeed, it has been reported that approximately 10% of the people aged 65 years or older in Japan have senile dementia. While Alzheimer's disease, one of the two major causes of dementia, accounts for approximately 50% of the affected population, there is no effective therapeutic method available to date.

Pathological findings of Alzheimer's disease have three characteristics: neuronal atrophy/loss; senile plaque formation due to aggregation and deposition of amyloid β (hereinafter, sometimes also abbreviated as "Aβ") proteins; and neurofibrillary tangles due to abnormal Tau protein. The major amyloid β proteins generated in the brain of Alzheimer's disease patients are of 40 to 43 amino acids resulting from cleavage of amyloid precursor proteins by β and γ secretases. Senile plaque is an aggregation composed of central amyloid β and surrounding microglia, fibrous astroglia, and dystrophic neurites. At present, the most convincing pathogenic hypothesis for Alzheimer's disease is "amyloid cascade hypothesis", suggesting the cause of the disease to be senile plaque formation by amyloid β aggregation and deposition.

Vaccine (immune) therapy based on this hypothesis is attracting attention as a novel therapeutic method for Alzheimer's disease. Vaccine therapy for Alzheimer's disease is a method of eliminating amyloid β from the brain by immunological techniques. Schenk *et al.* of Elan Corporation intramuscularly administered pre-aggregated Aβ42 in combination with an adjuvant to PDAPP-transgenic mice and confirmed a decrease of amyloid deposition in the brain (Patent Document 1 and Non-patent Document 1). In the clinical trials conducted by Elan Corporation and Wyeth Corporate, a synthetic Aβ42 peptide (AN-1792) was injected intramuscularly in combination with an adjuvant (QS21), and anti-Aβ antibody recognizing β sheet structure of amyloid was detected in the serum of the subjects (Non-patent Document 2). Improvements of higher brain functions were also reported (Non-patent Document 3). Unfortunately the Phase II clinical trial was discontinued because 6% of the patients (18 of 298 patients) developed meningoencephalitis as an adverse effect, and one death was reported. Post-mortem pathological examination of the brain tissue of the patient confirmed absence of senile plaques in the neocortex, suggesting the effectiveness of the vaccine. Figures showing microglial phagocytosis of Aβ degradation products were observed in the regions where senile plaques had disappeared. This finding suggests that the microglia phagocytoses antibody bound to Aβ *via* Fc receptor.

The anti-Aβ antibody described above has been found to be non-reactive to neurons, glial cells, amyloid precursor proteins (APPs), and intracellular Aβ. Therefore, it is unlikely that the adverse effect described above results from brain inflammation caused by the antibody. To administer AN-1792 vaccine, an adjuvant is required. Adjuvant has a strong activation effect on immunity and induces T lymphocyte-mediated cellular immunity. It is possible that the adverse effect described above is an experimental allergic encephalomyelitis-like meningoencephalitis, which is caused by the infiltration of Aβ- or APP-reactive Th1 type CD4⁺ T cells in the brain due to adjuvant-induced cellular immunity (Non-patent Document 4).

Thus the pathological effect of a vaccine therapy using an Aβ peptide has been confirmed with AN-1792 vaccine. However, for the clinical application of vaccines, reduction of adverse effects such as meningoencephalitis is essential. Based on this view, improved vaccination methods have been devised, including an immunization method that involves the combined use of an Aβ peptide N-terminal fragment linked to a carrier protein recognizable by T cells and a Th2 adjuvant (Patent Document 2), and an adenoviral vector that comprises a nucleic acid encoding a fused protein consisted of an Aβ peptide and cholera toxin B subunit (Patent Document 3). Furthermore, the present inventors developed a highly safe oral vaccine, an adeno-associated viral vector comprising a DNA that encodes a peptide fragment of an Aβ peptide to induce humoral immunity (Patent Document 4). The above-mentioned adeno-associated viral vector vaccine does not require any adjuvant because it utilizes the intestinal mucosal immune system where Th2 T cells can be easily induced. In addition, such excellent characteristics have been confirmed by the experiments using APP transgenic mice that a relatively long lasting antigen presentation as 6 months in the intestinal tract is available with a single administration; it has an effect of reducing brain amyloid deposition and senile plaque formation; there is absence of any observable inflammation in other organs, and so on. Nevertheless, vaccines with even better safety and effectiveness are needed for the practical application of vaccine therapy for Alzheimer's disease.
Patent Document 1: WO 99/27944
Patent Document 2: WO 02/096350
Patent Document 3: WO 2004/050876
Patent Document 4: WO 2004/111250
Patent Document 5: WO 00/70070
Patent Document 6: Japanese Patent Application Kokai Publication No. (JP-A) 2000-253876
Patent Document 7: WO 2001/072340
Non-Patent Document 1: Schenk D. et al, Nature 400: 173-177, 1999
Non-Patent Document 2: Hock C. et al., Nat. Med. 8: 1270-1275, 2002
Non-Patent Document 3: Hock C. et al., Neuron 38, 547-554, 2003
Non-Patent Document 4: Clinical Research (Rinsho to Kenkyu) 82(3): 439-444, 2005

### DISCLOSURE OF THE INVENTION

The present invention was achieved in view of the circumstances described above. An objective of the present invention is to provide highly safe and effective vaccine therapies for Alzheimer's disease.

In order to achieve the above objective, the present inventors conducted dedicated research aiming at development of novel vaccine therapies for Alzheimer's disease. The inventors are experienced in the development of vectors for gene introduction and applicable to gene therapy, using *Sendai virus* (SeV). *Sendai virus* is a minus (-) strand RNA virus. The virus is not integrated into chromosomes because it proliferates only in the host cell cytoplasm and does not have a DNA phase in their life cycle. The virus therefore ensures a high level of genetic safety when used as a vector for gene introduction. In developing *Sendai virus*-based vectors for gene introduction, the present inventors deleted gene(s) from the *Sendai virus* genome to further improve the safety and make the vectors suitable for target diseases. The inventors succeeded in improvement of the vectors to be safer and incapable of causing secondary infections by deleting the gene of a membrane fusion protein (F protein), which is involved in the viral invasion into host cells, from the genome and in establishing an efficient viral vector reconstruction system (WO 00/70070). In addition, the inventors successfully developed influenza vaccines (Japanese Patent Application Kokai Publication No. (JP-A) 2000-253876) and AIDS vaccines (WO 2001/072340) using the *Sendai virus* vector.

Th2 cytokines, such as IL-10, play important roles in Th1/Th2 balance closely associated with immune responses in the body. Th1 cells produce IFN-γ and such, and enhance cellular immunity. IFN-γ produced by Th1 cells suppresses IL-10 production in Th2 cells. Meanwhile, Th2 cells produce IL-10 and such, and enhance humoral immunity. IL-10 produced by Th2 cells suppresses the production of IFN-γ and such in The cells. In an attempt to produce vaccines for Alzheimer's disease that would suppress cellular immunity-mediated adverse effects, such as encephalomeningitis, the inventors thought to introduce a gene encoding a Th2 cytokine together with Aβ into the above-mentioned *Sendai virus* vector. The inventors then constructed a *Sendai virus* vector encoding Aβ-43 and IL-10 and tested its effects. First, the vector was administered intranasally to 24- to 25-month old APP transgenic mice. Anti-Aβ42 antibody levels in the serum were determined 4 weeks and 8 weeks after the treatment. Only low levels of anti-Aβ antibody were detected in the control group mice and the levels increased with time. In contrast, in the group of mice administered with the vector of the present invention, high levels of anti-Aβ antibody were detected in the blood, and the antibody levels were found to be slightly lower at 8 weeks than at 4 weeks. Histological examination showed that senile plaques in the frontal lobe; parietal lobe, and hippocampus were all markedly reduced upon the administration of the vector of the present invention. Further, the amount of Aβ in the brain tissue was determined with ELISA. The result showed a significant decrease of Aβ in the brain tissue. In addition, CD3 and Iba-1 were used as indices to examine whether the administration of this vector caused lymphocyte infiltration in the central nervous system. The result indicated that neither lymphocyte infiltration nor abnormal (inordinate) microglial activation occurred.

The above study revealed excellent characteristics of a minus strand RNA viral vector expressing an Aβ peptide. First, this vector exhibited a significant effect in reducing Aβ in Alzheimer's disease model mice with extremely old age. The Tg2576 mice used in the experiment described above were 24 to 25 months old and nearly at the end of their lives. In the studies conducted so far to assess treatment methods for Alzheimer's disease, mice with age of up to, at the most, 18 months were used. This is the first time that a therapeutic effect was confirmed in mice with such an old age. Such old-aged mice have an enormous amount of Aβ deposited in the brain. Thus, the vector of the present invention can be highly effective for patients with more advanced Alzheimer's disease. Second, the vector of the present invention was effective at a lower dosage and frequency of administration compared to conventional vaccine therapies. For example, when conventional methods using an Aβ peptide and an adjuvant in combination are adopted, multiple-dose administration is generally required to establish the immunity. Indeed, administration was carried out several times to around a dozen times in the clinical trials ofAN-1792. In contrast, the vector of the present invention demonstrated significant effectiveness after a single-dose administration. Furthermore, in a vaccine therapy using an adeno-associated viral vector (WO 2004/111250), vaccination was carried out by the same single dose administration procedure used in the Examples described herein, and the dosage was 5x 10¹¹ genomes/body. In contrast, the dosage applied in the Examples described herein was 5x 10⁶ CIU (Cell Infectious Unit)/body (about 5x 10⁷ genomes/body when converted to genome copy number). The dosages of these two vectors differ by as much as about 10⁴. Third, the vector administration in the Examples did not cause meningoencephalitis in the mouse model. Meningoencephalitis observed in the AN-1792 clinical trial was reportedly found in a mouse model as well (M. Shoji, et al., 44th Annual Meeting of Japanese Society of Neurology, May 15-17, 2003). Development of meningoencephalitis in normal non-model mice (C57B6 mice) immunized with an Aβ peptide and an adjuvant has also been reported (Furlan R, et al., Vaccination with amyloid-beta peptide induces autoimmune encephalomyelitis in C57/BL6 mice, Brain 126:285-291, 2003). The results described herein suggest that the vectors of the present invention are also safe when administered to humans.

As described above, the vector of the present invention exhibited a high Aβ-eliminating effect at a low dosage and frequency of administration. The effect of a *Sendai virus* vector-based AIDS vaccine was confirmed to be partly due to cellular immunity (WO 2001/072340). Although the vector used in the Examples was designed to purposely depend on humoral immunity rather than on cellular immunity to prevent adverse effects, the excellent vaccine effect demonstrated herein implies the importance of humoral immunity in vaccine therapies for Alzheimer's disease. Adverse effects can be reduced with making humoral immunity dominant through the use of a Th2 cytokine or Th2 adjuvant. That is, the present invention provides minus strand RNA viral vectors encoding Aβ and uses thereof, in particular, the inventions described below:
[1] A minus strand RNA viral vector comprising an amyloid β-encoding nucleic acid.
[2] The minus strand RNA viral vector of [1], further comprising a nucleic acid encoding a Th2 cytokine or a partial peptide thereof.
[3] The minus strand RNA viral vector of [2], wherein the Th2 cytokine or a partial peptide thereof is IL-10 or a partial peptide thereof.
[4] The vector of any one of [1] to [3], wherein the minus strand RNA viral vector is a Paramyxoviral vector.
[5] The vector of [4], wherein the Paramyxoviral vector is a *Sendai virus* vector.
[6] The vector of any one of [1] to [5] wherein the amyloid β is Aβ43 or a partial peptide thereof.
[7] The vector of any one of [1] to [6] wherein the amyloid β is derived from human.
[8] The vector of any one of [3] to [7] wherein IL-10 is derived from mouse or human.
[9] The vector of any one of [1] to [8] wherein the amyloid β is a polypeptide encoded by the nucleotide sequence of SEQ ID NO: 1, or a portion thereof.
[10] The vector of any one of [3] to [9] wherein IL-10 is a polypeptide encoded by the nucleotide sequence of SEQ ID NO: 2 or 3.
[11] The minus strand RNA viral vector of any one of [1] to [10] which comprises the nucleic acid of SEQ ID NO: 5.
[12] A composition which comprises the vector of any one of [1] to [11] and a pharmaceutically acceptable carrier.
[13] The composition of [12], further comprising (i) a nucleic acid encoding a Th2 cytokine or a partial peptide thereof in an expressible manner, (ii) a Th2 cytokine or a partial peptide thereof, or (iii) a Th2 adjuvant.
[14] The composition of [13], comprising a Th2 cytokine, a partial peptide thereof, or a Th2 adjuvant.
[15] The composition of [13], comprising a nucleic acid encoding a Th2 cytokine or a partial peptide thereof in an expressible manner.
[16] The composition of [15], wherein the Th2 cytokine is encoded by the minus strand RNA viral vector comprising an amyloid β-encoding nucleic acid.
[17] The composition of [15], wherein the Th2 cytokine is encoded by a vector other than the minus strand RNA viral vector comprising an amyloid β-encoding nucleic acid.
[18] The composition of any one of [12] to [17], wherein the composition is a pharmaceutical composition.
[19] A pharmaceutical composition for use in treating or preventing Alzheimer's disease, wherein the composition comprises the vector of any one of [1] to [18].
[20] The composition of [19], further comprising (i) a nucleic acid encoding a Th2 cytokine or a partial peptide thereof in an expressible manner, (ii) a Th2 cytokine or a partial peptide thereof, or (iii) a Th2 adjuvant.
[21] The pharmaceutical composition of any one of [18] or [20], for use in intranasal administration.
[22] A method for reducing deposition of amyloid β, wherein the method comprises a step of administering the vector of any one of [1] to [11] or a composition comprising the vector.
[23] A method for treating or preventing Alzheimer's disease, wherein the method comprises a step of administering the vector of any one of [1] to [11] or a composition comprising the vector.
[24] The method of [22] or [23], wherein the administration is intranasal administration.
[25] The method of [22] or [23], wherein the administration is intramuscular injection.
[26] The method of any one of [22] to [25], further a step of administering a Th2 cytokine, a partial peptide thereof, a Th2 adjuvant, or a vector encoding a Th2 cytokine or a partial peptide thereof.
[27] The method of [26], wherein a Th2 cytokine, a partial peptide thereof, or a Th2 adjuvant is administered.
[28] The composition of [26], wherein a vector encoding a Th2 cytokine or a partial peptide thereof is administered.

The present invention provides novel minus strand RNA viral vectors that can effectively reduce the level of amyloid β deposition at a low dosage and frequency of administration. The vector described in the Examples herein was confirmed not to induce inflammation in the central nervous system, and thus it is very unlikely that the administration of the vectors of the present invention will cause such encephalomeningitis as caused when conventional vaccines are administered. The vectors of the present invention can be used as extremely effective means for treating Alzheimer's disease.

The present invention relates to minus strand RNA viral vectors comprising a nucleic acid encoding amyloid β.

Herein, a gene refers to a genetic material or nucleic acid encoding a transcriptional unit. The gene may be an RNA or DNA. Herein, a nucleic acid encoding a protein is referred to as the protein's gene. The gene may not encode a protein and, for example, may encode a functional RNA, such as a ribozyme or an antisense RNA. The gene may be a naturally occurring or artificially designed sequence. Herein, "DNA" includes single-stranded and double-stranded DNAs. The phrase "encoding a protein" means that a polynucleotide comprises an ORF encoding the amino acid sequence of a protein either in a sense or antisense direction so as to express the protein under appropriate conditions.

Minus strand RNA viruses refer to viruses comprising minus strand RNAs (antisense strands complementary to the viral protein-encoding sense strands) as the genome. Minus strand RNA viruses are also called as negative strand RNA viruses. In particular, the minus strand RNA virus preferably used in the present invention is a single-stranded minus strand RNA virus (also referred to as a non-segmented minus strand RNA virus). The "single-stranded minus strand RNA virus" refers to a virus having single-stranded minus strand RNAs as the genome. Such viruses include viruses belonging to the families of, for example, Paramyxoviridae (including the genera such as *Paramyxovirus, Morbillivirus, Rubulavirus,* and *Pneumovirus*), Rhabdoviridae (including the genera such as *Vesiculovirus, Lyssavirus,* and *Ephemerovirus*)*,* Filoviridae, Orthomyxoviridae (including *Influenza virus* A, B, and C, and *Thogoto-like virus,* etc.), Bunyaviridae (including the genera such as *Bunyavirus, Hantavirus, Nairovirus,* and *Phlebovirus*), and Arenaviridae.

Specific examples of minus strand RNA viruses that can be preferably used in the present invention include *Sendai virus, Newcastle disease virus, Mumps virus, Measles virus, RS virus (Respiratory syncytial virus), rinderpest virus, distemper virus, Monkey parainfluenza virus* (SV5), *human parainfluenza type 1, 2, and 3 viruses,* belonging to the Paramyxoviridae family; *Influenza virus* belonging to the Orthomyxoviridae family; *Vesicular stomatitis virus* and *Rabies virus* belonging to the Rhabdoviridae family; and such.

In the present invention, Paramyxoviruses are preferably used. A Paramyxovirus refers to a virus belonging to Paramyxoviridae or derivatives thereof. Paramyxoviruses are a group of viruses with non-segmented negative-strand RNAs as their genome, and include Paramyxovirinae (including *Respirovirus* also referred to as *Paramyxovirus, Rubulavirus,* and *Morbillivirus*), and Pneumovirinae (including *Pneumovirus* and *Metapneumovirus*). Specific examples of Paramyxoviruses applicable to the present invention are *Sendai virus, Newcastle disease virus, Mumps virus, Measles virus, Respiratory syncytial virus (RS virus), Rinderpest virus, Distemper virus, Simian parainfluenza virus* (SV5), and *Human parainfluenza viruses 1, 2, and 3*. More specifically, such examples include *Sendai virus* (SeV), *Human parainfluenza virus-1* (HPIV-1), *Human parainfluenza virus-3* (HPIV-3), *Phocine distemper virus* (PDV), *Canine distemper virus* (CDV), *Dolphin molbillivirus* (DMV), *Peste-des-petits-ruminants virus* (PDPR), *Measles virus* (MV), *Rinderpest virus* (RPV), *Hendra virus* (Hendra), *Nipah virus* (Nipah), *Human parainfluenza virus-2* (HPIV-2), *Simian parainfluenza virus* 5 (SV5), *Human parainfluenza virus-4a* (HPIV-4a), *Human parainfluenza virus-4b* (HPIV-4b), *Mumps virus* (Mumps), and *Newcastle disease virus* (NDV). A more preferred example is a virus selected from a group consisting of *Sendai virus* (SeV), *Human parainfluenza virus-1* (HPIV-1), *Human parainfluenza virus-3* (HPIV-3), *Phocine distemper virus* (PDV), *Canine distemper virus* (CDV), *Dolphin molbillivirus* (DMV), *Peste-des-petits-ruminants virus* (PDPR), *Measles virus* (MV), *Rinderpest virus* (RPV), *Hendra virus* (Hendra), and *Nipah virus* (Nipah). Viruses of this invention are preferably those belonging to Paramyxovirinae (including *Respirovirus, Rubulavirus,* and *Morbillivirus*) or derivatives thereof, and more preferably those belonging to the genus *Respirovirus* (also referred to as *Paramyxovirus*) or derivatives thereof. Examples of viruses of the genus *Respirovirus* applicable to this invention are *Human parainfluenza virus-1* (HPIV-1), *Human parainfluenza virus-3* (HPIV-3), *Bovine parainfluenza virus-3* (BPIV-3), *Sendai virus* (also referred to as *Murine parainfluenza virus-1),* and *Simian parainfluenza virus-10* (SPIV-10). It is most preferable that *Sendai virus* be used in the present invention. These viruses may be derived from natural strains, wild strains, mutant strains, laboratory-passaged strains, artificially constructed strains, or the like.

In the present invention, a "vector" is a carrier for introducing nucleic acids into cells. Minus strand RNA viral vectors are carriers derived from minus strand RNA viruses to introduce nucleic acids into cells. Minus strand RNA viruses such as SeV are excellent gene transfer vectors. Since minus strand RNA viruses do not have a DNA phase and carry out transcription and replication only in the cytoplasm of host cells, chromosomal integration does not occur. Therefore, they do not give rise to safety problems caused by chromosomal aberrations, such as canceration and immortalization. This characteristic of minus strand RNA viruses contributes greatly to safety when using a minus strand RNA virus as a vector. When used for a foreign gene expression, SeV hardly showed any nucleotide mutations even after multiple continuous passages, indicating high stability of its genome and long-term stable expression of inserted foreign genes (Yu, D. et al., Genes Cells 2: 457-466, 1997). The lack of a capsid structure protein in SeV yields further qualitative merits, such as flexibility in the size of genes to be inserted and in the packaging thereof. A transmissible SeV vector can introduce a foreign gene of at least 5.5 kb in size, and can simultaneously express two or more genes by adding transcription units.

*Sendai virus* is known to be pathogenic to rodents, causing pneumonia; however, it is not reported to be pathogenic in humans. This was supported by previous reports that intranasal administration of a wild type *Sendai virus* to non-human primates and humans did not cause severe adverse effects (Hurwitz, J. L. et al., Vaccine 15: 533-540, 1997; Slobod, K. S. et al., Vaccine 22: 3182-3186, 2004). Two points, namely "high infectivity" and "high expression level", can be given as examples of the advantage. SeV vectors infect cells by binding to sialic acid in the glucolipids and glycoproteins of the cell membrane. This sialic acid is expressed in almost all mammalian and avian cells, giving rise to a broad spectrum of infection, *i.e*., high infectivity. When a transmissible SeV replicon-based vector releases viruses, they re-infect neighboring cells. Multiple RNPs are replicated in the cytoplasm of the infected cells and distributed into the daughter cells via cell division, and thus continuous expression can be expected. Further, SeV vectors can be applied to an extremely wide range of tissues. Furthermore, their characteristic expression machinery, wherein transcription and replication occur only in the cytoplasm, has been shown to express inserted genes at very high levels (Moriya, C. et al., FEBS Lett. 425(1): 105-111, 1998; WO 00/70070). Furthermore, SeV vectors made non-transmissible by deleting an envelope gene have been successfully recovered (WO 00/70070; Li, HO. et al., J. Virol. 74(14): 6564-6569, 2000). Thus, SeV vectors have been improved to further enhance their "safety" while maintaining their "high infectivity" and "high expression levels".

A minus strand RNA viral vector of the present invention comprises the genomic RNA of a minus strand RNA virus. Genomic RNA refers to an RNA that has a function to form an RNP comprising certain viral proteins of a minus strand RNA virus, which cause the expression of genes in the genome. Thus the nucleic acid is replicated to form daughter RNPs. RNAs of a minus strand RNA virus encode genes as antisense sequences. In general, in a minus strand RNA viral genome, viral genes are arranged as antisense sequences between 3'-leader region and 5'-trailer region. Between ORFs of respective genes are a transcription ending sequence (E sequence) - intervening sequence (I sequence) - transcription starting sequence (S sequence), such that the RNA encoding the ORF of each gene is transcribed as an individual cistron. Genomic RNAs in a vector of the present invention comprise antisense RNA sequences encoding N (nucleocapsid, also referred to as nucleoprotein (NP))-, P (phospho)-, and L (large)-proteins, which are viral proteins essential for the expression of a group of genes encoded by the RNA, and for the autonomous replication of the RNA itself. The RNAs may also encode M (matrix) protein essential for virion formation. Further, the RNAs may encode envelope proteins essential for virion infection. Envelope proteins of a minus strand RNA virus include F (fusion) protein causing cell membrane fusion, and HN (hemagglutinin-neuraminidase) or H (hemagglutinin) protein essential for viral adhesion to cells. However, HN protein is not required in infection to certain types of cells (Markwell, M.A. et al., Proc. Natl., Acad. Sci. USA 82(4): 978-982, 1985), and infection is achieved with F protein alone. The RNAs may encode envelope proteins other than F and/or HN proteins.

A minus strand RNA viral vector of the present invention may be, for example, complexes of the genomic RNAs of a minus strand RNA virus and viral proteins, that is, ribonucleoproteins (RNPs). RNPs can be introduced into cells, for example, in combination with a desired transfection reagent. Specifically, such RNPs are complexes comprising the genomic RNAs of a minus strand RNA virus, N protein, P protein, and L protein. On introducing RNPs into cells, cistrons encoding the viral proteins are transcribed from the genomic RNAs by the action of viral proteins, and at the same time, the genome itself is replicated to form daughter RNPs. Replication of a genomic RNA can be confirmed by RT-PCR, Northern blot hybridization, or the like to detect an increase in the RNA copy number.

Further, minus strand RNA viral vectors of the present invention are preferably minus strand RNA virus virions. "Virion" refers to a micro-particle comprising nucleic acids and released from cells by the action of viral proteins. Virions of a minus strand RNA virus have a structure in which the above-described RNP, comprising genomic RNAs and viral proteins, is enclosed in a cell membrane-derived lipid membrane (referred to as an envelope). Virions may have infectivity. Infectivity refers to the ability of virions to introduce carried nucleic acids into cells that they have adhered to, based on the cell adhesion and membrane-fusion abilities of the minus strand RNA viral vector. Minus strand RNA viral vectors of the present invention may be transmissible or non-transmissible vectors. "Transmissible" means that when a viral vector infects a host cell, the virus can replicate itself within the cell to produce infectious virions.

For example, each gene in viruses belonging to Paramyxovirinae is generally described as follows. In general, N gene is also described as "NP".

| | | | | | | | |
|---|---|---|---|---|---|---|---|
| *Respirovirus* | NP | P/C/V | M | F | HN | - | L |
| *Rubulavirus* | NP | P/V | M | F | HN | (SH) | L |
| *Morbillivirus* | NP | P/C/V | M | F | H | - | L |

For example, the database accession numbers for the nucleotide sequences of each of *Sendai virus* genes are: M29343, M30202, M30203, M30204, M51331, M55565, . M69046, and X17218 for N gene; M30202, M30203, M30204, M55565, M69046, X00583, X17007, and X17008 for P gene; D11446, K02742, M30202, M30203, M30204, M69046, U31956, X00584, and X53056 for M gene; D00152, D11446, D17334, D17335, M30202, M30203, M30204, M69046, X00152, and X02131 for F gene; D26475, M12397, M30202, M30203, M30204, M69046, X00586, X02808, and X56131 for HN gene; and D00053, M30202, M30203, M30204, M69040, X00587, and X58886 for L gene. Examples of viral genes encoded by other viruses are: CDV, AF014953; DMV, X75961; HPIV-1, D01070; HPIV-2, M55320; HPIV-3, D10025; Mapuera, X85128; Mumps, D86172; MV, K01711; NDV, AF064091; PDPR, X74443; PDV, X75717; RPV, X68311; SeV, X00087; SV5, M81442; and Tupaia, AF079780 for N gene; CDV, X51869; DMV, Z47758; HPIV-1, M74081; HPIV-3, X04721; HPIV-4a, M55975; HPIV-4b, M55976; Mumps, D86173; MV, M89920; NDV, M20302; PDV, X75960; RPV, X68311; SeV, M30202; SV5, AF052755; and Tupaia, AF079780 for P gene; CDV, AF014953; DMV, Z47758; HPIV-1, M74081; HPIV-3, D00047; MV, ABO16162; RPV, X68311; SeV, AB005796; and Tupaia, AF079780 for C gene; CDV, M12669; DMV, Z30087; SPIV-1, S38067; HPIV-2, M62734; HPIV-3, D00130; HPIV-4a, D10241; HPIV-4b, D10242; Mumps, D86171; MV, AB012948; NDV, AF089819; PDPR, Z47977; PDV, X75717; RPV, M34018; SeV, U31956; and SV5, M32248 for M gene; CDV, M21849; DMV, AJ224704; HPN-1, M22347; HPIV-2, M60182; HPIV-3, X05303; HPIV-4a, D49821; HPIV-4b, D49822; Mumps, D86169; MV, AB003178; NDV, AF048763; PDPR, Z37017; PDV, AJ224706; RPV, M21514; SeV, D17334; and SV5, AB021962 for F gene; and, CDV, AF112189; DMV, AJ224705; HPIV-1, U709498; HPIV-2, D000865; HPIV-3, AB012132; HPIV-4A, M34033; HPIV-4B, AB006954; Mumps, X99040; MV, K01711; NDV, AF204872; PDPR, Z81358; PDV, Z36979; RPV, AF132934; SeV, U06433; and SV-5, S76876 for HN (H or G) gene. However, a number of strains are known for each virus, and genes comprising sequences other than those cited above exist due to differences in strains.

The ORFs of these viral proteins are arranged as antisense sequences in the genomic RNAs using the above-described E-I-S sequence. The ORF closest to 3'-end of the genomic RNAs only requires S sequence between 3'-leader region and the ORF, and does not require E and I sequence. Further, the ORF closest to 5'-end of the genomic RNA only requires E sequence between 5'-trailer region and the ORF, and does not require I and S sequences. Furthermore, two ORFs can be transcribed as a single cistron, for example, using an IRES sequence. In such a case, the E-I-S sequence is not required between these two ORFs. For example, in wild type Paramyxoviruses, a typical RNA genome comprises a 3'-leader region, six ORFs encoding N, P, M, F, HN, and L proteins, and a 5'-trailer region in the antisense order. As in wild type viruses, genomic RNAs of the present invention preferably have ORFs encoding N, P, M, F, HN, and L proteins arranged in this order with a preceding 3'-leader region and a succeeding 5'-trailer region; however, the gene arrangement is not limited to this. Certain types of minus strand RNA viruses do not comprise all the six viral genes, but even in such cases, it is preferable to arrange their viral genes as in the wild type described above. In general, vectors carrying N, P, and L genes can autonomously express genes from the RNA genome in cells, resulting in replication of the genomic RNAs. Furthermore, by the action of genes such as F and HN (or H) genes and M gene encoding envelope-constituting proteins, infectious virions are formed and released to the outside of cells. Thus, such vectors become transmissible viral vectors. A gene encoding a polypeptide that comprises Aβ or IL-10 may be inserted into a non-protein coding region in this genome as described below.

Further, a minus strand RNA viral vector of the present invention may lack any gene(s) of the wild type minus strand RNA virus. As long as the viral genome RNAs encode the necessary viral proteins (N, L, and P) for RNP reconstruction, it can replicate within cells and express genes even if it does not encode envelope-constituting proteins. For example, depending on the virus type, vectors deficient in at least one of the genes encoding envelope-constituting proteins such as F, H, HN, G, M, and M1 can be illustrated (WO 00/70055 and WO 00/70070; Li, H.-O. et al., J. Virol. 74(14): 6564-6569, 2000). For example, a vector that does not comprise M, F, or HN gene, or any combinations thereof, can be suitably used as a viral vector of the present invention. Such viral vectors can be reconstructed, for example, by supplying the product(s) of the defective gene(s) from the outside. The viral vectors thus prepared adhere to host cells to cause cell fusion as well as wild type viruses, but they cannot form daughter virions that have the same infectivity as the original virus because the vector genome introduced into the cells lacks any of the original viral genes. Therefore, such vectors are safe and useful viral vectors to introduce genes for only once. Examples of genes, that the genome may be defective of, are the F gene and/or HN gene. For example, viral vectors can be reconstructed by transfecting host cells with a plasmid vector expressing a recombinant minus strand RNA viral genome defective of F gene, along with an F protein expression vector as well as expression vectors for NP, P, and L proteins (WO 00/70055 and WO 00/70070; Li, H.-O. et al., J. Virol. 74(14): 6564-6569,2000). Viruses can also be produced, for example, using host cells that have F gene incorporated into their chromosomes. When supplying these proteins from the outside, their amino acid sequences do not need to be the same as the original viral sequences, and a mutant or homologous gene from another virus may be used as a substitute, as long as the nucleic acid-introducing activity is the same as or greater than that of the natural type.

Further, a vector comprising an envelope protein other than that of the virus from which the vector genome was derived may be produced as a viral vector of the present invention. For example, when reconstructing a virus, a viral vector comprising a desired envelope protein can be generated by expressing an envelope protein other than the envelope protein of the original virus of the vector on a packaging cell. Such proteins are not particularly limited, and include envelope proteins of other viruses, for example, G protein of *Vesicular stomatitis virus* (VSV-G)(J. Virology 39: 519-528, 1981). Viral vectors of the present invention include a pseudo-type viral vector comprising an envelope protein such as VSV-G protein derived from viruses other than the virus from which the genome was derived. By designing such a viral vector that the envelope protein(s) is not encoded in the virus RNA genome, the protein(s) will not be expressed after the virion infected cells.

Furthermore, a viral vector of the present invention may be, for example, a vector with, on the envelope surface, a protein that can attach to a specific cell, such as an adhesion factor, ligand, receptor, antibody or fragment thereof; or a vector comprising a chimeric protein with such a protein in the extracellular domain and a polypeptide derived from a virus envelope in the intracellular domain. Thus, vectors targeting specific tissues can also be produced. Such proteins may be encoded in the viral genome, or supplied by expressing the genes other than the viral genome (for example, other expression vectors or genes existing in the host cell chromosome) at the time of viral vector reconstruction.

Further, in vectors of the present invention, any viral gene comprised in the vectors may be modified from the wild type gene in order to reduce the immunogenicity caused by the viral protein, or to enhance RNA transcriptional or replication efficiency, for example. Specifically, for example, in a minus strand RNA viral vector, modification of at least one of the replication factors, N, P, and L genes, is considered to enhance transcription or replication efficiency. Further, HN protein, an envelope protein, has both hemagglutinin activity and neuraminidase activity. So, it is possible, for example, to improve viral stability in the blood if the former activity can be attenuated, and to control infectivity if the latter activity is modified. Further, it is also possible to control membrane fusion ability by modifying F protein. For example, analysis of antigen-presenting epitopes of F protein and HN protein, both potential cell surface antigenic molecules, enables to make viral vectors with reduced antigenicity of these proteins. Furthermore, a temperature-sensitive mutation may be introduced into a viral gene to suppress the release of secondarily released particles or virus like particles (VLPs) (WO 2003/025570). For example, mutations such as G69E, T116A and A183S, can be introduced into M gene; A262T, G264, and K461 G into HN gene; L511 F into P gene; and N1197S and K1795E into L gene. Temperature-sensitive mutations that can be introduced are not limited to the mutations described above (see WO 2003/025570).

Furthermore, vectors of the present invention may be defective of accessory genes. For example, by knockout of V gene, one of the SeV accessory genes, the pathogenicity of SeV toward hosts such as mice is remarkably reduced without hindering gene expression and replication in cultured cells (Kato, A. et al., J. Virol. 71: 7266-7272, 1997; Kato, A. et al., EMBO J. 16: 578-587, 1997; Curran, J. et al., WO 01/04272, EP1067179). Such attenuated vectors are particularly useful as nontoxic viral vectors for *in vivo* or *ex vivo* gene transfer.

Vectors of the present invention comprise an Aβ-encoding nucleic acid as a foreign gene within the genome of a minus strand RNA viral vector described above. There is no limitation on the number of amino acids encoding Aβ. For example, Aβ1-40, Aβ1-42, and Aβ1-43 are all acceptable. Any desired fragment having the antigenicity of a naturally occurring full-length Aβ or any polypeptide comprising the fragment can also be used as Aβ. To exhibit antigenicity against Aβ, such polypeptides must comprise six or more consecutive amino acids, preferably seven or more consecutive amino acids, or eight or more consecutive amino acids, derived from the amino acid sequence of naturally occurring Aβs, for example, Aβ43 (Harlow, Antibodies: A laboratory Manual, 1998; Chapter 5 page 76). For example, the majority of B cell epitopes against Aβ42 (1 to 42 in SEQ ID NO: 27) are concentrated in the 1st to 15th amino acids ofN-terminal region (Cribbs, D.H. et al., Int. Immunol. 15(4): 505-14, 2003). Thus, a polypeptide comprising the I st to 15th amino acids of Aβ42 can be suitably used as an Aβ peptide to be expressed from the vector. Alternatively, a polypeptide comprising the 1 st to 21 st amino acids or the 4th to 10th amino acids of Aβ42 may be expressed (JP-A No. 2005-21149). In addition, the anti-Aβ monoclonal antibodies 10D5 and 6C6, known for their ability to inhibit amyloid fibril formation and to protect neurons, have been reported to recognize a four amino acid-epitope corresponding to the 3rd to 6th amino acids of Aβ42 (EFRH; 3 to 6 in SEQ ID NO: 27) (Frenkel, D. et al., J. Neuroimmunol. 88: 85-90, 1998; Frenkel, D. et al., J. Neuroimmunol. 95: 136-142, 1999). The monoclonal antibody 508F that recognizes the same epitope also suppresses Aβ neurotoxicity (Frenkel, D. et al., J. Neuroimmunol. 106: 23-31, 2000). Thus, a polypeptide of 6 to 8 amino acids or more in the Aβ sequence including this sequence can be suitably used. Epitopes corresponding to cellular immunity are concentrated in the C-terminal region of Aβ. Accordingly, humoral immunity can be made relatively dominant over cellular immunity by expressing a fragment, which comprises an N-terminal fragment such as Aβ1-21, but not sequences near to the C-terminal as Aβ22-43 (the 22nd to 43rd amino acids of SEQ ID NO: 27). A vector encoding a polypeptide that comprises the sequence of naturally occurring Aβ peptides (Aβ39, Aβ40, Aβ41, Aβ42, and Aβ43 corresponding to respectively 1-39, 1-40, 1-41, 1-42, and 1-43 amino acids of SEQ ID NO: 27) can be suitably used as a vector of the present invention. Short Aβ peptide fragments may be used for making polypeptides fused with an appropriate carrier protein to increase their immunogenicity. Carrier proteins include keyhole limpet hemocyanin (KLH), bovine serum albumin (BSA), rabbit serum albumin (RSA), human serum albumin (HSA), ovalbumin (OVA), thyroglobulin (TG), and such. The short peptides can be fused with a carrier protein via a spacer of about I to 5 amino acids. The length (amino acid length excluding the carrier protein portion) of an antigen polypeptide (a mature polypeptide when it is in a secretory form) preferably ranges from 10 to 200 amino acids, more preferably from 15 to 100 amino acids, still more preferably from 20 to 80 amino acids.

Furthermore, an Aβ polypeptide encoded in a vector preferably comprises a secretion signal to ensure its secretion from the cells. As a secretion signal sequence, the signal sequence of a desired secretory protein, such as interleukin (IL)-2 or tissue plasminogen activator (tPA), can be used, and the N-terminal signal sequence of amyloid precursor proteins (APPs) is preferably used. Specifically, such secretion signal sequences include, for example, the secretion signal sequence of Accession number NP_958817 (for the nucleotide sequence, see, for example, Accession number NM_201414). In other words, a suitable vector of the present invention encodes an Aβ peptide to which a secretion signal has been attached.

In addition, vectors of the present invention may comprise nucleic acids encoding Th2 cytokines and/or anti-inflammatory cytokines as foreign genes. Th2 cytokines refer to cytokines produced predominantly by type 2 helper T cells (Th2 cells) rather than type 1 helper T cells (Th1 cells). Specifically, such Th2 cytokines include IL-4, IL-5, IL-6, IL-9, IL-10, and IL-13. In the present invention, a cytokine encoded in a vector is preferably selected from a group consisting of IL-4, IL-10, and IL-13, and most preferably IL-10 is encoded. The nucleotide sequence of each cytokine gene and its amino acid sequence are already known (IL-4 : NM_000589, NP_000580, AAH66277, AAH67515, NP_758858, NP_067258, NP_958427 ; IL-5 : NM_000879, NP_000870, CAA31210, NP_034688, NP_068606 ; IL-6 : NM_000600, NP_000591, XP_518992, AAB30962, NP_112445 ; IL-9 : NM_000590, NP_000581, AAH66284, AAH66287, NP_032399, XP_341488 ; IL-10 : NM_000572, NP_000563, CAG46825, NP_034678, NP_036986 ; IL-13 : NM_002188,NP_002179, AAB01681,NP_032381, NP_446280).

A Th2 cytokine, such as IL-10, encoded in a vector may be a complete (i.e. wild-type) or partial peptide (i.e. active fragment) as long as it retains the activity. For example, the N-terminal signal sequence can be appropriately replaced by another signal sequence. Alternatively, the cytokine may be expressed as a fused protein with other peptides.

There is no limitation of the origin of Aβ and cytokines such as IL-10 encoded in a vector, which can be of any mammals including mice, rats, guinea pigs, rabbits, pigs, cattle, horses, donkeys, goats, dogs, chimpanzees, monkeys, and humans. The origins of Aβ and cytokines may be the same or different. It is appropriate to use Aβ and cytokines derived from a species that is the same as the subject of administration. Nucleotide sequences of the nucleic acids encoding such cytokines are available from the databases described above. Additional examples such as Genbank Accession NOs. AY410237 and NM_010548 are available for mouse IL-10; and Genbank Accession NOs. AY029171 and NM_000572 are available for human IL-10. An example of "Aβ-encoding nucleic acid" that can be suitably used as a vector of the present invention is human Aβ cDNA (SEQ ID NO: 1). Examples of "IL-10-encoding nucleic acid" that can be suitably used are mouse IL-10 cDNA sequence (SEQ ID NO: 2) and human IL-10 cDNA sequence (SEQ ID NO: 3). Nucleic acids having sequences different from the above nucleotide sequences of SEQ ID NOs 1 to 3 can also be suitably used for a vector of the present invention like the sequences of SEQ ID NOs I to 3, provided that they have the same amino acid sequences as the above-mentioned nucleotide sequences as a result of codon degeneracy. Insert region of the above foreign genes can be selected, for example, in a desired portion in the non-protein coded region in the genome. For example, they can be inserted between 3'-leader region and a viral protein ORF closest to 3'-end; between each of the viral protein ORFs; and/or between a viral protein ORF closest to 5'-end and 5'-trailer region. Further, in genomes defective of F or HN gene or the like, foreign gene(s) can be inserted into those defected regions. When introducing a foreign gene into a Paramyxovirus, the chain length of the polynucleotide to be inserted into the genome is preferably a multiple of six (J. Virology 67 (8): 4822-4830, 1993). A set of E-I-S sequences should be arranged between the inserted foreign gene and the viral ORF. Two or more foreign genes can be inserted in a tandem manner using E-I-S sequences. Alternatively, a desired foreign gene may be inserted using IRES.

Expression levels of a foreign gene carried by a vector can be controlled depending on the type of a transcriptional initiation sequence added in the upstream (to the 3'-side of a minus strand) of the gene (WO 01/18223). Expression levels can also be controlled depending on the position at which the foreign gene is inserted in the genome: the closer the inserting position is to 3'-end of a minus strand, the higher the expression level; the closer the inserting position is to 5'-end, the lower the expression level. Thus, the position inserting a foreign gene can be appropriately adjusted to obtain a desired gene expression level, or to achieve the most suitable combination with viral protein-encoding genes in the vicinity of the foreign gene. In general, as it is thought to be advantageous to get high expression levels of Aβ, it is preferable to link a foreign gene encoding Aβ to a high-efficiency transcriptional initiation sequence and thus to insert it near to 3'-end of the minus strand genome. Specifically, a foreign gene is inserted between 3'-leader region and a viral protein ORF closest to 3'-end. Alternatively, a foreign gene may be inserted between an ORF of the viral gene closest to 3'-end and the next viral gene. In wild type Paramyxoviruses, the viral protein gene closest to 3'-end of the genome is N gene, and the second closest gene is P gene. Alternatively, when a high level expression of the introduced gene is undesirable, the level of gene expression from the viral vector can be suppressed in order to get an appropriate effect, for example, by inserting the foreign gene at a site in the vector as close as possible to 5'-side of the minus strand genome, or by selecting a low-efficiency transcriptional initiation sequence.

To express two polypeptides, one comprising Aβ and the other comprising a Th2 cytokine from a vector, the nucleic acids encoding the respective polypeptides are inserted into a vector genome. The two nucleic acids may be inserted into separate sites, or into a single site in a tandem manner *via* an E-I-S sequence. It is preferable to use a high-efficiency transcription initiation sequence as an S sequence. For example, 3'-UCCCACUUU-5'(minus strand RNA; SEQ ID NO: 6), 3'-UCCCAGUUU-5'(minus strand RNA; SEQ ID NO: 7), or 3'-UCCCACUUA-5'(minus strand RNA; SEQ ID NO: 8) can be suitably used as the S sequence.

A vector of the present invention may carry another foreign gene at a position other than where genes encoding an Aβ and a Th2 cytokine have been inserted. Such foreign genes are not limited. For example, they may be marker genes for monitoring vector infection, or immune system-regulating genes such as cytokines and hormones. A vector of the present invention may carry one or more genes encoding such as signal transduction regulators, cytokines, hormones, receptors, antibodies, and fragments thereof. A vector of the present invention can introduce a gene either by a direct (*in vivo*) administration to a target site of a living body, or by an indirect (*ex vivo*) administration, in which a vector is infected to patient's cells or other cells and then the cells are injected into the target site.

Vectors of the present invention can be used as highly advantageous means for treating, preventing, or inhibiting the progression of Alzheimer's disease. A vector of the present invention exhibited a remarkable Aβ-eliminating effect in 24- to 25-month-old APP Tg mice, which generally have a large amount of accumulated Aβ. Since this effect was confirmed with only a single intranasal administration, vectors of the present invention can be a much less-invasive therapeutic method for patients. The finding that no inflammation was observed in the central nervous system also proves the vector highly safe to use.

To make a vector of the present invention, cDNA encoding the genomic RNA of a minus strand RNA virus of this invention is transcribed in mammalian cells in the presence of the viral proteins (i.e., N, P, and L proteins) essential for reconstruction of RNP that comprises the genomic RNA of a minus strand RNA virus (or a complementary strand thereof). The viral RNP can be reconstructed by generating either a minus strand genome (that is, same as the viral genome antisense strand) or a plus strand (the viral protein-encoding sense strand) via transcription. Generating a plus strand is preferable to increase the efficiency of the vector reconstruction. The RNA termini preferably reflect the termini of 3'-leader sequence and 5'-trailer sequence as accurately as possible, as in the natural viral genome. To accurately regulate 5'-end of the transcription products, for example, a sequence recognizable by T7 RNA polymerase may be used as a transcription initiation site and the RNA polymerase may be expressed within a cell. To regulate 3'-end of the transcription products, for example, an autocleavage-type ribozyme may be encoded at 3'-end of the transcription products to allow accurate cleavage of 3'-end by this ribozyme (Hasan, M. K. et al., J. Gen. Virol. 78: 2813-2820, 1997; Kato, A. et al., EMBO J. 16: 578-587, 1997; and Yu, D. et al., Genes Cells 2: 457-466, 1997).

For example, based on the descriptions by Hasan, M. K. et al., J. Gen. Virol. 78: 2813-2820, 1997; Kato, A. et al., EMBO J. 16: 578-587, 1997; Yu, D. et al., Genes Cells 2: 457-466, 1997; or the like, a recombinant *Sendai virus* vector carrying a foreign gene can be constructed as follows;

First, a DNA sample comprising a cDNA nucleotide sequence of a foreign gene of interest is prepared. The DNA sample is preferably one that can be confirmed as a single plasmid by electrophoresis at a concentration of no less than 25 ng/µl. The following explains an example using *Not*I site to insert a foreign gene into a DNA encoding a viral genomic RNA. In cases where *Not*I recognition sites are contained in a cDNA nucleotide sequence of interest, the nucleotide sequence should be altered using site-directed mutagenesis or the like, such that the encoded amino acid sequence does not change, and the *Not*I sites are preferably excised in advance. The gene fragment of interest is amplified from this sample by PCR, and then recovered. By adding a *Not*I site to the 5' region of two primers, both ends of the amplified fragment become *Not*I sites. E-I-S sequences, or parts thereof, are included in the primers such that, after a foreign gene is inserted into the viral genome one E-I-S sequence is placed between the ORF of the foreign gene and the ORF of the viral genes on each side.

For example, to guarantee *Not*I cleavage, the forward synthetic DNA sequence has a form in which an arbitrary sequence of no less than two nucleotides (preferably four nucleotides not comprising a *Not*I recognition site-derived sequence such as GCG and GCC, and more preferably ACTT) is selected at the 5'-side, and a *Not*I recognition site gcggccgc is added to its 3'-side. To this 3'-side, nine arbitrary nucleotides, or nine plus a multiple of six nucleotides are further added as a spacer sequence. Further to the 3'-side, a sequence corresponding to about 25 nucleotides of the ORF of a desired cDNA including the initiation codon ATG is added. The 25 nucleotides are preferably selected from a desired cDNA such that the final nucleotide at the 3'-end of the forward synthetic oligo DNA is G or C.

For the reverse synthetic DNA sequence, no less than two arbitrary nucleotides (preferably four nucleotides not comprising a *Not*I recognition site-derived sequence such as GCG and GCC, and more preferably ACTT) are selected at the 5'-side, a *Not*I recognition site 'gcggccgc' is added to its 3'-side, and further an oligo DNA fragment is added to this 3'-side for length adjustment. The length of this oligo DNA is designed such that the chain length of the *Not*I fragment, the final PCR-amplification product, will become a multiple of six nucleotides including the added E-I-S sequences (so-called "rule of six": Kolakofski, D., et al., J. Virol. 72: 891-899, 1998; Calain, P. and Roux, L., J. Virol. 67: 4822-4830, 1993; Calain, P. and Roux, L., J. Virol. 67: 4822-4830, 1993). When an E-I-S sequence is added to the 3'-side of the inserted oligo DNA fragment of this primer, a complementary strand sequence of the *Sendai virus* S sequence, preferably 5'-TTTCACCCT-3' (SEQ ID NO: 9), 5'-TTTGACCCT-3' (SEQ ID NO: 10), or 5'-ATTCACCCT-3' (SEQ ID NO: 11), a complementary strand sequence of I sequence, preferably 5'-AAG-3', a complementary strand sequence of E sequence, preferably 5'-TTTTTCTTACTACGG-3' (SEQ ID NO: 12) are added. Further to this 3'-side, a complementary strand sequence with G or C as the last nucleotide is added, in which the length was selected to be about 25 nucleotides counted backwards from the termination codon of a desired cDNA sequence. Thus the 3'-end of the reverse synthetic DNA is made.

PCR can be performed by a standard method using Taq polymerase or other DNA polymerases. The amplified fragments of interest are digested with *Not*I and then inserted into the *Not*I site of a plasmid vector such as pBluescript^{™} (Stratagene). The nucleotide sequence of the PCR products thus obtained is confirmed with a sequencer, and plasmids comprising the correct sequence are selected. The inserted fragment is excised from these plasmids using *Not*I*,* and cloned into the *Not*I site of a plasmid comprising the genomic DNA. The recombinant *Sendai virus* cDNA can also be obtained by inserting the fragment directly into the *Not*I site without using a plasmid vector.

For example, a recombinant *Sendai virus* genomic cDNA can be constructed according to the methods described in literature (Yu, D. et al., Genes Cells 2: 457-466, 1997; Hasan, M. K. et al., J. Gen. Virol. 78: 2813-2820, 1997). For example, an 18 bp spacer sequence (5'-(G)-CGGCCGCAGATCTTCACG-3') (SEQ ID NO: 13) comprising *a Not*I restriction site is inserted between the leader sequence and the ORF of N protein in a cloned *Sendai virus* genomic cDNA (pSeV(+)) to obtain plasmid pSeV18⁺b(+), which comprises an autocleavage ribozyme site derived from an antigenomic strand of delta hepatitis virus (Hasan, M. K. et al., J. General Virology 78: 2813-2820, 1997). A recombinant *Sendai virus* cDNA comprising a desired foreign gene can be obtained by inserting a foreign gene fragment into the *Not*I site of pSeV18⁺b(+).

A vector of the present invention can be reconstructed by transcribing a DNA encoding the genomic RNA of a recombinant virus thus prepared in cells under the presence of the above-described viral proteins (L, P, and N). The present invention provides DNAs encoding the viral genomic RNAs of a vector of the present invention for producing a vector of this invention. The present invention also relates to the use of DNAs encoding the genomic RNAs of a vector for application in producing a vector of this invention. Recombinant viruses can be reconstructed by the methods known in literature (WO 97/16539; WO 97/16538; WO 00/70055; WO 00/70070; WO 01/18223; WO 03/025570; Durbin, A. P. et al., Virology 235: 323-332, 1997; Whelan, S. P. et al., Proc. Natl. Acad. Sci. USA 92: 8388-8392, 1995; Schnell. M. J. et al., EMBO J. 13: 4195-4203, 1994; Radecke, F. et al., EMBO J. 14: 5773-5784, 1995; Lawson, N. D. et al., Proc. Natl. Acad. Sci. USA 92: 4477-4481, 1995; Garcin, D. et al., EMBO J. 14: 6087-6094, 1995; Kato, A. et al., Genes Cells 1: 569-579, 1996; Baron, M. D. and Barrett, T., J. Virol. 71: 1265-1271, 1997; Bridgen, A. and Elliott, R. M., Proc. Natl. Acad. Sci. USA 93: 15400-15404, 1996; Hasan, M. K. et al., J. Gen. Virol. 78: 2813-2820, 1997; Kato, A. et al., EMBO J. 16: 578-587, 1997; Yu, D. et al., Genes Cells 2: 457-466, 1997; Tokusumi, T. et al., Virus Res. 86: 33-38, 2002; Li, H.-O. et al., J. Virol. 74: 6564-6569, 2000). With these methods, minus strand RNA viruses including parainfluenza virus, vesicular stomatitis virus, rabies virus, measles virus, rinderpest virus, and *Sendai virus* can be reconstructed from DNAs. Vectors of the present invention can be reconstructed according to these methods. A viral vector DNA made defective of F gene, HN gene, and/or M gene does not form infectious virions. However, infectious virions can be formed by introducing the missing gene(s) and/or gene(s) encoding the envelope protein(s) of another virus into the host cell, and then expressing the gene(s).

Specifically, a virus can be produced according to the steps of: (a) transcribing DNAs encoding the genomic RNAs (minus strand RNAs) of a minus strand RNA virus or complementary strands thereof (plus strands) in cells expressing N, P, and L proteins; and (b) collecting the RNP complex comprising the genomic RNA from these cells or culture supernatant thereof. For transcription, a DNA encoding a genomic RNA is linked to the downstream of an appropriate promoter. The genomic RNAs thus transcribed are replicated in the presence ofN, L, and P proteins to form the RNP complex. Then, in the presence of M, HN, and F proteins, virions enclosed in an envelope are formed. For example, a DNA encoding a genomic RNA can be linked to the downstream of a T7 promoter and transcribed to RNA by a T7 RNA polymerase. A desired promoter beside those comprising a T7 polymerase recognition sequence can also be used. Alternatively, RNAs transcribed *in vitro* may be transfected into cells. N, L, and P proteins may be expressed in cells using appropriate expression vectors such as plasmids. Examples of promoters include a CMV promoter and a CAG promoter (Niwa, H. et al., Gene. 108: 193-199, 1991; Japanese Patent Application Kokai Publication No. (JP-A H3-168087).

Enzymes essential for the initial transcription of a genomic RNA from DNA, such as T7 RNA polymerase, can be supplied by introducing plasmid vectors or viral vectors that express them or by, for example, incorporating genes of these enzymes into the cellular chromosome to enable induction of their expression, and then inducing expression at the time of viral reconstruction. Further, the genomic RNA and viral proteins essential for the vector reconstruction are supplied, for example, by introducing plasmids that express them. In supplying these viral proteins, the helper virus of a wild type minus strand RNA virus or certain types of mutants thereof can be used, but is not preferred since contamination of these viruses may occur.

Methods of introducing DNAs that express genomic RNAs into cells include, for example, (i) methods to make DNA precipitates that can be taken in by the cells of interest; (ii) methods to make positively charged complexes comprising DNAs that have low cytotoxicity and are suitable for intake by the cells of interest; and (iii) methods using electric pulses to instantaneously bore pores on the cell membrane with sufficient sizes through which DNA molecules can pass into the cells of interest.

Various transfection reagents can be used in method (ii), for example, DOTMA (Roche), Superfect (QIAGEN #301305), DOTAP, DOPE, DOSPER (Roche #1811169), and such. An example of (i) is a transfection method using calcium phosphate, and although DNAs transferred into cells by this method are taken in phagosomes, a sufficient amount of DNA is also known to enter the nucleus (Graham, F. L. and Van Der Eb, J., Virology 52: 456, 1973; Wigler, M. and Silverstein, S., Cell 11: 223, 1977). Chen and Okayama investigated the optimization of transfer techniques, reporting that (1) conditions for cell incubation with co-precipitates are 2 to 4% CO₂, 35°C, and 15 to 24 hours, (2) circular DNA has a higher activity than linear DNA, and (3) optimal precipitation is obtained when the precipitate mixture has a DNA concentration of 20 to 30 µg/ml (Chen, C. and Okayama, H., Mol. Cell. Biol. 7: 2745, 1987). The methods of (ii) are suitable for transient transfections. Transfection methods where a DEAE-dextran (Sigma #D-9885 M.W. 5x 10⁵) mixture is prepared based on a desired DNA concentration ratio have been known formerly. Since most complexes are decomposed in endosomes, chloroquine may also be added to enhance the efficiency (Calos, M. P., Proc. Natl. Acad. Sci. USA 80: 3015, 1983). The methods of (iii) are referred to as electroporation methods, and are more general ones than the methods (i) and (ii) because they are not cell selective. The efficiency of the methods is considered to be good under optimal conditions of: duration of pulse electric current, pulse shape, electric field potency (gap between electrodes, voltage), buffer conductivity, DNA concentration, and cell density.

Of the above three categories, the methods of (ii) are simple to operate and can examine many samples with a large amount of cells, and thus transfection reagents are suitable for introducing DNAs into cells in vector reconstruction. Transfection reagents such as Superfect Transfection Reagent (QIAGEN, Cat No. 301305), DOSPER Liposomal Transfection Reagent (Roche, Cat No. 11811169), and TransIT (Mirus, Cat No. MIR2300) can be preferably used. However it is not limited thereto.

Specifically, virus reconstruction from cDNA can be carried out, for example, as follows:

In a plastic plate of about 6- to 24-wells, or a 100-mm Petri dish or the like, simian kidney-derived LLC-MK2 cells are cultured till near 100% confluency in a minimum essential medium (MEM) containing 10% fetal calf serum (FCS) and antibiotics (100 units/ml penicillin G and 100 µg/ml streptomycin), and infected at 2 PFU/cell with, for example, the recombinant vaccinia virus vTF7-3, which expresses T7 RNA polymerase and has been inactivated by 20-minutes of UV irradiation in the presence of 1 µg/ml psoralen (Fuerst, T. R. et al., Proc. Natl. Acad. SCi. USA 83: 8122-8126,1986; Kato, A. et al., Genes Cells 1: 569-579, 1996). The amount of psoralen added and the UV irradiation time can be adjusted appropriately. One hour after infection, 2 to 60 µg and more preferably 3 to 20 µg of DNA encoding the genomic RNA of a recombinant *Sendai virus* is transfected together with plasmids expressing the trans-acting viral proteins essential for viral RNP production (0.5 to 24 µg of pGEM-N, 0.25 to 12 µg of pGEM-P, and 0.5 to 24 µg of pGEM-L) (Kato, A. et al., Genes Cells 1: 569-579, 1996) by a lipofection method using Superfect (QIAGEN) or such. The amount ratio of the expression vectors encoding N, P, and L proteins is preferably 2:1:2; and the amount of plasmids was appropriately adjusted within a range of 1 to 4 µg of pGEM-N, 0.5 to 2 µg of pGEM-P, and 1 to 4 µg of pGEM-L, for example.

The transfected cells are cultured in serum-free MEM containing 100 µg/ml of rifampicin (Sigma) and cytosine arabinoside, (AraC) (Sigma), more preferably 40 µg/ml of cytosine arabinoside (AraC) alone if desired. Optimal drug concentrations are set so as to minimize the cytotoxicity caused by the vaccinia virus, and to maximize the virus recovery rate (Kato, A. et al., Genes Cells 1: 569-579; 1996). After 48 to 72 hours of culture following transfection, cells are harvested and then disintegrated by freeze-thaw three times. LLC-MK2 cells are infected with the disintegrated materials that contain RNPs and further cultured. Alternatively, the culture supernatant is collected and added to a culture medium of LLC-MK2 cells for infection, followed by cell culturing. Transfection can be conducted by, for example, forming a complex with lipofectamine, polycationic liposome, or the like, and then introducing the complex into cells. Specifically, various transfection reagents can be used, for example, DOTMA (Roche), Superfect (QIAGEN #301305), DOTAP, DOPE, and DOSPER (Roche #1811169). Chloroquine may also be added to prevent decomposition in the endosome (Calos, M. P., Proc. Natl. Acad. Sci. USA 80: 3015, 1983). In the cells to which RNPs have been introduced, the vector is amplified as a result of the expression of viral genes from the RNP and RNP replication. By diluting the viral fluid (supernatant) thus obtained (for ' example, 10⁶-fold), and then repeating the amplification using new cells, the vaccinia virus vTF7-3 can be completely eliminated. Such amplification is repeated, for example, three or more times. The vectors thus obtained can be stored at -80°C. In order to reconstruct a viral vector incapable of transmission and defective of a gene(s) encoding an envelope protein(s), LLC-MK2 cells expressing the envelope protein(s) may be used for the transfection, or a plasmid(s) expressing the envelope protein(s) may be co-transfected. Alternatively, a defective viral vector can be amplified by culturing the transfected cells further overlaid with LLK-MK2 cells expressing the envelope protein(s) (see WO 00/70055 and WO 00/70070).

Titers of the virus thus recovered can be determined, for example, by measuring CIU (Cell Infectious Unit) or hemagglutination activity (HA) (WO 00/70070; Kato, A. et al., Genes Cells 1: 569-579, 1996; Yonemitsu, Y. & Kaneda, Y., Hemaggulutinating virus of Japan-liposome-mediated gene delivery to vascular cells. Ed. by Baker AH. Molecular Biology of Vascular Diseases, Method in Molecular Medicine: Humana Press: pp. 295-306, 1999). Titers of the vector carrying a GFP (green fluorescent protein) marker gene and the like can be quantified by directly counting the infected cells using the marker as an indicator (for example, as GFP-CIU). Titers thus measured can be handled in the same way as CIU (WO 00/70070).

As long as a viral vector can be reconstructed, the host cell used in the reconstruction is not particularly limited: For example, in reconstructing Sendai viral vectors and such, cultured cells such as LLC-MK₂ cells (ATCC CCL-7) and CV-1 cells (for example ATCC CCL-70) derived from monkey kidney, BHK cells derived from hamster kidney (for example ATCC CCL-10), and cells derived from humans can be used. By expressing a suitable envelope protein(s) in these cells, infectious virions that contain the protein(s) in their envelope can also be obtained. Further, to obtain large quantities of a Sendai viral vector, a viral vector obtained from an above-described host can be used to infect embryonated hen eggs for amplification of the vector. The method for generating viral vectors using hen eggs has already been developed (Nakanishi,M. et al. ed. "State-of-the-Art Technology Protocol in Neuroscience Research III (Shinkei Kagaku Kenkyu-no Sentan Gijutu Protocol III); Molecular Neuron Physiology (Bunshi Sinkeisaibou Seirigaku)", Koseisha, Osaka, pp. 153-172,1993). Specifically, for example, an embryo is grown by placing a fertilized egg in an incubator, and culturing for nine to twelve days at 37 to 38°C. After a viral vector is inoculated into the allantoic cavity, the egg is cultured for several days (for example, three days) to allow the viral vector to proliferate. Conditions such as the incubation period may vary depending upon the recombinant *Sendai virus* being amplified. Then, the allantoic fluid comprising the vector is collected. The Sendai viral vector can be separated and purified from the allantoic fluid according to a standard method (Tashiro, M., "Virus Experiment Protocol", Nagai, Ishihama, ed., Medical View Co., Ltd., pp. 68-73, 1995).

For example, reconstruction and production of *Sendai virus* vectors defective of F gene can be performed as described below (see WO 00/70055 and WO 00/70070):

### <1> Construction of F-gene defective Sendai virus genomic cDNA and F gene-expressing plasmids

A full-length genomic cDNA of *Sendai virus* (SeV), pSeV 18⁺ b(+) cDNA (Hasan, M. K. et al., J. General Virology 78: 2813-2820, 1997) ("pSeV 18⁺ b(+)" is also referred to as "pSeV 18⁺"), is digested with SphI/KpnI to generate a fragment (14673 bp). This fragment is then cloned into pUC18 to prepare a plasmid pUC18/KS, on which an F gene-deficient site is constructed. F gene deletion is created by a combination of PCR and ligation methods. As a result, the F gene ORF (ATG-TGA = 1698 bp) is removed. Then, atgcatgccggcagatga (SEQ ID NO: 14), for example, is ligated to construct an F gene-defective type SeV genomic cDNA (pSeV 18⁺/ΔF). A PCR product formed using primers for the upstream region of F [forward: 5'-gttgagtactgcaagagc/SEQ ID NO: 15, reverse: 5'-tttgccggcatgcatgtttcccaaggggagagttttgcaacc/SEQ ID NO: 16], and a PCR product formed using primers for the downstream region of F [forward: 5'-atgcatgccggcagatga/SEQ ID NO: 17, reverse: 5'-tgggtgaatgagagaatcagc/SEQ ID NO: 18] were ligated via the EcoT22I restriction site. A 4931 bp fragment that covers the region comprising the F gene-defective site is recovered by digesting the plasmid thus obtained with SacI and SalI, and cloned into pUC18 to form pUC18/dFSS. This pUC18/dFSS is digested with DraIII, and the fragment recovered is used to replace the DraIII fragment that covers the region comprising the F gene of pSeV18⁺ through ligation to obtain the plasmid pSeV18⁺/ΔF.

A foreign gene is inserted, for example, into the NsiI and NgoMIV restriction enzyme sites in the F gene-deleted site of pUC18/dFSS. For this, a foreign gene fragment may be, for example, amplified using an NsiI-tailed primer and an NgoMIV-tailed primer.

### <2> Production of the helper cell expressing SeV-F protein

To construct a Cre/IoxP-induced expression plasmid expressing *Sendai virus* F gene (SeV-F), SeV-F gene is amplified by PCR and inserted into an unique SwaI site of a plasmid pCALNdlw (Arai, T. et al., J. Virology 72: 1115-1121, 1998), which is designed to enable expression of a gene product by Cre DNA recombinase, to construct a pCALNdLw/F plasmid.

To recover infectious virions from F gene-defective genome, a helper cell line expressing SeV-F protein is established. For example, LLC-MK2 cells derived from monkey kidney, which is commonly used for SeV proliferation, can be used. LLC-MK2 cells are cultured in MEM supplemented with 10% heat-inactivated fetal bovine serum (FBS), penicillin G sodium (50 units/ml), and streptomycin (50 µg/ml) at 37°C in 5% CO₂. Since the SeV-F gene product is cytotoxic, LLC-MK2 cells are transfected with the above-described pCALNdLw/F plasmid, which is designed to express the F gene product with Cre DNA recombinase, by a calcium phosphate method using a mammalian transfection kit (Stratagene) according to a protocol publicly well known.

The pCALNdLw/F plasmid (10 µg) is introduced into LLC-MK2 cells grown to 40% confluence in a 10-cm plate, and the cells are then cultured in 10% FBS/MEM (10 ml) in a 5% CO₂ incubator at 37°C for 24 hours. Then, the cells are detached and suspended in the medium (10 ml). The suspension is then seeded on five 10-cm dishes: 5 ml for one dish, 2 ml each for two dishes, and 0.2 ml each for two dishes. The cells are cultured in MEM (10 ml) containing G418 (GIBCO-BRL) (1200 µg/ml) and 10% FBS for 14 days, and the medium is exchanged every two days. Cell lines stably transduced the gene are selected. The cells growing on the above medium are resistant against G418, and then recovered using a cloning ring. Each cell clone thus recovered continues to be cultured in 10-cm plates until it becomes confluent.

After the cells have grown to confluence in 6-cm dishes, F protein expression is induced by infecting the cells with Adenovirus AxCANCre, for example, at MOI = 3, according to the method of Saito, *et al.* (Saito et al., Nucl. Acids Res. 23: 3816-3821,1995; Arai, T. et al., J. Virol 72: 1115-1121, 1998).

### <3> Reconstruction and amplification of F gene-defective Sendai virus (SeV/ΔF)

The above-described pSeV18⁺/ΔF plasmid, into which a foreign gene has been inserted, is used to transfect LLC-MK2 cells as follows. LLC-MK2 cells are seeded at 5x 10⁶ cells/dish in 100-mm dishes. When genomic RNA transcription is carried out with T7 RNA polymerase, the cells are cultured for 24 hours, and then infected with a recombinant vaccinia virus expressing T7 RNA polymerase at MOI = 2 or such, for one hour at room temperature, wherein the virus has been treated with psoralen and long-wave ultraviolet rays (365 nm) for 20 minutes (PLWUV-VacT7: Fuerst, T. R. et al., Proc. Natl. Acad. Sci. USA 83: 8122-8126, 1986). For the ultraviolet ray irradiation of the vaccinia virus, for example, an UV Stratalinker^{™} 2400 equipped with five 15-watt bulbs can be used (Catalogue No. 400676 (100V), Stratagene, La Jolla, CA, USA). The cells are washed with serum-free MEM, and then transfected with a plasmid expressing the genomic RNA as well as expression plasmids expressing N, P, L, and F plus HN proteins of Paramyxovirus using an appropriate lipofection reagent. The amounts of these plasmids can be preferably set to 6 : 2 : 1 : 2 : 2 in this order, without being limited thereto. For example, a genomic RNA-expressing plasmid as well as expression plasmids expressing N, P, L, and F plus HN proteins (pGEM/NP, pGEM/P, pGEM/L, and pGEM/F-HN; WO 00/70070, Kato, A. et al., Genes Cells 1: 569-579, 1996) are transected at a respective amount of 12, 4, 2, 4 and 4 µg per dish. After culturing for several hours, the cells are washed twice with serum-free MEM, and cultured in MEM containing 40 µg/ml of cytosine β-D-arabinofuranoside (AraC: Sigma, St. Louis, MO) and 7.5 µg/ml of trypsin (Gibco-BRL, Rockville, MD). These cells are recovered, and the pellets are suspended in Opti-MEM (10⁷ cells/ml). The suspensions are freeze-thawed three times, mixed with a lipofection reagent DOSPER (Boehringer Mannheim) (10⁶ cells/25 µl DOSPER), left at room temperature for 15 minutes, and used to transfect the above-described cloned F-expressing helper cells (10⁶ cells/well in a 12-well-plate), which were cultured in serum-free MEM (containing 40 µg/ml AraC and 7.5 µg/ml trypsin) to recover supernatants. Any virus defective of a gene other than F, for example, HN and/or M gene, can also be produced by methods similar to this.

In producing viral gene-defective vectors, for example, if two or more types of vectors, in which a different viral gene has been deleted from the viral genome, are introduced into the same cell, the viral protein defective of the each vector is supplied through the expression of the other vector and thus such mutual complementation allows the construction of infectious virions and the amplification of the viral vectors through the replication cycle. As a result, mixtures of the individual viral gene-defective virus vectors can be produced at a low cost and on a large scale. Since such viruses lack any viral gene(s), their genome size is smaller than that of the intact virus. Thus, such viruses can retain larger foreign genes. In addition, such viruses are advantageous in that their release into the environment is under a control because such viruses, non-transmissible based on the defectiveness of the viral gene(s), are simply diluted following once released to the outside of the cell and become sterile due to the difficulties in maintaining infection. For example, vectors encoding Aβ and Th2 cytokine may be constructed separately so as to complement each other, and may be used for co-infection. The present invention provides compositions that comprise a minus strand RNA viral vector encoding a polypeptide comprising Aβ, and a minus strand RNA viral vector encoding a Th2 cytokine. The present invention also provides kits that comprise a minus strand RNA viral vector encoding a polypeptide comprising Aβ, and a minus strand RNA viral vector encoding a Th2 cytokine. Such compositions and kits can be used to reduce amyloid β deposition.

After a transmissible minus strand RNA viral vector is administered to an individual or cell, and when the proliferation of the viral vector needs to be arrested upon, for example, the completion of the treatment, it is also possible to specifically arrest only the proliferation of the viral vector without incurring any damage to the host by administering an RNA-dependent RNA polymerase inhibitor.

According to the methods of the present invention, a viral vector of this invention can be released into the culture medium of the virus-producing cells, for example, at a titer of 1x 10⁵ CIU/ml or more, preferably 1 x 10⁶ CIU/ml or more, more preferably 5x 10⁶ CIU/ml or more, more preferably 1x 10⁷ CIU/ml or more, more preferably 5x 10⁷ CIU/ml or more, more preferably 1x 10⁸ CIU/ml or more, and more preferably 5x 10⁸ CIU/ml or more. Viral titers can be measured by methods disclosed in the present specification and others (Kiyotani, K. et al., Virology 177(1): 65-74, 1990; WO 00/70070).

The recovered viral vectors can be purified to become substantially pure. Purification of the vectors can be performed by purification and separation methods publicly known in literature, including filtration, centrifugal separation, and column purification, or any combinations thereof. "Substantially pure" means what a viral vector accounts for a major proportion of a sample in which the viral vector exists as a component. Typically, a substantially pure viral vector can be identified by confirming that the proportion of proteins derived from the viral vector is 10% or more of all the proteins in a sample, preferably 20% or more, more preferably 50% or more, preferably 70% or more, more preferably 80% or more, and even more preferably 90% or more (excluding, however, proteins added as carriers and stabilizers). Examples of specific methods for purifying viruses are those that use cellulose sulfate ester or cross-linked polysaccharide sulfate ester (Japanese Patent Application Kokoku Publication No. (JP-B) S-62-30752, JP-B S-62-33879, and JP-B S-62-30753), and methods that attach viruses to polysaccharides comprising fucose sulfate and/or degradation products thereof (WO 97/32010).

In preparing compositions comprising a vector, the vector can be combined with a desired pharmaceutically acceptable carrier or vehicle, as necessary. A "pharmaceutically acceptable carrier or vehicle" refers to a material that can be administered together with a vector without significantly inhibiting the vector-mediated gene introduction. For example, a vector can be appropriately diluted with a physiological saline or phosphate-buffered saline (PBS) to form a composition. When a vector is grown in hen eggs or the like, the "pharmaceutically acceptable carrier or vehicle" may comprise allantoic fluids. Further, compositions comprising a vector may include carriers or vehicles such as deionized water and 5% dextrose aqueous solution. Furthermore, compositions may comprise, besides the above, plant oils, suspending agents, surfactants, stabilizers, biocides, and so on. The compositions can also comprise preservatives or other additives. The compositions comprising vectors of the present invention are useful as reagents and pharmaceuticals. The present invention relates to methods for producing anti-amyloid deposition agents, which comprise the step of producing a composition comprising a minus strand RNA viral vector encoding an Aβ peptide or cells to which the vector has been introduced, and a pharmaceutically acceptable carrier or vehicle. The present invention also relates to the use of minus strand RNA viral vectors, cells producing the vectors, or cells to which the vectors have been introduced, in producing anti-amyloid deposition agents.

Compositions comprising a vector of the present invention may contain in combination as a carrier, organic materials such as biopolymer and inorganic materials such as hydroxyapatite, specifically, collagen, matrix, polylactate polymer or copolymer, polyethylene glycol polymer or copolymer, and chemical derivatives thereof.

The compositions of the present invention may contain a Th2 cytokine and/or a nucleic acid encoding a Th2 cytokine in an expressible manner. Such aTh2 cytokine may be the full length (wild-type) polypeptide or a partial peptide as long as it retains the activity. For example, deletion of N-terminal and/or C-terminal amino acids (for example, 1 to 30 amino acids, specifically, 1, 2, 3, 4, 5, 10, 15, 20, or 25 amino acids) does not likely affect cytokine activity. Furthermore, the N-terminal signal sequence can be appropriately replaced by another signal sequence. Alternatively, the cytokine may be expressed as a fused protein with other peptides. The nucleic acid encoding a Th2 cytokine may be inserted in an expression vector, or connected to an appropriate promoter, such as actin promoter, EF1 promoter, CMV promoter, CAG promoter, and a like. The nucleic acid may be contained in a minus strand RNA viral vector or any other expression vectors. When a Th2 cytokine is encoded in a minus strand RNA viral vector, the Th2 cytokine may be encoded in the same vector as Aβ, alternatively, the Th2 cytokine may be encoded in another minus strand RNA viral vector. Specifically, when an Aβ-encoding minus strand RNA viral vector of the present invention does not encode a Th2 cytokine, Th1/Th2 balance can be shifted to Th2 by co-administering a Th2 cytokine or a Th2 cytokine vector in combination with the Aβ-encoding vector of the invention. For example, the Th2 cytokines that can be used are IL-4, IL-5,1L-6,1L-9, IL-10, and IL-13, or a combination thereof. The Th2 cytokine is preferably selected from a group consisting of IL-4, IL-10, and IL-13.

Compositions of the present invention may also comprise an adjuvant. Specifically, even when a vector of the present invention does not encode a Th2 cytokine, Th1/Th2 balance can be shifted to Th2 using a composition comprising a Th2 adjuvant. A Th2 adjuvant refers to an adjuvant that predominantly activates type 2 helper T cells (Th2 cells) over type 1 helper T cells (Th1 cells). Specifically, the adjuvant includes aluminum hydroxide (alum), cholera toxin (B subunit), protein extract from Schistosoma mansoni eggs (for example, Lacto-N-fucopentaose III), and so on (Grun, J. L. and P. H. Maurer, Cellular Immunol. 121: 134-145, 1989; Holmgren, J. et al., Vaccine 11:1179-1184, 1993; Wilson, A.D. et al., Vaccine 11: 113-118, 1993; Lindsay, D.S. et al, Int. Arch. Allergy Immunol. 105: 281-288, 1994; Xu-Amano, J. et al., J. Exp. Med. 178: 1309-1320, 1993; Okano, M. et al., J. Immunol. 167(1): 442-50, 2001). Compositions of the present invention may contain any other ingredients. For example, they may contain a marker for monitoring administration, or one or more compounds such as immune system-regulators, signal transduction regulators, cytokines, hormones, receptors, antibodies, and fragments thereof. Compositions of the present invention may contain one or more vectors encoding one or more polypeptides such as signal transduction regulators, signal transduction regulators, cytokines, hormones, receptors, antibodies, and fragments thereof.

Administration of a minus strand RNA virus thus generated or a composition comprising the virus to individuals induces an immune response to Aβ and thereby reduces Aβ deposition. The vector may be administered in combination with a Th2 cytokines, a Th2 cytokine-expressing vector, or a Th2 adjuvant. The vector administration may be an *in vivo* administration or a cell-mediated *ex vivo* administration. The minus strand RNA viral vector used in inoculation does not need to be infectious virions, and may be non-infectious virions, virus core (an RNP complex comprising the genome and genome-binding viral proteins), or the like, as long as it has the ability to express antigen polypeptide genes carried by the genome RNA. Herein, the minus strand RNA viral vector refers to a complex that comprises a ribonucleoprotein (RNP) complex containing a genome RNA derived from a minus strand RNA virus and viral proteins required for replication of the genome RNA and expression of the genes carried by the virus, wherein the complex replicates the genome RNA in the infected cell and expresses the carried genes. The RNP is, for example, a complex comprising genome RNAs and N, L, and Proteins of a minus strand RNA virus. Specifically, minus strand RNA viral vectors of the present invention include infectious virions, non-infectious virions (also referred to as virus-like particles (VLPs)), and RNPs comprising the genome RNAs and viral proteins that bind to the genome RNA such as nucleocapsids of a minus strand RNA virus. When introduced into cells, even an RNP (viral core) in which the envelope is removed from the virion can replicate the viral genome RNA in the cells (WO 97/16538; WO 00/70055). RNPs or VLPs are desirably used for inoculation, for example, in combination with a transfection reagent or the like (WO 00/70055; WO 00/70070).

When a vector inoculation is via cells, a minus strand RNA viral vector is introduced into appropriate culture cells, or cells collected from an animal subject of the inoculation, or such. When infecting cells with a minus strand RNA virus *in vitro* (for example, in a test tube or dish), the infection can be performed, for example, *in vitro* (or *ex vivo*) in a desired physiological solution, such as a culture medium or physiological saline. MOI (multiplicity of infection; i.e., the number of infected viruses per cell) preferably ranges from 1 to 1000, more preferably from 2 to 500, even more preferably from 3 to 300, still more preferably from 5 to 100. Only a short period of contact between a minus strand RNA virus and cells is sufficient. The contact may be, for example, 1 minute or longer, preferably 3 minutes or longer, 5 minutes or longer, 10 minutes or longer, or 20 minutes or longer, such as 1 to 60 minutes, more specifically about 5 to 30 minutes. Alternatively, the contact may take place over a longer period of time, for example, several days or longer.

RNPs or non-infectious virions (virus-like particles (VLPs)) comprising a viral genome RNA can be introduced into cells by conventional transfection methods. Specifically, transfection to cells can be achieved by various techniques known to those skilled in the art, such as calcium phosphate methods (Chen, C. & Okayama, H., BioTechniques 6:632-638, 1988; Chen, C. and Okayama, H., Mol. Cell. Biol. 7: 2745, 1987), DEAE-dextran methods (Rosenthal, N., Methods Enzymol. 152:704-709, 1987), various liposome-based transfection reagents (Sambrook, J. et al., Molecular Cloning: A Laboratory Manual, Cold Spring Harbor Laboratory, Cold Spring Harbor, NY, 1989), and electroporation methods (Ausubel, F. et al., In Current Protocols in Molecular Biology, John Wiley and Sons, NY, Vol. 1, Ch. 5 and 9, 1994). Chloroquine may be added in the transfection to suppress endosomal degradation (Calos, M. P., Proc. Natl. Acad. Sci. USA 80: 3015, 1983). Transfection reagents include DOTMA (Roche), Superfect Transfection Reagent (QIAGEN, Cat No. 301305), DOTAP, DOPE, DOSPER (Roche #1811169), TransIT-LT1 (Mirus, Product No.MIR 2300), CalPhos^{™} Mammalian Transfection Kit (Clontech #K2051-1), CLONfectin^{™} (Clontech #8020-1) and such. Enveloped viruses are known to take up host cell-derived proteins during viral particle formation. Such proteins may have some antigenicity and cause cytotoxicity when introduced into cells (J. Biol. Chem., 272: 16578-16584, 1997). Thus, it is advantageous to use envelope-free RNPs (WO 00/70055).

Viral RNPs can be formed directly in cells by introducing an expression vector of a viral genome RNA and expression vectors encoding viral proteins (N, P, and L proteins) required for replication of the genome RNA into cells. Cells to which viral vectors have been introduced may be prepared by such a procedure.

Once prepared, the cells to which a minus strand RNA viral vector has been introduced may be cultured for about 12 hours to 5 days (preferably for 1 to 3 days) to express an Aβ peptide. The harvested cells can be used directly or as cell lysates to inoculate to animals; however, Aβ peptide-expressing cells are preferably used for inoculation. Aβ having a signal peptide may be expressed from the vector and secreted to the outside of the cell. Lysates of cells introduced a vector can be prepared by a procedure that lyses the cell membrane with a detergent or a procedure that involves repetition of freeze-thaw cycles. Non-ionic detergents, such as Triton X-100 and Nonidet P-40, can be applied within the concentration range of 0.1 to 1%. Such lysates can be obtained by, for example, collecting cell cluster with centrifugation after PBS washing and re-suspension in TNE buffer [25 mM Tris-HCl (pH7.5), 150 mM NaCl, I mM EDTA, 1% Nonidet P-40], and incubating the cell suspension allowed to stand on ice for 10 to 30 minutes. If the protein to be used as an antigen is soluble in the cytoplasm, the prepared lysates can be centrifuged (10,000x g for 10 minutes) to remove the unnecessary insoluble fraction as precipitates and the resulting supernatant can be used for immunization. If a lysate is administered at a site where detergents are undesirable, the lysate may be prepared by re-suspending the cells in PBS after washing and disrupting the cells through 5 to 6 freeze-thaw cycles. The lysate may be administered in combination with a transfection reagent.

Alternatively, immunity can be induced by administering nucleic acids which produce an Aβ peptide-encoding minus strand RNA viral vector to an animal, and producing the Aβ peptide expressed by the minus strand RNA viral vector in the body of the animal. The nucleic acids generating a minus strand RNA viral vector refer to a set of nucleic acids that allow the expression of RNAs as well as the expression of a group of proteins required for the production of the recombinant minus strand RNA viral vector. Specifically, the nucleic acids refer to a set of nucleic acids comprising a nucleic acid encoding the genome RNA of an Aβ-encoding minus strand virus or a complementary strand thereof (antigenome RNA; i.e., plus strand), as well as nucleic acids encoding a group of viral proteins that construct an RNP comprising the genome RNA of the minus strand RNA virus. Such nucleic acids comprise an appropriate promoter to express the encoded RNAs or proteins. Such promoters include, for example, CMV promoters (Foecking, M.K, and Hofstetter H., Gene 45: 101-105, 1986), retroviral LTRs (Shinnik, T. M. et al., Nature, 293: 543-548, 1981), EF 1 promoters, and CAG promoters (Niwa, H. et al., Gene. 108: 193-199, 1991. JP-A No. H3-168087). Plasmid vectors can be suitably used as nucleic acids. The genome of a minus strand RNA virus preferably carries the genes encoding a group of viral proteins (typically N, L, and P) that construct the RNP comprising the genome RNA, as well as all the genes of envelope-constituting proteins of the parental wild-type virus. If a minus strand RNA virus genome lacks any gene(s) of the envelope-constituting proteins, the nucleic acids encoding the envelope protein(s) (for example, the envelope protein(s) lacked by the genome, or other envelope protein(s), such as VSV-G) that complement the formation of infectious virions may be included in the set of nucleic acids described above. Administration of these nucleic acids to an animal results in the production of the recombinant minus strand RNA viruse in the animal. A group of viral proteins that construct an RNP comprising the genome RNA of a minus strand RNA virus refer to the proteins that form an RNP together with the viral genome RNA, and constitute the nucleocapsid. These are a group of proteins required for genome replication and gene expression, and typically include N, P, and L proteins. Specifically, the present invention also relates to methods comprising the step of inoculating (i) a nucleic acid encoding the genome RNA of an Aβ-encoding minus strand RNA virus or a complementary strand thereof; and (ii) nucleic acids encoding proteins N, P, and L to an animal. In this animal, an RNP comprising the genome RNA is formed and an Aβ peptide is expressed. The genome may further encode a Th2 cytokine.

Alternatively, inoculation of cells or lysates thereof, to which a nucleic acid encoding the genome RNA or a complementary strand thereof and a group of viral proteins constituting the above-described RNP are introduced previously, may also be performed. See the following documents for more detailed information on the nucleic acids generating a minus strand RNA viral vector:
WO 97/16539; WO 97/16538; WO 00/70055; WO 00/70070; WO 01/18223; WO 03/025570; Durbin, A. P. et al., Virology 235: 323-332, 1997; Whelan, S. P. et al., Proc. Natl. Acad. Sci. USA 92: 8388-8392, 1995; Schnell. M. J. et al., EMBO J. 13: 4195-4203, 1994; Radecke, F. et al., EMBO J. 14: 5773-5784, 1995; Lawson, N. D. et al., Proc. Natl. Acad. Sci. USA 92: 4477-4481, 1995; Garcin, D. et al., EMBO J. 14: 6087-6094, 1995; Kato, A. et al., Genes Cells 1: 569-579, 1996; Baron, M. D. and Barrett, T., J. Virol. 71: 1265-1271, 1997; Bridgen, A. and Elliott, R. M., Proc. Natl. Acad. Sci. USA 93: 15400-15404, 1996; Hasan, M. K. et a/., J. Gen. Virol. 78: 2813-2820, 1997; Kato, A. et al., EMBO J. 16: 578-587, 1997; Yu, D. et al., Genes Cells 2: 457-466, 1997; Tokusumi, T. et al., Virus Res. 2002: 86; 33-38, 1997; Li, H.-O. et al., J. Virol., 74: 6564-6569, 2000.

When an animal is inoculated with nucleic acids generating a minus strand RNA viral vector, a nucleic acid encoding the genome RNA of an Aβ-encoding minus strand RNA virus or a complementary strand thereof, and nucleic acids encoding N, P, and L proteins may be introduced into the animal at a weight ratio of 5:0.5:0.5:2. The nucleic acid ratio may be adjusted suitably. For example, when expression plasmids are used, the administration can be carried out using 5 to 1000 µg of a plasmid encoding an Aβ-encoding viral genome RNA (plus or minus strand), and 0.5 to 200 µg of an N-expressing plasmid, 0.5 to 200 µg of a P-expressing plasmid, and 2 to 500 µg of an L-expressing plasmid. Administration of the nucleic acids can be performed, for example, by injecting naked DNAs directly or after being combined with a transfection reagent. The transfection reagent includes, for example, lipofectamine and polycationic liposomes. Specifically, DOTMA (Roche), Superfect (QIAGEN #301305), DOTAP, DOPE, DOSPER (Roche #1811169), TransIT-LT1 (Mirus, Product No.MIR 2300), or such can be used.

The dosage of a vector of the present invention to be administered varies depending on the type of disease, body weight, age, sex and symptoms of a patient, purpose of administration, dosage form in which a composition is introduced, method of administration, type of gene to be introduced, and so on. One skilled in the art can determine the appropriate dosage. The administration route can be suitably selected, and include transdermal, intranasal, transbronchial, intramuscular, intraperitoneal, intravenous, and subcutaneous routes, without being limited thereto. The particularly preferred routes are intramuscular injection (for example, into the gastrocnemial muscle), subcutaneous injection, intranasal dropping or spray, intradermal injection into palm or sole, direct injection into the spleen, intraperitoneal injection, and such. There may be one or more inoculation sites, for example, 2 to 15 sites. The inoculation dosage may be suitably adjusted depending on the animal subject, inoculation site, and inoculation frequency, and the like. It is preferable to administer the vector in combination with a pharmaceutically acceptable carrier at a dose within a range of about 10⁵ to about 10¹¹ CIU/ml, more preferably about 10⁷ to about 10⁹ CIU/ml, most preferably about 1 x 10⁸ to about 5x 10⁸ CIU/ml. In terms of the virus titer, a single dose for human ranges from 1x 10⁴ CIU to 5x 10¹¹ CIU (cell infectious unit), and preferably from 2x 10⁵ CIU to 2x 10¹⁰ CIU. The frequency of administration is once, or several times as long as the side effects are within a clinically acceptable range. The number of administrations per day may also be determined in the same manner. Although a single dose produces significant effects, stronger effects can be obtained by introducing a vector more than once.

When a vector is administered more than once, for example, the second administration may be carried out approximately one year after the first administration. The preferred route of administration is, for example, intranasal or intramuscular administration. The administration may be repeated for stronger effects. Inoculation of the above-mentioned minus strand RNA viral vector carrying an Aβ-encoding nucleic acid, nucleic acids generating the viral vector, cells introduced with the viral vector or the nucleic acids generating the vector, or lysates of the cells may be preferably used for multiple-dose administration.

When a vector is inoculated *via* cells (*ex vivo* administration), for example, human cells, and preferably autologous cells are infected with the minus strand RNA viral vector, and 10⁴ to 10⁹ cells, and preferably 10⁵ to 10⁸ cells, or lysates thereof, can be used. The vector can also be used to inoculate to non-human animals, and the dosage of administration can be converted from the above-described dosage based on the weight or volume ratio of the target site of administration (for example, an average value) of a subject animal to that of human. The subject for administration of the compositions comprising a vector of the present invention includes desired vertebrates (humans and non-human vertebrates) having an immune system, preferably birds and mammals, more preferably mammals (including humans and non-human mammals). Specifically, the mammals include humans and non-human primates such as monkeys, rodents such as mice and rats, and all other mammals as rabbits, goats, sheep, pigs, cattle, and dogs. The target animals for administration include, for example, individuals with Alzheimer's disease, individuals with an elevated level of Aβ, individuals with enhanced Aβ deposition, individuals carrying an Alzheimer-type mutant gene, and so on.

Immune responses against Aβ are induced by the methods of the present invention. Immune responses against Aβ can be confirmed by anti-Aβ antibody production, decrease of the amount of Aβ in brain tissues, and decrease in Aβ deposition, or the like. The production of anti-Aβ antibody can be evaluated by detection of the anti-Aβ antibody in blood samples. The antibody level can be measured with an ELISA (enzyme-linked immunosorbent assay) or Ouchterlony's method. An ELISA method is carried out, for example, by attaching an antigen to a microplate, preparing an antiserum and making a series of 2-fold dilutions of the prepared antiserum (starting solution 1:1000), and adding the diluted antisurum to the plate for antigen-antibody reaction to take place. Then, for color development, the antibody of the immunized animal is reacted with a xenogenic antibody, which has been enzymatically labeled with peroxidase and serves as a secondary antibody. The antibody titer can be calculated based on the dilution fold of the antibody when the absorbance is one half of the maximum color absorbance Alternatively, in Ouchterlony's method, antigens and antibodies diffuse within an agar gel, and white precipitation lines are formed as a result of immunoprecipitation reactions. The precipitation lines can be used to determine the antibody titer, which is the dilution fold of the antiserum when the immunoprecipitation reaction occurs. Aβ level in the brain tissue can be determined, for example, using brain tissue extracts and a BioSource ELISA kit or the like.

The effect of reducing Aβ deposition (senile plaques) can be determined, for example, by the following procedure: treating brain tissue sections with 70% formic acid and after inactivating endogenous peroxidase with 5% H₂O₂, reacting the sections with an anti-Aβ antibody and performing DAB stain using a peroxidase-labeled secondary antibody. After the staining, the area of Aβ accumulated site can be determined by microscopic observation, If the area fraction of the accumulated site decreased in comparison with that when a vector of the present invention was not administered, the level of Aβ deposition can be judged as reduced. Alternatively, senile plaques in a living subject can be observed using MRI after intravenous administration of such a compound as 1-fluoro-2,5-bis-(3-hydroxycarbonyl-4-hydroxy) styrylbenzene (FSB), which has an affinity to amyloid or such (Higuchi M et al., Nat. Neurosci. 8(4):527-33, 2005; Sato, K. et al., Eur. J. Med. Chem. 39: 573, 2004; Klunk, W. E. et al., Ann. Neurol. 55(3):306-19, 2004). Effects of a vector of the present invention can be confirmed by such a non-invasive imaging technique for amyloids.

The present invention also relates to methods of determining immune responses to amyloid β, which comprise steps of: introducing a vector of the present invention or a composition comprising the vector into a subject with amyloid β deposition and of detecting anti-amyloid β antibody in the subject. The present invention also relates to methods of measuring amyloid β deposition, which comprise steps of: administering a vector of the present invention or a composition comprising the vector to a subject with amyloid β deposition and of detecting the level of amyloid β deposition in the subject. These methods allow immune responses to amyloid β and/or effects of reducing amyloid β deposition to be monitored.

### BRIEF DESCRIPTION OF THE DRAWINGS

Fig. 1 is a diagram showing the process of constructing Litmus SalI/NheIfrg-MtsHNtsPLmut-dF mIL 10 (hereinafter abbreviated as Litmus TS-dF mIL10). Mutations were introduced into the F gene-deleted site of Litmus TS-dF to create a *Not*I site, into which the mIL-10 gene excised using *Not*I was inserted.
Fig. 2 is a diagram showing the process of constructing pSeV18+Aβ/MtsHNtsP511 Lmut-dF (hereinafter abbreviated as pSeV18+Aβ/TS-dF). The Aβ gene, inserted into the *Not*I site of pBluescript^{™}, was excised and inserted into the *Not*I site of pSeV 18+NotI/TS-dF.
Fig. 3 is a diagram showing the process of constructing pSeV18+Aβ/MtsHNtsP511 Lmut-dF mIL10 (hereinafter abbreviated as pSeV18+Aβ/TS-dF mIL10). The necessary fragments were excised from pSeV18+Aβ/TS-dF and Litmus TS-dF mIL10 using *Sal*I and *Nhe*I*,* and switched.
Fig. 4 is a graph showing the level of serum anti-Aβ antibody of APP transgenic mice 4 and 8 weeks after the intranasal administration of SeV-Aβ1-43/mIL-10 or the control vector.
Fig. 5 is a photograph showing senile plaques in the frontal lobe of APP transgenic mice 8 weeks after the intranasal administration of SeV-Aβ-43/mIL-10 or the control vector.
Fig. 6 is a photograph showing senile plaques in the parietal lobe of APP transgenic mice 8 weeks after the intranasal administration of SeV-Aβ1-43/mIL-10 or the control vector.
Fig. 7 is a photograph showing senile plaques in the hippocampus of APP transgenic mice 8 weeks after the intranasal administration of SeV-Aβ1-43/mIL-10 or the control vector.
Fig. 8 is a graph showing the result of quantifying senile plaques in APP transgenic mice (frontal lobe, parietal lobe, and hippocampus) 8 weeks after the intranasal administration of SeV-Aβ1-43/mIL-10 or the control vector.
Fig. 9 shows the result of examining lymphocyte infiltration in the central nervous system in APP transgenic mice 8 weeks after the intranasal administration of SeV-Aβ1-43/mIL-10 or the control vector. T cell infiltration in the frontal lobe was detected using CD3 as an indicator. Microglial alteration in the hippocampus was detected using Iba-1 as an indicator.
Fig. 10 is a set of graphs showing the amount of Aβ in the brain tissue of APP transgenic mice 8 weeks after the intranasal administration of SeV-Aβ1-43/mIL-10 or the control vector.
Fig. 11 is a graph showing the level of serum Aβ antibody of APP transgenic mice 4 and 8 weeks after the intramuscular administration of SeV-Aβ1-43/mIL-10 or the control vector.
Fig. 12 is a photograph showing senile plaques in the frontal lobe of APP transgenic mice 8 weeks after the intramuscular administration of SeV-Aβ1-43/mIL-10 or the control vector.

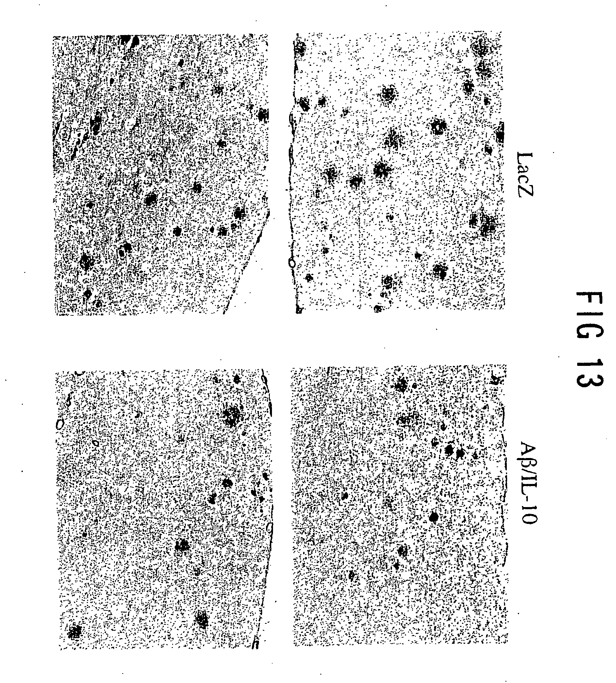

Fig. 13 is a photograph showing senile plaques in the parietal lobe of APP transgenic mice 8 weeks after the intramuscular administration of SeV-Aβ1-43/mIL-10 or the control vector.

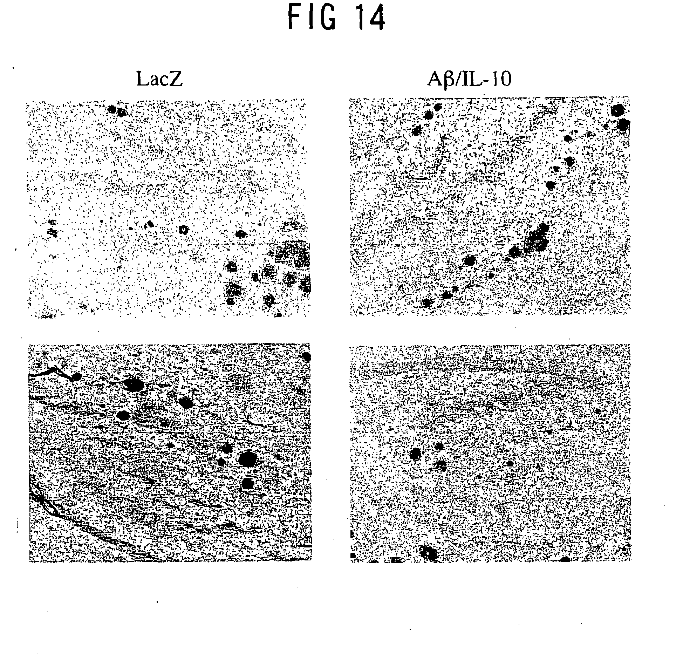

Fig. 14 is a photograph showing senile plaques in the hippocampus of APP transgenic mice 8 weeks after the intramuscular administration of SeV-Aβ1-43/mIL-10 or the control vector.
Fig. 15 is a graph showing the result of quantifying senile plaques in APP transgenic mice (frontal lobe, parietal lobe, and hippocampus) 8 weeks after the intramuscular administration of SeV-Aβ1-43/mIL-10 or the control vector.
Fig. 16 is a photograph showing the result of examining lymphocyte infiltration in the frontal lobe 8 weeks after the intramuscular administration of SeV-Aβ1-43/mIL-10 or the control vector.
Fig. 17 is a set of graphs showing the amount of Aβ in the brain tissue 8 weeks after the intramuscular administration of SeV-Aβ1-43/mIL-10 or the control vector.
Fig. 18 is a graph showing the level of serum anti-Aβ antibody of normal mice after the intranasal administration of SeV-Aβ1.43/mIL-10.
Fig. 19 is a graph showing the level of serum anti-Aβ antibody of normal mice after the intranasal administration of *Sendai virus* therapeutic vectors encoding cytokines.

### BEST MODE FOR CARRYING OUT THE INVENTION

Herein below, the present invention will be specifically described using Examples, however, it is not to be construed as being limited thereto. All documents cited in the instant specification are incorporated by reference.

### EXAMPLE 1. Construction of a minus strand RNA viral genome cDNA carrying an Aβ gene

### (1-1) Construction of NotI fragment (addition of a transcription signal of a minus strand RNA virus)

PCR was performed using as a template, a gene (JP-A No. 2005-21149; SEQ ID NO: 1) encoding an Aβ peptide (SEQ ID NO: 28) which has been made into a secretory type by adding the secretion signal (amino acids 1 to 18) of an amyloid precursor protein (APP: Accession number AF282245) to a human Aβ peptide 1-43 (SEQ ID NO: 27), and two primers, phAbeta-NnotI (SEQ ID NO: 19) and phAbeta-CnotI (SEQ ID NO: 20). The resulting PCR products were digested with *Not*I*,* and then subcloned into pBluescript^{™} II KS (Stratagene) to construct a *Not*I fragment of the Aβ peptide gene comprising a *Sendai virus* transcription signal (SEQ ID NO: 21). Similarly, for mouse IL-10 (mIL10), PCR was performed using mIL10 cDNA (Accession number NM_010548; SEQ ID NO: 2) as a template, and two primers pmIL10-N (SEQ ID NO: 22) and pmIL10-C (SEQ ID NO: 23). The resulting PCR products were digested with *Not*I*,* and then subcloned into pBluescript^{™} II KS (Stratagene) to construct a *Not*I fragment (SEQ ID NO: 24) of the mIL10 gene comprising a *Sendai virus* transcription signal. SEQ ID NO:19 ATTGCGGCCGCCAAGGTTCACTTATGCTGCCCGGTTTGGCACTGCTCCTG SEQ ID NO:22
ACTTGCGGCCGCCAAAGTTCAATGCCTGGCTCAGCACTGCTATGCTGCCTG

### (1-2) Insertion of mIL10 NotI fragment into F gene-deleted site

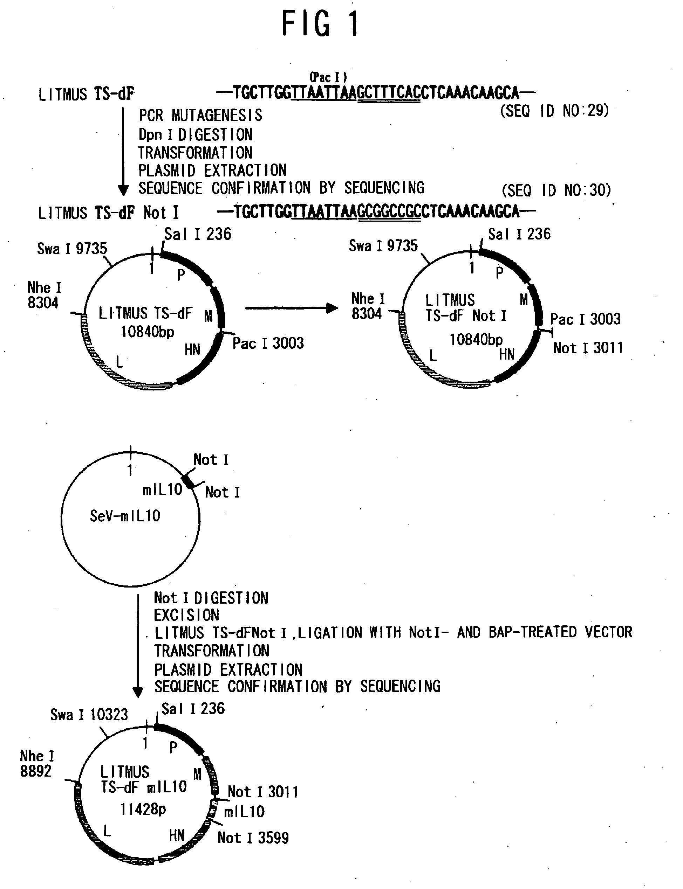

The cDNA (pSeV18+NotI/TSΔF) of an F gene-defective SeV vector (WO 2003/025570), to which amino acid mutations at 9 spots (M protein, G69E, T116A and A183S; HN protein, A262T, G264R and K461 G; P protein, L511F; L protein, N1197S and K1795E) had been introduced, was digested with *Sal*I and *Nhe*I and subcloned into Litmus38 (New England Biolabs). Then, a mutation to insert a *Not*I cleavage site was introduced into the F gene-deleted site of the resulting Litmus TSΔF plasmid comprising M and HN genes. Specifically the insertion of the *Not*I cleavage site (Fig 1, top drawing) was carried out by PCR using the Litmus TSΔF as a template and two primers F_lit3008_NotI (SEQ ID NO: 25) and R_lit3020_NotI (SEQ ID NO: 26). After the resulting Litmus TSΔF NotI was digested with *Not*I*,* the mIL10 gene *Not*I fragment was inserted (Fig 1, bottom drawing).
SEQ ID NO:25 AAGCGGCCGCCTCAAACAAGCACAGATCATGGATG
SEQ ID NO:26 AGGCGGCCGCTTAATTAACCAAGCACTCACAAGG

### (1-3) Construction of F gene-defective SeV cDNA carrying Aβ gene

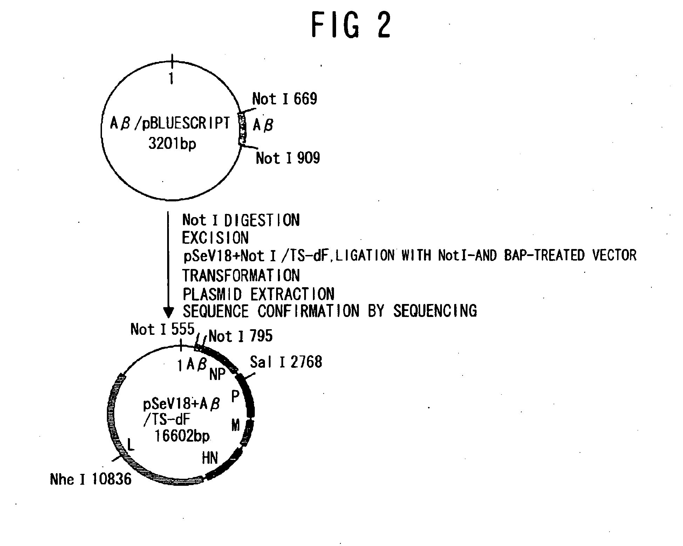

The Aβ peptide gene *Not*I fragment was inserted into the *Not*I site on pSeV18+NotI/TSΔF, which had been digested with *Not*I*,* to construct an F gene-defective SeV cDNA carrying the Aβ gene (psev18+Aβ/TSΔF) (Fig. 2).

### (1-4) Construction of F gene-defective SeV genome cDNA carrying both Aβ and IL-10 genes

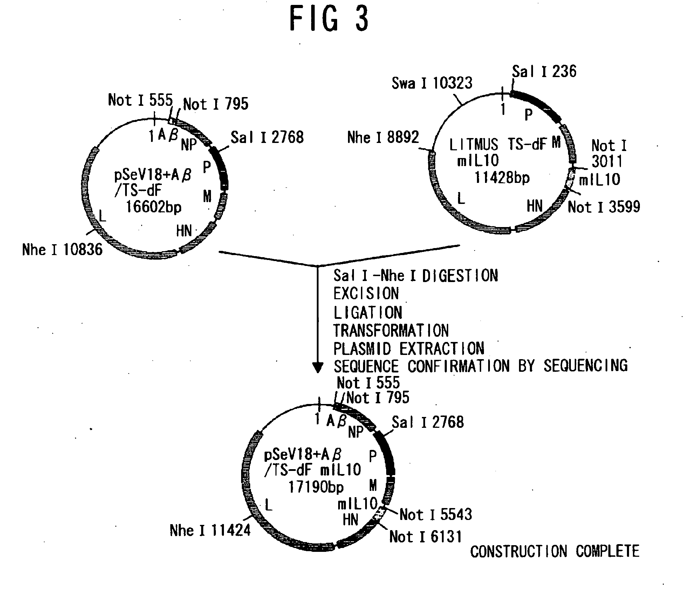

After digesting pSeV8+Aβ/TSΔF with *Sal*I and *Nhe*I*,* a fragment not comprising M and HN genes was recovered. Meanwhile, Litmus TSΔF-mIL10 was digested with *Stal*I and *Nhe*I to prepare a fragment comprising both M and HN genes. The two fragments were ligated to construct an F gene-defective SeV genome cDNA carrying both Aβ and IL-10 genes (pSeV18+Aβ/TSAF-mIL10) (Fig. 3).

### EXAMPLE 2. Reconstruction and amplification of Sendai virus vector

Viral reconstruction and amplification were carried out with the method reported by Li *et al.* (Li, H.-O. et al., J. Virology 74: 6564-6569, 2000; WO 00/70070) and its modified method (PCT/JP2005/00705). Since the viral vector is F gene-defective, a helper cell for supplying F protein was used. The helper cells were prepared using a Cre/loxP induced expression system. The system uses a plasmid pCALNdLw (Arai, T. et al., J. Virol. 72: 1115-1121, 1988), which is designed to induce expression of gene products by Cre DNA recombinase, and a recombinant adenovirus (AxCANCre) expressing Cre DNA recombinase to infect the transformant, which has been transformed with the pCALNdLw plasmid, according to the method of Saito *et al.* (Saito, I. et al., Nucl. Acid Res. 23: 3816-3821, 1995; Arai, T. et al., J. Virol. 72: 1115-1121, 1998).

The F gene-defective SeV vector carrying an Aβ gene (SeV18+Aβ/TSΔF) and the F gene-defective SeV vector carrying both Aβ and IL-10 genes (Sev18+Aβ/TSΔF-mIL10) were prepared by the methods described above. The DNA sequence encoding the plus strand of SeV18+Aβ/TSΔF-mIL10 is shown in SEQ ID NO: 4, and the nucleotide sequence of the genome RNA (minus strand) is shown in SEQ ID NO: 5. A SeV vector carrying LacZ gene instead of the Aβ and IL-10 genes in the F gene-deleted site (SeV18+ LacZ /TSΔF; also referred to as "SeV LacZ") was prepared as a control.

### EXAMPLE 3. Effect of intranasally administered SeV-Aβ1-43/ mIL10 in an Alzheimer's disease animal model

### (3-1) Animals and method of administration

Effect of SeV18+Aβ/TSΔF-mIL10 of the present invention (hereinafter also referred to as "SeV-Aβ1-43/mIL-10") was tested using 24- to 25-month-old APP transgenic mice (Tg2576) (Hsiao, K., et al., Science 274:99-102, 1996). The mice were divided into two groups, the treatment group and control group, each consisting of four mice. SeV-Aβ1-43/mIL-10 (5x 10⁶ CIU/head) was administered intranasally to the treatment group, and SeV LacZ (5x 10⁶ CIU/head) was administered intranasally to the control group.

### (3-2) Determination of anti-Aβ antibody in serum

Blood was collected from the mice 4 and 8 weeks after the above-described treatment to determine the amount of anti-Aβ42 antibody present in serum. Aβ1-42 peptide (5 µg/mL) was adsorbed onto each well of a 96-well plate (Nunc, MaxiSorp). After blocking with 5% nonfat milk/TBS-T buffer, the mouse serum (500 times diluted) was added and detection was carried out using a peroxidase-labeled anti-mouse IgG antibody. Antibody titer was assessed with an absorbance measurement (O.D.450) using an ELISA reader.

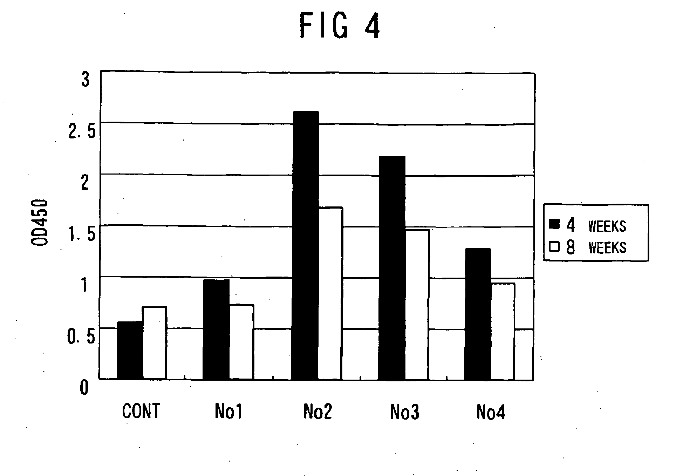

The result is shown in Fig. 4. The antibody titer was found to be markedly elevated in all subjects of the treatment group as compared with the control group.

### (3-3) Senile plaque-eliminating effect of SeV-Aβ1-43/mIL-10

The mice intranasally administered the above-described *Sendai virus* vector were killed 8 weeks after the administration (26 or 27 months old), and the brain tissue sections of the cortex of frontal lobe, parietal lobe, and hippocampus were prepared. The frozen sections of the above-mentioned tissues were treated as described below. To detect Aβ proteins or senile plaques, the tissue sections were treated with 70% formic acid and endogenous peroxidase was inactivated with 5% H₂O₂. Then the tissue sections were reacted with a rabbit anti-pan-Aβ antibody (1000 times diluted), and a peroxidase-labeled secondary antibody was added, followed by DAB staining. The stained sections were observed using a 3CCD camera connected to a microscope, and the area of Aβ accumulation was measured in each region and the area fraction was calculated.

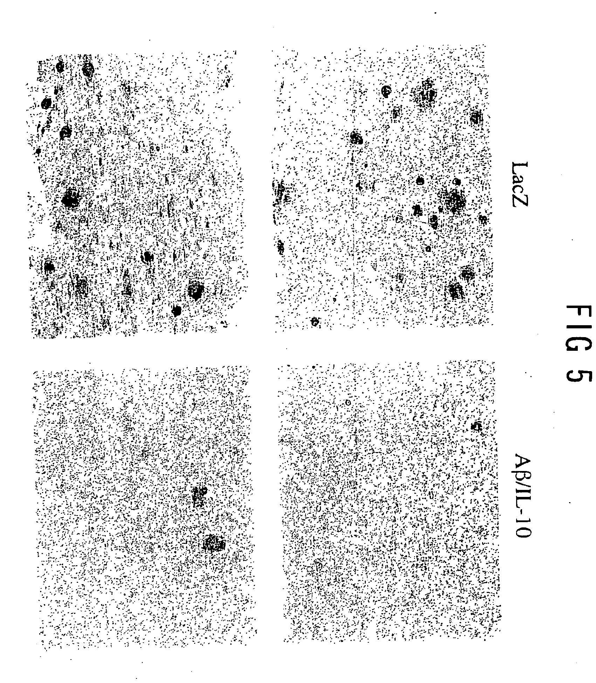

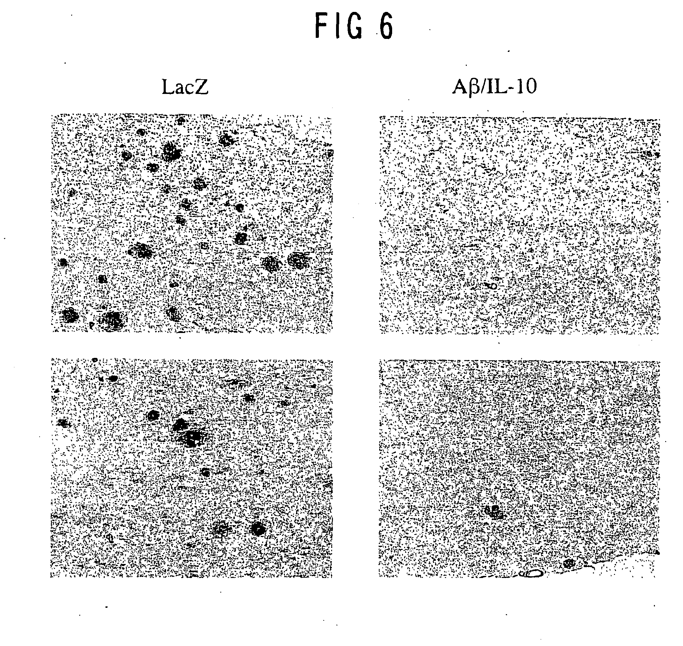

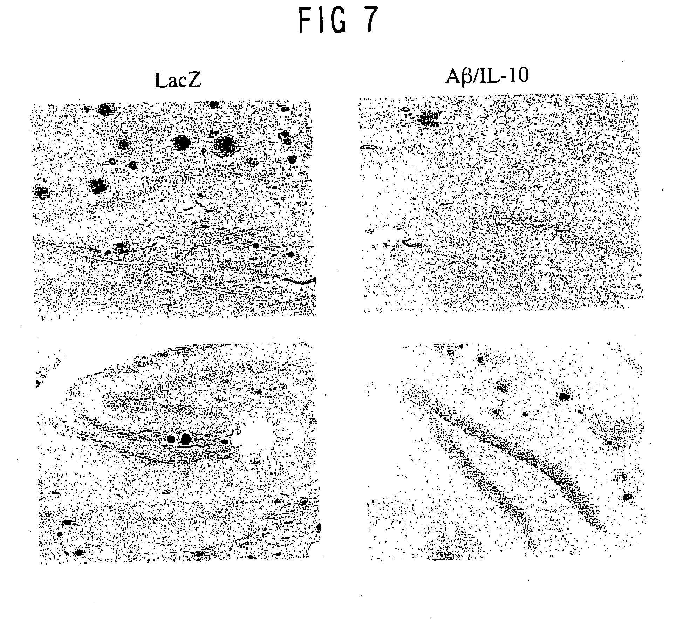

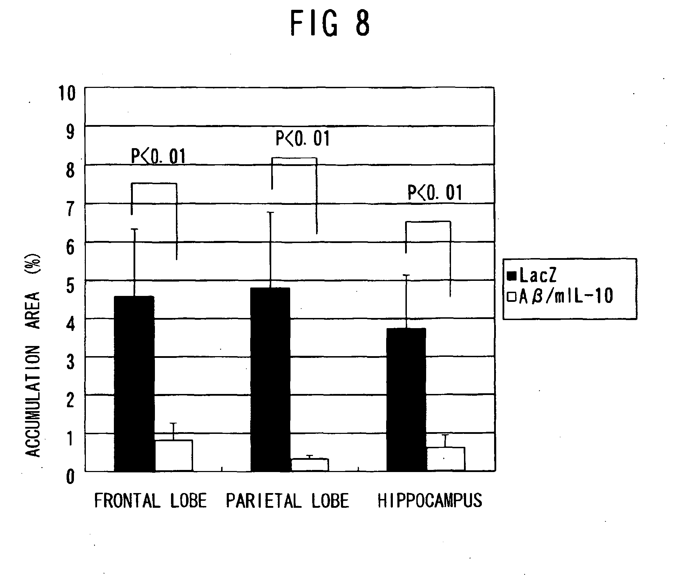

The results of section staining are shown in Fig. 5 (frontal lobe), Fig. 6 (parietal lobe), and Fig. 7 (hippocampus). The area ratio of Aβ deposition is shown in Fig. 8. It was confirmed that SeV-Aβ1-43/mIL-10 administration markedly reduced senile plaques to 10 to 20% of the control in all of the frontal and parietal lobes, and hippocampus.

### (3-4) Safety evaluation of SeV-Aβ1-43/mIL administration

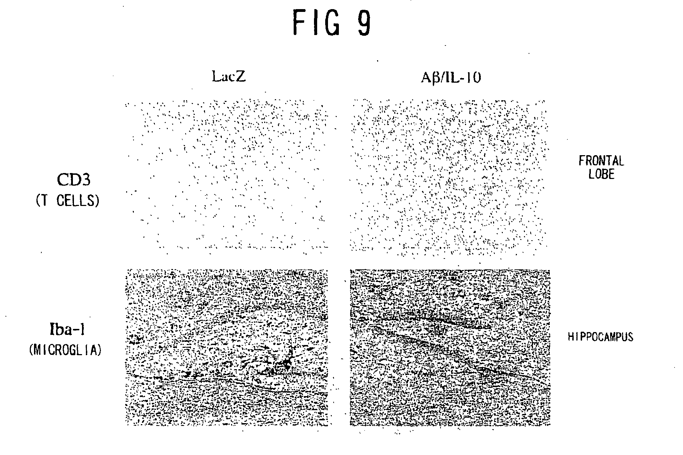

Frozen tissue sections of the frontal lobe and hippocampus were prepared from the treatment group and the control group 8 weeks after the administration (26- to 27-months-old) using the same procedure described above in (3-3). The frozen sections were stained with an ABC method using an anti-CD3 antibody (T cells) and an anti-Iba-1 antibody (microglia) to assess whether lymphocyte infiltration in the central nervous system and microglial alteration took place. As shown in Fig. 9, infiltration of T lymphocytes (cells positive for CD3, which is a pan T cell marker) was not observed in both the frontal lobe and hippocampus in either the treatment group or the control. Thus, the possibility that inflammation of the central nervous system may be caused by using the present vectors for treatment was suggested to be very low. Furthermore, the quantity of cells positive for IBA-1, a microglial marker, was comparable between the control group and the treatment group, confirming that the treatment did not result in an extreme activation (accumulation) in the treatment group.

### (3-5) Determination of Aβ in brain tissues

The cerebrum and cerebellum of the mice were dissected along the fissura mediana, and the hemispheres were snap frozen and stored at -80°C. The brain hemisphere was homogenized in 1 ml of TBS solution, and centrifuged at 100,000 g for one hour using a benchtop ultracentrifuge. The soluble fraction (TBS fraction) was stored. The insoluble fraction was dissolved in 2% SDS and homogenized, and then centrifuged again at 100,000 g for one hour. The resulting soluble fraction (2% SDS fraction) was stored. The insoluble fraction was dissolved in 70% formic acid and homogenized, and then centrifuged again at 100,000 g for one hour. The resulting soluble fraction (formic acid fraction) was stored. Aβ40 and 42 in the brain tissues were determined using a BioSource ELISA kit. The TBS fraction was diluted 4 times; the 2% SDS fraction was diluted 400 to 2000 times; and the formic acid fraction was diluted 1000 times with I M Tris solution. Then, the diluted fractions were further diluted (2 to 10 times) with an ELISA dilution solution for determination.

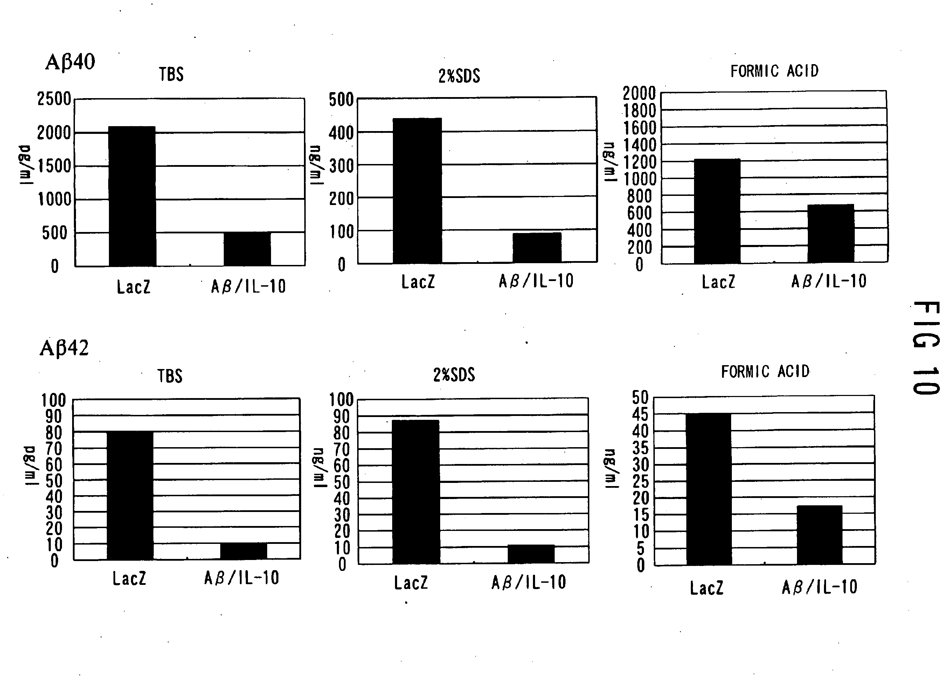

As shown in Fig 10, it was proven that SeV-Aβ1-43/mIL-10 administration markedly reduced the level of Aβ in the brain tissues in all of the SDS, formic acid, and TBS fractions.

### EXAMPLE 4. Effect of intramuscularly administered SeV-Aβ1-43/mIL-10 in an Alzheimer's disease animal model

### (4-1) Animals and method of administration

Except for the administration route, all the procedures were the same as that used in the intranasal administration described above. SeV-Aβ1-43/mIL-10 vector was administered to the hind thigh muscle at a dose of 5 x 10⁶ CIU.

### (4-2) Determination of anti-Aβ antibody in serum

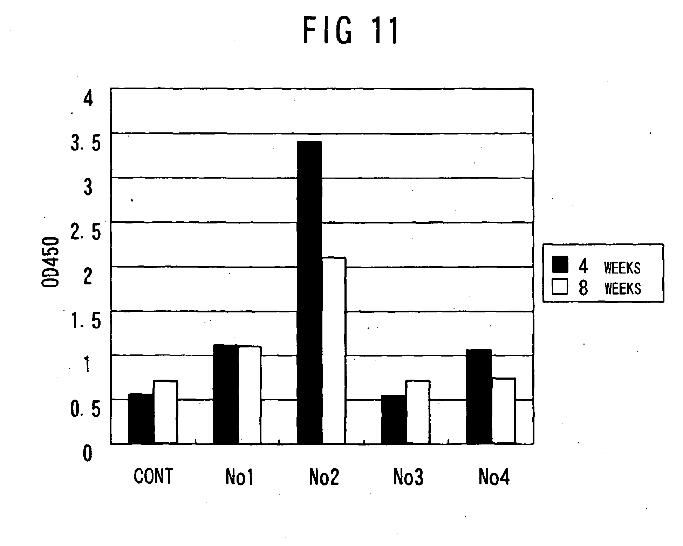

The amount of anti-Aβ antibody in the serum of mice that had been intramuscularly administered SeV-Aβ1-43/mIL-10 vector was determined by the same method as used for the intranasal administration described above. The result is shown in Fig. 11.

### (4-3) Senile plaque-eliminating effect of SeV-Aβ1-43/mIL

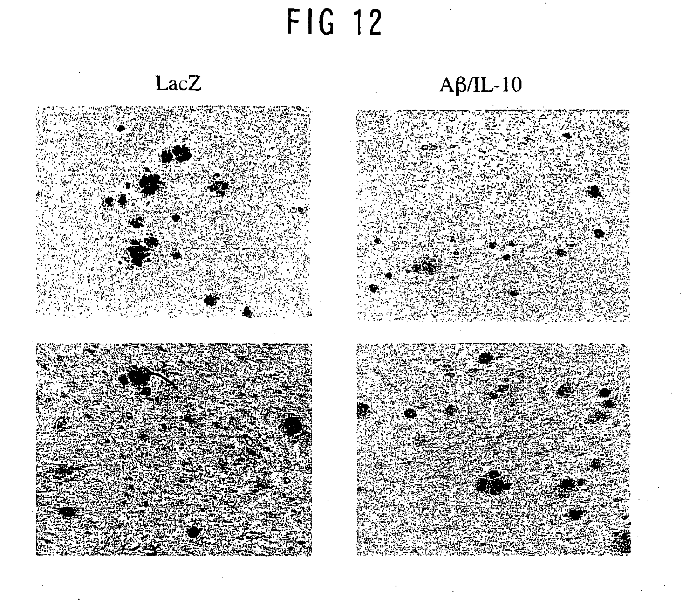

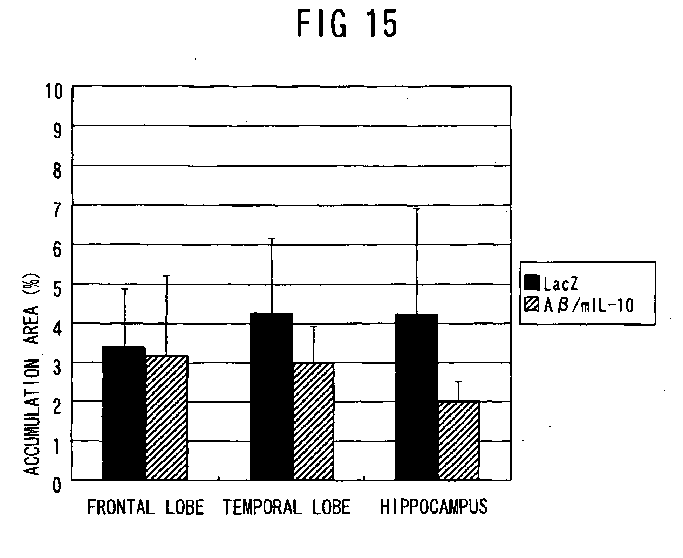

The senile plaque-eliminating effect in the frontal lobe, parietal lobe, and hippocampus after intramuscular administration of SeV-Aβ1-43/mIL-10 vector was assessed by the same method as used for the intranasal administration described above. The results are shown in Figs. 12 (frontal lobe), 13 (parietal lobe), and 14 (hippocampus). The area ratio of Aβ deposition is shown in Fig. 15. Each region showed a certain degree of Aβ deposition reduced.

### (4-4) Safety evaluation of SeV-Aβ1-43/mIL-10 administration

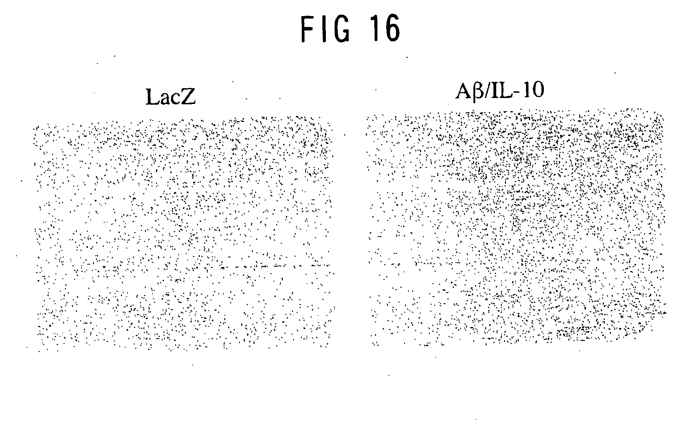

The presence of lymphocyte infiltration in the central nervous system after intramuscular administration of the SeV-Aβ1-43/mIL-10 vector was assessed by the same method as used for the intranasal administration described above. As shown in Fig. 16, infiltration of T lymphocytes in the frontal lobe was not observed in either the control group or the treatment group.

### (4-5) Determination of Aβ in brain tissues

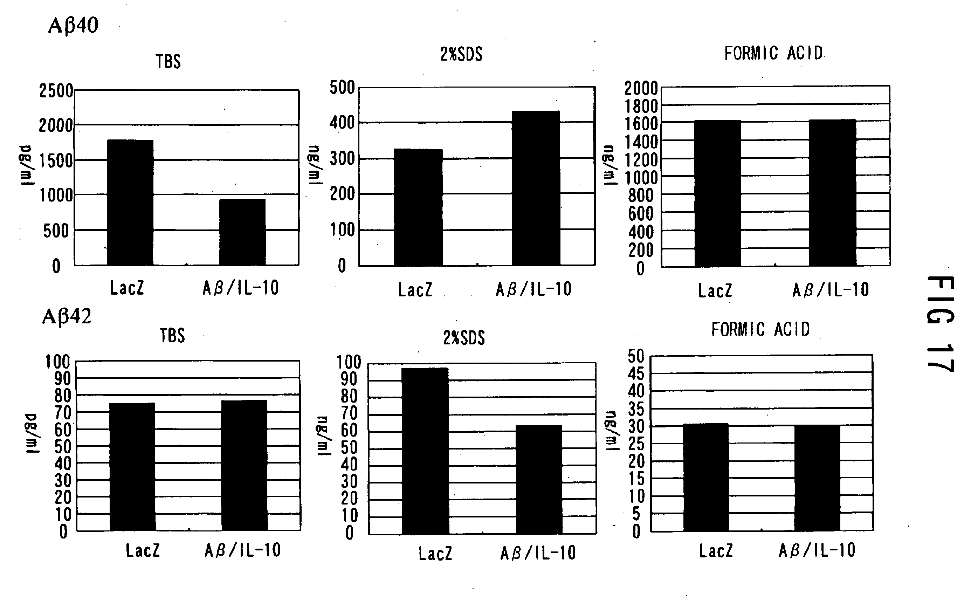

Aβ40 and Aβ42 in brain tissues after intramuscular administration of the SeV-Aβ1-43/mIL-10 vector were determined by the same method as used for the intranasal administration described above. The result is shown in Fig. 17. It was confirmed that the level of Aβ decreased in some soluble fractions.

### EXAMPLE 5. Elevation of the antibody titer by intranasal administration of SeV-Aβ1-43/hIL-10 in normal mice

### (5-1) Animals and method of administration

Effect of SeV18+Aβ1-43/TSΔF-hIL10 (hereinafter also referred to as "SeV-Aβ1-43/hIL-10") of the present invention was tested using 6-week-old C57BL/6N mice. The mice were divided into three groups, each consisting of six mice. SeV-Aβ1-43/hIL-10 (group 1: 5x 10⁵ CIU/head, group 2: 5x 10⁶, group 3: 5x 10⁷ CIU/head) was administered intranasally to each mouse.

### (5-2) Determination of anti-Aβ antibody in plasma

Blood was collected from the mice before and 2, 4 and 8 weeks after the administeration to determine the amount of anti-Aβ42 antibody present in plasma. Aβ1-42 peptide (4 µg/mL) was adsorbed onto each well of a 96-well plate (Nunc, MaxiSorp). After blocking with 1% BSA/2% goat serum/TBS-T buffer, the mouse plasma (300 times diluted) were added and detection was carried out using a peroxidase-labeled anti-mouse IgG antibody. Antibody titer was assessed with an absorbance measurement (O.D.450) using an ELISA reader.

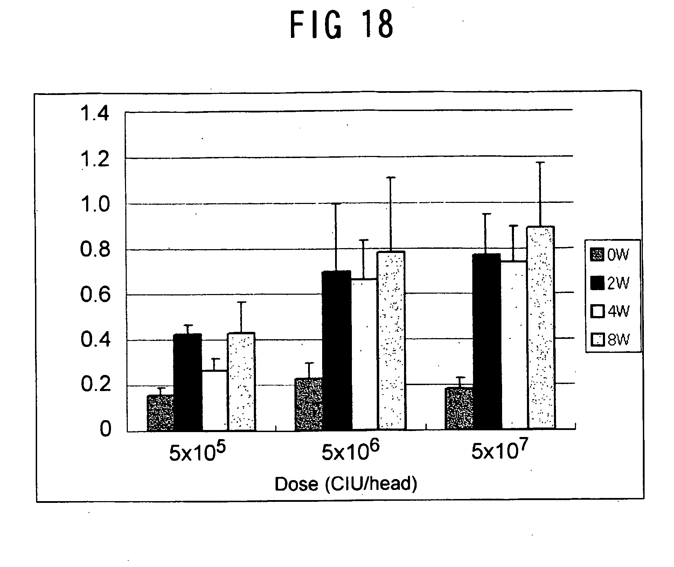

The result is shown in Fig. 18. The amount of anti-Aβ antibody was increased during the time course in all the three groups. While the amount of the antibody was increased by the vector administration in dose dependent manner, significant difference of the amount of the antibody was not observed between group 2 (5x 10⁶ CIU/head) and group 3 (5x 10⁷ CIU/head). The result shows that the induction of anti-Aβ antibody by the administration of the reagent of the invention (SeV-Aβ1-43/hIL-10) is not specific to Alzheimer's disease model mice (APP Tg mice), but is sufficiently achieved in normal mice.

### EXAMPLE 6. Effects of cytokine-encoding therapeutic vectors for Alzheimer's disease in intranasal administration to normal mice

### (6-1) Animals and method of administration

Gene encoding several cytokines were inserted into the therapeutic vectors for Alzheimer's disease described above. Effect of these *Sendai virus* vectors were tested using 6-week-old C57BL/6N mice. The mice were divided into three groups, each consisting of six mice. The *Sendai virus* vector (carrying a gene encoding Aβ peptide as well as a gene encoding mouse IL-4, IL-5 or IL-6; referred to as SeV-Aβ1-42/mIL-4, SeV-Aβ1-42/mIL-5 or SeV-Aβ1-42/mIL-6, respectively) was administered intranasally to each mouse (5 x 10⁶ CIU/10µl).

### (6-2) Determination of anti-Aβ antibody in plasma

Blood was collected from the mice before and 1 and 2 weeks after the administeration to determine the amount of anti-Aβ antibody present in plasma. Aβ1-42 peptide (4 µg/mL) was adsorbed onto each well of a 96-well plate (Nunc, MaxiSorp). After blocking with 1% BSA/2% goat serum/TBS-T buffer, the mouse plasma (300 times diluted) were added and detection was carried out using a peroxidase-labeled anti-mouse IgG antibody. Antibody titer was assessed with an absorbance measurement (O.D.450) using an ELISA reader.

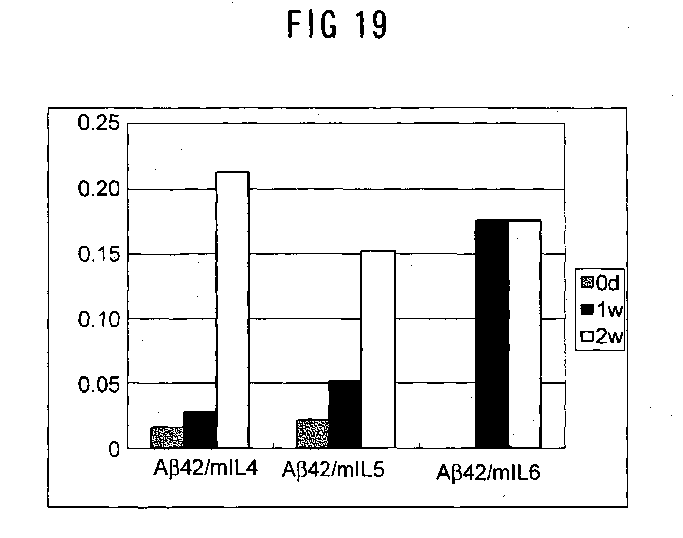

The result is shown in Fig. 19. The amount of anti-Aβ antibody was increased during the time course in the all three groups. The result shows that IL-4, IL-5, and IL-6 are effective for inducing anti-Aβ antibody, and the formulation comprising the *Sendai virus* vectors carrying both nucleic acids encoding an Aβ antigen and one or more cytokines selected from IL-4, IL-5, and IL-6 are preferable for inducing anti-Aβ antibody in treatment for Alzheimer's disease.

### INDUSTRIAL APPLICABILITY

The present invention provides novel minus strand RNA viral vectors that can effectively reduce the level of amyloid β deposition. The vectors of the present invention can be used to treat Alzheimer's disease.

## Claims

1. A minus strand RNA viral vector comprising an amyloid β-encoding nucleic acid.

2. The minus strand RNA viral vector of claim 1, further comprising a nucleic acid encoding a Th2 cytokine or a partial peptide thereof.

3. The minus strand RNA viral vector of claim 2, wherein the Th2 cytokine or a partial peptide thereof is IL-10 or a partial peptide thereof.

4. The vector of claim 1, wherein the minus strand RNA viral vector is a Paramyxoviral vector.

5. The vector of claim 4, wherein the Paramyxoviral vector is a *Sendai virus* vector.

6. The vector of claim 1, wherein the amyloid β is Aβ43 or a partial peptides thereof.

7. The vector of claim 1, wherein the amytoid β is derived from human.

8. The vector of claim 3, wherein IL-10 is derived from mouse or human.

9. The vector of claim 1, wherein the amyloid β is a polypeptide encoded by the nucleotide sequence of SEQ ID NO: 1, or a portion thereof.

10. The vector of claim 3, wherein IL-10 is a polypeptide encoded by the nucleotide sequence of SEQ ID NO: 2 or 3.

11. The minus strand RNA viral vector of claim 1, which comprises the nucleic acid of SEQ ID NO: 5.

12. A composition, which comprises the vector of any one of claims 1 to 11 and a pharmaceutically acceptable carrier.

13. The composition of claim 12, further comprising (i) a nucleic acid encoding a Th2 cytokine or a partial peptide thereof in an expressible manner, (ii) a Th2 cytokine or a partial peptide thereof, or (iii) a Th2 adjuvant.

14. The composition of claim 13, comprising a Th2 cytokine, a partial peptide thereof, or a Th2 adjuvant.

15. The composition of claim 13, comprising a nucleic acid encoding a Th2 cytokine or a partial peptide thereof in an expressible manner.

16. The composition of claim 15, wherein the Th2 cytokine is encoded by the minus strand RNA viral vector comprising an amyloid β-encoding nucleic acid.

17. The composition of claim 15, wherein the Th2 cytokine is encoded by a vector other than the minus strand RNA viral vector comprising an amyloid β-encoding nucleic acid.

18. The composition of claim 12, wherein the composition is a pharmaceutical composition.

19. A pharmaceutical composition for use in treating or preventing Alzheimer's disease, wherein the composition comprises the vector of any one of claims 1 to 11.

20. The composition of claim 19, further comprising (i) a nucleic acid encoding a Th2 cytokine or a partial peptide thereof in an expressible manner, (ii) a Th2 cytokine or a partial peptide thereof, or (iii) a Th2 adjuvant.

21. The pharmaceutical composition of claim 18 or 19, for use in intranasal administration.

22. Use of the vector of any one of claims 1 to 11 for the preparation of a pharmaceutical composition for reducing deposition of amyloid β

23. Use of the vector of any one of claims 1 to 11 for the preparation of a pharmaceutical composition for treating or preventing Alzheimer's disease.

24. The use of claim 22 or 23, wherein the pharmaceutical composition is to be administered intranasally.

25. The use of claim 22 or 23, wherein the pharmaceutical composition is to be administered in the form of an intramuscular injection.

26. The use of any one of claims 22 to 25, wherein, in addition to the pharmaceutical composition, a Th2 cytokine, a partial peptide thereof, a Th2 adjuvant, or a vector encoding a Th2 cytokine or a partial peptide thereof is to be administered.

## Patentansprüche

1. Minus-Strang-RNA-Virus-Vektor, umfassend eine ein β-Amyloid codierende Nucleinsäure.

2. Minus-Strang-RNA-Virus-Vektor nach Anspruch 1, des Weiteren umfassend eine Nucleinsäure, die ein Th2-Cytokin oder ein Teilpeptid davon codiert.

3. Minus-Strang-RNA-Virus-Vektor nach Anspruch 2, wobei das Th2-Cytokin oder ein Teilpeptid davon IL-10 oder ein Teilpeptid davon ist.

4. Vektor nach Anspruch 1, wobei der Minus-Strang-RNA-Virus-Vektor ein Paramyxovirus-Vektor ist.

5. Vektor nach Anspruch 4, wobei der Paramyxovirus-Vektor ein *Sendai-Virus-*Vektor ist.

6. Vektor nach Anspruch 1, wobei das β-Amyloid Aβ43 oder ein Teilpeptid davon ist.

7. Vektor nach Anspruch 1, wobei das β-Amyloid menschlichen Ursprungs ist.

8. Vektor nach Anspruch 3, wobei IL-10 menschlichen oder murinen Ursprungs ist.

9. Vektor nach Anspruch 1, wobei das β-Amyloid ein Polypeptid, das von der Nucleotidsequenz der SEQ ID NO:1 codiert wird, oder ein Teil davon ist.

10. Vektor nach Anspruch 3, wobei IL-10 ein Polypeptid ist, das von der Nucleotidsequenz der SEQ ID NO:2 oder 3 codiert wird.

11. Minus-Strang-RNA-Virus-Vektor nach Anspruch 1, der die Nucleinsäure der SEQ ID NO:5 umfasst.

12. Zusammensetzung, die den Vektor nach einem der Ansprüche 1 bis 11 und einen pharmazeutisch verträglichen Träger umfasst.

13. Zusammensetzung nach Anspruch 12, die des Weiteren (i) in exprimierbarer Weise eine Nucleinsäure, die Th2-Cytokin oder ein Teilpeptid codiert, (ii) ein Th2-Cytokin oder ein Teilpeptid davon oder (iii) ein Th2-Adjuvans umfasst.

14. Zusammensetzung nach Anspruch 13, die ein Th2-Cytokin, ein Teilpeptid davon oder ein Th2-Adjuvans umfasst.

15. Zusammensetzung nach Anspruch 13, die in exprimierbarer Weise eine Nucleinsäure umfasst, die ein Th2-Cytokin oder ein Teilpeptid davon codiert.

16. Zusammensetzung nach Anspruch 15, wobei das Th2-Cytokin vom Minus-Strang-RNA-Virus-Vektor codiert wird, der eine ein β-Amyloid codierende Nucleinsäure umfasst.

17. Zusammensetzung nach Anspruch 15, wobei das Th2-Cytokin von einem anderen Vektor als dem Minus-Strang-RNA-Virus-Vektor, der eine ein β-Amyloid codierende Nucleinsäure umfasst, codiert wird.

18. Zusammensetzung nach Anspruch 12, wobei die Zusammensetzung ein Arzneimittel ist.

19. Arzneimittel zur Verwendung bei der Behandlung oder der Prävention der Alzheimer-Erkrankung, wobei die Zusammensetzung den Vektor nach einem der Ansprüche 1 bis 11 umfasst.

20. Zusammensetzung nach Anspruch 19, die des Weiteren (i) in exprimierbarer Weise eine Nucleinsäure, die ein Th2-Cytokin oder ein Teilpeptid davon codiert, (ii) ein Th2-Cytokin oder ein Teilpeptid davon oder (iii) ein Th2-Adjuvans umfasst.

21. Arzneimittel nach Anspruch 18 oder 19 zur Verwendung für intranasale Verabreichung.

22. Verwendung des Vektors nach einem der Ansprüche 1 bis 11 zur Herstellung eines Arzneimittels für die Verringerung der β-Amyloid-Ablagerung.

23. Verwendung des Vektors nach einem der Ansprüche 1 bis 11 zur Herstellung eines Arzneimittels für die Behandlung oder Prävention der Alzheimer-Erkrankung.

24. Verwendung nach Anspruch 22 oder 23, wobei das Arzneimittel intranasal zu verabreichen ist.

25. Verwendung nach Anspruch 22 oder 23, wobei das Arzneimittel in Form einer intramuskulären Injektion zu verabreichen ist.

26. Verwendung nach einem der Ansprüche 22 bis 25, wobei zusätzlich zum Arzneimittel ein Th2-Cytokin, ein Teilpeptid davon, ein Th2-Adjuvans oder ein Vektor, der ein Th2-Cytokin oder ein Teilpeptid davon codiert, zu verabreichen ist.

## Revendications

1. Vecteur de virus à ARN négatif comprenant un acide nucléique codant l'amyloïde-β.

2. Vecteur de virus à ARN négatif selon la revendication 1, comprenant en outre un acide nucléique codant une cytokine Th2 ou un peptide partiel de celle-ci.

3. Vecteur de virus à ARN négatif selon la revendication 2, où la cytokine Th2 ou un peptide partiel de celle-ci est IL-10 ou un peptide partiel de celle-ci.

4. Vecteur selon la revendication 1, où le vecteur de virus à ARN négatif est un vecteur de Paramyxovirus.

5. Vecteur selon la revendication 4, où le vecteur de Paramyxovirus est un vecteur de *virus Sendai.*

6. Vecteur selon la revendication 1, où l'amyloïde-β est Aβ43 ou un peptide partiel de celle-ci.

7. Vecteur selon la revendication 1, où l'amyloïde-β est dérivée de l'humain.

8. Vecteur selon la revendication 3, où l'IL-10 est dérivée de la souris ou de l'humain.

9. Vecteur selon la revendication 1, où l'amyloïde-β est un polypeptide codé par la séquence nucléotidique de SEQ ID NO : 1, ou une portion de celui-ci.

10. Vecteur selon la revendication 3, où l'IL-10 est un polypeptide codé par la séquence nucléotidique de SEQ ID NO : 2 ou 3.

11. Vecteur de virus à ARN négatif selon la revendication 1, comprenant l'acide nucléique de SEQ ID NO : 5.

12. Composition comprenant le vecteur selon l'une quelconque des revendications 1 à 11 et un support pharmaceutiquement acceptable.

13. Composition selon la revendication 12, comprenant en outre (i) un acide nucléique codant une cytokine Th2 ou un peptide partiel de celle-ci de façon exprimable, (ii) une cytokine Th2 ou un peptide partiel de celle-ci, ou (iii) un adjuvant Th2.

14. Composition selon la revendication 13, comprenant une cytokine Th2, un peptide partiel de celle-ci, ou un adjuvant Th2.

15. Composition selon la revendication 13, comprenant un acide nucléique codant une cytokine Th2 ou un peptide partiel de celle-ci de façon exprimable.

16. Composition selon la revendication 15, où la cytokine Th2 est codée par le vecteur de virus à ARN négatif comprenant un acide nucléique codant l'amyloïde-β.

17. Composition selon la revendication 15, où la cytokine Th2 est codée par un vecteur autre que le vecteur de virus à ARN négatif comprenant un acide nucléique codant l'amyloïde-β.

18. Composition selon la revendication 12, où la composition est une composition pharmaceutique.

19. Composition pharmaceutique pour une utilisation dans le traitement ou la prévention de la maladie d'Alzheimer, où la composition comprend le vecteur selon l'une quelconque des revendications 1 à 11.

20. Composition selon la revendication 19, comprenant en outre (i) un acide nucléique codant une cytokine Th2 ou un peptide partiel de celle-ci de façon exprimable, (ii) une cytokine Th2 ou un peptide partiel de celle-ci, ou (iii) un adjuvant Th2.

21. Composition pharmaceutique selon la revendication 18 ou 19, pour une utilisation en administration intranasale.

22. Utilisation du vecteur selon l'une quelconque des revendications 1 à 11 pour la préparation d'une composition pharmaceutique pour réduire le dépôt d'amyloïde-β.

23. Utilisation du vecteur selon l'une quelconque des revendications 1 à 11 pour la préparation d'une composition pharmaceutique pour traiter ou prévenir la maladie d'Alzheimer.

24. Utilisation selon la revendication 22 ou 23, où la composition pharmaceutique est à administrer par voie intranasale.

25. Utilisation selon la revendication 22 ou 23, où la composition pharmaceutique est à administrer sous la forme d'une injection intramusculaire.

26. Utilisation selon l'une quelconque des revendications 22 à 25, où, en plus de la composition pharmaceutique, une cytokine Th2, un peptide partiel de celle-ci, un adjuvant Th2, ou un vecteur codant une cytokine Th2 ou un peptide partiel de celle-ci est à administrer.


### SEQUENCE LISTING

<110> DNAVEC CORPORATION
   JAPAN AS REPRESENTED BY PRESIDENT OF NATIONAL CENTER FOR GERIATRICS AND GERONTOLOGY
<120> HIGHLY SAFE INTRANASALLY ADMINISTRABLE GENE VACCINES FOR TREATING ALZHEIMER'S DISEASE
<130> D4-A0503P
<150> JP 2005-123015
   <151> 2005-04-20
<150> US 60/727,690
   <151> 2005-10-18
<160> 30
<170> PatentIn version 3.3
<210> 1
   <211> 186
   <212> DNA
   <213> Homo sapiens
<400> 1
   atgctgcccg gtttggcact gctcctgctg gccgcctgga cggctcgggc gcttgatgca      60
   gaattccgac atgactcagg atatgaagtt catcatcaaa aattggtgtt ctttgcagaa     120
   gatgtgggtt caaacaaagg tgcaatcatt ggactcatgg tgggcggtgt tgtcatagcg     180
   acttaa                                                                186
<210> 2
   <211> 537
   <212> DNA
   <213> Mus musculus
<400> 2
   atgcctggct cagcactgct atgctgcctg ctcttactga ctggcatgag gatcagcagg      60
   ggccagtaca gccgggaaga caataactgc acccacttcc cagtcggcca gagccacatg     120
   ctcctagagc tgcggactgc cttcagccag gtgaagactt tctttcaaac aaaggaccag     180
   ctggacaaca tactgctaac cgactcctta atgcaggact ttaagggtta cttgggttgc     240
   caagccttat cggaaatgat ccagttttac ctggtagaag tgatgcccca ggcagagaag     300
   catggcccag aaatcaagga gcatttgaat tccctgggtg agaagctgaa gaccctcagg     360
   atgcggctga ggcgctgtca tcgatttctc ccctgtgaaa ataagagcaa ggcagtggag     420
   caggtgaaga gtgattttaa taagctccaa gaccaaggtg tctacaaggc catgaatgaa     480
   tttgacatct tcatcaactg catagaagca tacatgatga tcaaaatgaa aagctaa        537
<210> 3
   <211> 537
   <212> DNA
   <213> Homo sapiens
<400> 3
   atgcacagct cagcactgct ctgttgcctg gtcctcctga ctggggtgag ggccagccca     60
   ggccagggca cccagtctga gaacagctgc acccacttcc caggcaacct gcctaacatg    120
   cttcgagatc tccgagatgc cttcagcaga gtgaagactt tctttcaaat gaaggatcag    180
   ctggacaact tgttgttaaa ggagtccttg ctggaggact ttaagggtta cctgggttgc    240
   caagccttgt ctgagatgat ccagttttac ctggaggagg tgatgcccca agctgagaac    300
   caagacccag acatcaaggc gcatgtgaac tccctggggg agaacctgaa gaccctcagg    360
   ctgaggctac ggcgctgtca tcgatttctt ccctgtgaaa acaagagcaa ggccgtggag    420
   caggtgaaga atgcctttaa taagctccaa gagaaaggca tctacaaagc catgagtgag    480
   tttgacatct tcatcaacta catagaagcc tacatgacaa tgaagatacg aaactga       537
<210> 4
   <211> 14412
   <212> DNA
   <213> artificial
<220>
   <223> a DNA endoding a Sendai virus antigenomic RNA
<400> 4
   accaaacaag agaaaaaaca tgtatgggat atgtaatgaa gttatacagg attttagggt 60
   caaagtatcc accctgagga gcaggttcca gaccctttgc tttgctgcca aagttcacgc 120
   ggccgccaag gttcacttat gctgcccggt ttggcactgc tcctgctggc cgcctggacg 180
   gctcgggcgc ttgatgcaga attccgacat gactcaggat atgaagttca tcatcaaaaa 240
   ttggtgttct ttgcagaaga tgtgggttca aacaaaggtg caatcattgg actcatggtg 300
   ggcggtgttg tcatagcgac ttaaccgtag taagaaaaac ttagggtgaa agttcatcgc 360
   ggccgcagat cttcacgatg gccgggttgt tgagcacctt cgatacattt agctctagga 420
   ggagcgaaag tattaataag tcgggaggag gtgctgttat ccccggccag aggagcacag 480
   tctcagtgtt cgtactaggc ccaagtgtga ctgatgatgc agacaagtta ttcattgcaa 540
   ctaccttcct agctcactca ttggacacag ataagcagca ctctcagaga ggggggttcc 600
   tcgtctctct gcttgccatg gcttacagta gtccagaatt gtacttgaca acaaacggag 660
   taaacgccga tgtcaaatat gtgatctaca acatagagaa agaccctaag aggacgaaga 720
   cagacggatt cattgtgaag acgagagata tggaatatga gaggaccaca gaatggctgt 780
   ttggacctat ggtcaacaag agcccactct tccagggtca acgggatgct gcagaccctg 840
   acacactcct tcaaatctat gggtatcctg catgcctagg agcaataatt gtccaagtct 900
   ggattgtgct ggtgaaggcc atcacaagca gcgccggctt aaggaaaggg ttcttcaaca 960
   ggttagaggc gttcagacaa gacggcaccg tgaaaggtgc cttagttttc actggggaga 1020
   cagttgaggg gataggctcg gttatgagat ctcagcaaag ccttgtatct ctcatggttg 1080
   agacccttgt gactatgaat actgcaagat ctgatctcac cacattagag aagaacatcc 1140
   agatcgttgg gaactacatc cgagatgcag ggctggcttc cttcatgaac actattaaat 1200
   atggggtgga aacaaagatg gcagctctaa cgttgtcaaa cctgaggccc gatattaata 1260
   agcttagaag cctcatagac acctacctgt caaaaggccc cagagctccc tttatctgta 1320
   tcctcaagga ccctgttcat ggtgaatttg ctccaggcaa ttatcctgca ctatggagtt 1380
   acgccatggg agtcgccgtc gtacagaaca aggcaatgca gcagtacgtc acagggagga   1440
   cataccttga tatggaaatg ttcttactag gacaagccgt ggcaaaggat gctgaatcga   1500
   agatcagcag tgccttggaa gatgagttag gagtgacgga tacagccaag gggaggctca 1560
   gacatcatct ggcaaacttg tccggtgggg atggtgctta ccacaaacca acaggcggtg 1620
   gtgcaattga ggtagctcta gacaatgccg acatcgacct agaaacaaaa gcccatgcgg 1680
   accaggacgc taggggttgg ggtggagata gtggtgaaag atgggcacgt caggtgagtg 1740
   gtggccactt tgtcacacta catggggctg aacggttaga ggaggaaacc aatgatgagg 1800
   atgtatcaga catagagaga agaatagcca tgagactcgc agagagacgg caagaggatt 1860
   ctgcaaccca tggagatgaa ggccgcaata acggtgtcga tcatgacgaa gatgacgatg 1920
   ccgcagcagt agctgggata ggaggaatct aggatcatac gaggcttcaa ggtacttgat 1980
   ccgtagtaag aaaaacttag ggtgaaagtt catccaccga tcggctcagg caaggccaca 2040
   cccaacccca ccgaccacac ccagcagtcg agacagccac ggcttcggct acacttaccg 2100
   catggatcaa gatgccttca ttcttaaaga agattctgaa gttgagaggg aggcgccagg 2160
   aggacgagag tcgctctcgg atgttatcgg attcctcgat gctgtcctgt cgagtgaacc 2220
   aactgacatc ggaggggaca gaagctggct ccacaacacc atcaacactc cccaaggacc 2280
   aggctctgct catagagcca aaagtgaggg cgaaggagaa gtctcaacac cgtcgaccca 2340
   agataatcga tcaggtgagg agagtagagt ctctgggaga acaagcaagc cagaggcaga 2400
   agcacatgct ggaaaccttg ataaacaaaa tatacaccgg gcctttgggg gaagaactgg 2460
   tacaaactct gtatctcagg atctgggcga tggaggagac tccggaatcc ttgaaaatcc 2520
   tccaaatgag agaggatatc cgagatcagg tattgaagat gaaaacagag agatggctgc 2580
   gcaccctgat aagaggggag aagaccaagc tgaaggactt ccagaagagg tacgaggaag 2640
   tacatcccta cctgatgaag gagaaggtgg agcaagtaat aatggaagaa gcatggagcc 2700
   tggcagctca catagtgcaa gagtaactgg ggtcctggtg attcctagcc ccgaacttga 2760
   agaggctgtg ctacggagga acaaaagaag acctaccaac agtgggtcca aacctcttac 2820
   tccagcaacc gtgcctggca cccggtcccc accgctgaat cgttacaaca gcacagggtc 2880
   accaccagga aaacccccat ctacacagga tgagcacatc aactctgggg acacccccgc 2940
   cgtcagggtc aaagaccgga aaccaccaat agggacccgc tctgtctcag attgtccagc 3000
   caacggccgc ccaatccacc cgggtctaga gaccgactca acaaaaaagg gcataggaga 3060
   gaacacatca tctatgaaag agatggctac attgttgacg agtcttggtg taatccagtc 3120
   tgctcaagaa ttcgaatcat cccgagacgc gagttatgtg tttgcaagac gtgccctaaa 3180
   gtctgcaaac tatgcagaga tgacattcaa tgtatgcggc ctgatccttt ctgccgagaa 3240
   atcttccgct cgtaaggtag atgagaacaa acaactgctc aaacagatcc aagagagcgt 3300
   ggaatcattc cgggatattt acaagagatt ctctgagtat cagaaagaac agaactcatt 3360
   gctgatgtcc aacctatcta cacttcatat catcacagat agaggtggca agactgacaa 3420
   cacagactcc cttacaaggt ccccctccgt ttttgcaaaa tcaaaagaga acaagactaa 3480
   ggctaccagg tttgacccat ctatggagac cctagaagat atgaagtaca aaccggacct 3540
   aatccgagag gatgaattta gagatgagat ccgcaacccg gtgtaccaag agagggacac 3600
   agaacccagg gcctcaaacg catcacgtct ctttccctcc aaagagaagc ccacaatgca 3660
   ctctctcagg ctcgtcatag agagcagtcc cctaagcaga gctgagaaag tagcatatgt 3720
   gaaatcatta tccaagtgca agacagacca agaggttaag gcagtcatgg aactcgtaga 3780
   agaggacata gagtcactga ccaactagat cccgggtgag gcatcctacc atcctcagtc 3840
   atagagagat ccaatctacc atcagcatca gccagtaaag attaagaaaa acttagggtg 3900
   aaagaaattt cacctaacac ggcgcaatgg cagatatcta tagattccct aagttctcat 3960
   atgaggataa cggtactgtg gagcccctgc ctctgagaac tggtccggat aagaaagcca 4020
   tcccccacat caggattgtc aaggtaggag accctcctaa acatggagtg agatacctag 4080
   atttattgct cttgggtttc tttgagacac cgaaacaaac aaccaatcta gagagcgtat 4140
   ctgacttgac agagccgacc agctactcaa tatgcggctc cgggtcgtta cccataggtg 4200
   tggccaaata ctacgggact gatcaggaac tcttaaaggc ctgcaccgat ctcagaatta 4260
   cggtgaggag ggctgttcga gcaggagaga tgatcgtata catggtggat tcgattggtg 4320
   ctccactcct accatggtca ggcaggctga gacagggaat gatatttaat gcaaacaagg 4380
   tcgcactagc tccccaatgc ctccctgtgg acaaggacat aagactcaga gtggtgtttg 4440
   tcaatgggac atctctaggg gcaatcacca tatccaagat cccaaagacc cttgcagacc 4500
   ttgcattgcc caactctata tctgttaatt tactggtgac actcaagacc gggatctcca 4560
   cagaacaaaa gggggtactc ccagtacttg atgatcaagg ggagaaaaag ctcaatttta 4620
   tggtgcacct cgggttgatc aggagaaagg tcgggaagat atactctgtt gagtactgca 4680
   agagcaagat tgagagaatg cggctgattt tctcacttgg gttaatcggc ggtataagct 4740
   tccatgttca ggttaatggg acactatcta agacattcat gagtcagctc gcatggaaga 4800
   gggcagtctg cttcccatta atggatgtga atccccatat gaacatggtg atttgggcgg 4860
   catctgtaga aatcacaggc gtcgatgcgg tgttccaacc ggccatccct cgtgatttcc 4920
   gctactaccc taatgttgtg gctaagaaca tcggaaggat cagaaagctg taaatgtgca 4980
   cccatcagag acctgcgaca atgccccaag cagacaccac ctggcagtcg gagccaccgg 5040
   gtcactcctt gtcttaaata agaaaaactt agggataaag tcccttgtga gtgcttggtt 5100
   aattaagcgg ccgccaaagt tcaatgcctg gctcagcact gctatgctgc ctgctcttac 5160
   tgactggcat gaggatcagc aggggccagt acagccggga agacaataac tgcacccact 5220
   tcccagtcgg ccagagccac atgctcctag agctgcggac tgccttcagc caggtgaaga 5280
   ctttctttca aacaaaggac cagctggaca acatactgct aaccgactcc ttaatgcagg 5340
   actttaaggg ttacttgggt tgccaagcct tatcggaaat gatccagttt tacctggtag 5400
   aagtgatgcc ccaggcagag aagcatggcc cagaaatcaa ggagcatttg aattccctgg 5460
   gtgagaagct gaagaccctc aggatgcggc tgaggcgctg tcatcgattt ctcccctgtg 5520
   aaaataagag caaggcagtg gagcaggtga agagtgattt taataagctc caagaccaag 5580
   gtgtctacaa ggccatgaat gaatttgaca tcttcatcaa ctgcatagaa gcatacatga 5640
   tgatcaaaat gaaaagctaa ccgtagtaag aaaaacttag ggtgaaagtt catcgcggcc 5700
   gcctcaaaca agcacagatc atggatggtg ataggggcaa acgtgactcg tactggtcta 5760
   cttctcctag tggtagcacc acaaaaccag catcaggttg ggagaggtca agtaaagccg 5820
   acacatggtt gctgattctc tcattcaccc agtgggcttt gtcaattgcc acagtgatca 5880
   tctgtatcat aatttctgct agacaagggt atagtatgaa agagtactca atgactgtag 5940
   aggcattgaa catgagcagc agggaggtga aagagtcact taccagtcta ataaggcaag 6000
   aggttatagc aagggctgtc aacattcaga gctctgtgca aaccggaatc ccagtcttgt 6060
   tgaacaaaaa cagcagggat gtcatccaga tgattgataa gtcgtgcagc agacaagagc 6120
   tcactcagca ctgtgagagt acgatcgcag tccaccatgc cgatggaatt gccccacttg 6180
   agccacatag tttctggaga tgccctgtcg gagaaccgta tcttagctca gatcctgaaa 6240
   tctcattgct gcctggtccg agcttgttat ctggttctac aacgatctct ggatgtgtta 6300
   ggctcccttc actctcaatt ggcgaggcaa tctatgccta ttcatcaaat ctcattacac 6360
   aaggttgtgc tgacataggg aaatcatatc aggtcctgca gctagggtac atatcactca 6420
   attcagatat gttccctgat cttaaccccg tagtgtccca cacttatgac atcaacgaca 6480
   atcggaaatc atgctctgtg gtgacaaccc ggactagggg ttatcagctt tgctccatgc 6540
   cgactgtaga cgaaagaacc gactactcta gtgatggtat tgaggatctg gtccttgatg 6600
   tcctggatct caaagggaga actaagtctc accggtatcg caacagcgag gtagatcttg 6660
   atcacccgtt ctctgcacta taccccagtg taggcaacgg cattgcaaca gaaggctcat 6720
   tgatatttct tgggtatggt ggactaacca cccctctgca gggtgataca aaatgtagga 6780
   cccaaggatg ccaacaggtg tcgcaagaca catgcaatga ggctctgaaa attacatggc 6840
   taggagggaa acaggtggtc agcgtgatca tccaggtcaa tgactatctc tcagagaggc 6900
   caaagataag agtcacaacc attccaatca ctcaaaacta tctcggggcg gaaggtagat 6960
   tattaaaatt gggtgatcgg gtgtacatct atacaagatc atcaggctgg cactctcaac 7020
   tgcagatagg agtacttgat gtcagccacc ctttgactat caactggaca cctcatgaag 7080
   ccttgtctag accaggaaat gaagagtgca attggtacaa taagtgtccg aaggaatgca 7140
   tatcaggcgt atacactgat gcttatccat tgtcccctga tgcagctaac gtcgctaccg 7200
   tcacgctata tgccaataca tcgcgtgtca acccaacaat catgtattct aacactacta 7260
   acattataaa tatgttaagg ataaaggatg ttcaattaga ggctgcatat accacgacat 7320
   cgtgtatcac gcattttggt aaaggctact gctttcacat catcgagatc aatcagaaga 7380
   gcctgaatac cttacagccg atgctcttta agactagcat ccctaaatta tgcaaggccg 7440
   agtcttaaat ttaactgact agcaggcttg tcggccttgc tgacactaga gtcatctccg 7500
   aacatccaca atatctctca gtctcttacg tctctcacag tattaagaaa aacccagggt 7560
   gaatgggaag cttgccatag gtcatggatg ggcaggagtc ctcccaaaac ccttctgaca 7620
   tactctatcc agaatgccac ctgaactctc ccatagtcag ggggaagata gcacagttgc 7680
   acgtcttgtt agatgtgaac cagccctaca gactgaagga cgacagcata ataaatatta 7740
   caaagcacaa aattaggaac ggaggattgt ccccccgtca aattaagatc aggtctctgg 7800
   gtaaggctct tcaacgcaca ataaaggatt tagaccgata cacgtttgaa ccgtacccaa 7860
   cctactctca ggaattactt aggcttgata taccagagat atgtgacaaa atccgatccg 7920
   tcttcgcggt ctcggatcgg ctgaccaggg agttatctag tgggttccag gatctttggt 7980
   tgaatatctt caagcaacta ggcaatatag aaggaagaga ggggtacgat ccgttgcagg 8040
   atatcggcac catcccggag ataactgata agtacagcag gaatagatgg tataggccat 8100
   tcctaacttg gttcagcatc aaatatgaca tgcggtggat gcagaagacc agaccggggg 8160
   gacccctcga tacctctaat tcacataacc tcctagaatg caaatcatac actctagtaa 8220
   catacggaga tcttgtcatg atactgaaca agttgacatt gacagggtat atcctaaccc 8280
   ctgagctggt cttgatgtat tgtgatgttg tagaaggaag gtggaatatg tctgctgcag 8340
   ggcatctaga taagaagtcc attgggataa caagcaaagg tgaggaatta tgggaactag 8400
   tggattccct cttctcaagt cttggagagg aaatatacaa tgtcatcgca ctattggagc 8460
   ccctatcact tgctctcata caactaaatg atcctgttat acctctacgt ggggcattta 8520
   tgaggcatgt gttgacagag ctacagactg ttttaacaag tagagacgtg tacacagatg 8580
   ctgaagcaga cactattgtg gagtcgttac tcgccatttt ccatggaacc tctattgatg 8640
   agaaagcaga gatcttttcc ttctttagga catttggcca ccccagctta gaggctgtca 8700
   ctgccgccga caaggtaagg gcccatatgt atgcacaaaa ggcaataaag cttaagaccc 8760
   tatacgagtg tcatgcagtt ttttgcacta tcatcataaa tgggtataga gagaggcatg 8820
   gcggacagtg gcccccctgt gacttccctg atcacgtgtg tctagaacta aggaacgctc 8880
   aagggtccaa tacggcaatc tcttatgaat gtgctgtaga caactataca agtttcatag 8940
   gcttcaagtt tcggaagttt atagaaccac aactagatga agatctcaca atatatatga 9000
   aagacaaagc actatccccc aggaaggagg catgggactc tgtatacccg gatagtaatc 9060
   tgtactataa agccccagag tctgaagaga cccggcggct tattgaagtg ttcataaatg 9120
   atgagaattt caacccagaa gaaattatca attatgtgga gtcaggagat tggttgaaag 9180
   acgaggagtt caacatctcg tacagtctca aagagaaaga gatcaagcaa gagggtcgtc 9240
   tattcgcaaa aatgacttat aagatgcgag ccgtacaggt gctggcagag acactactgg 9300
   ctaaaggaat aggagagcta ttcagcgaaa atgggatggt taaaggagag atagacctac 9360
   ttaaaagatt gactactctt tctgtctcag gcgtccccag gactgattca gtgtacaata 9420
   actctaaatc atcagagaag agaaacgaag gcatggaaaa taagaactct ggggggtact 9480
   gggacgaaaa gaagaggtcc agacatgaat tcaaggcaac agattcatca acagacggct 9540
   atgaaacgtt aagttgcttc ctcacaacag acctcaagaa atactgctta aactggagat 9600
   ttgagagtac tgcattgttt ggtcagagat gcaacgagat atttggcttc aagaccttct 9660
   ttaactggat gcatccagtc cttgaaaggt gtacaatata tgttggagat ccttactgtc 9720
   cagtcgccga ccggatgcat cgacaactcc aggatcatgc agactctggc attttcatac 9780
   ataatcctag ggggggcata gaaggttact gccagaagct gtggacctta atctcaatca 9840
   gtgcaatcca cctagcagct gtgagagtgg gtgtcagggt ctctgcaatg gttcagggtg 9900
   acaatcaagc tatagccgtg acatcaagag tacctgtagc tcagacttac aagcagaaga 9960
   aaaatcatgt ctatgaggag atcaccaaat atttcggtgc tctaagacac gtcatgtttg 10020
   atgtagggca cgagctaaaa ttgaacgaga ccatcattag tagcaagatg tttgtctata 10080
   gtaaaaggat atactatgat gggaagattt taccacagtg cctgaaagcc ttgaccaagt 10140
   gtgtattctg gtccgagaca ctggtagatg aaaacagatc tgcttgttcg aacatctcaa 10200
   catccatagc aaaagctatc gaaaatgggt attctcctat actaggctac tgcattgcgt 10260
   tgtataagac ctgtcagcag gtgtgcatat cactagggat gactataaat ccaactatca 10320
   gcccgaccgt aagagatcaa tactttaagg gtaagaattg gctgagatgt gcagtgttga 10380
   ttccagcaaa tgttggagga ttcaactaca tgtctacatc tagatgcttt gttagaaata 10440
   ttggagaccc cgcagtagca gccctagctg atctcaaaag attcatcaga gcggatctgt 10500
   tagacaagca ggtattatac agggtcatga atcaagaacc cggtgactct agttttctag 10560
   attgggcttc agacccttat tcgtgtaacc tcccgcattc tcagagtata actacgatta 10620
   taaagaatat cactgctaga tctgtgctgc aggaatcccc gaatcctcta ctgtctggtc 10680
   tcttcaccga gactagtgga gaagaggatc tcaacctggc ctcgttcctt atggaccgga 10740
   aagtcatcct gccgagagtg gctcatgaga tcctgggtaa ttccttaact ggagttaggg 10800
   aggcgattgc agggatgctt gatacgacca agtctctagt gagagccagc gttaggaaag 10860
   gaggattatc atatgggata ttgaggaggc ttgtcaatta tgatctattg cagtacgaga 10920
   cactgactag aactctcagg aaaccggtga aagacaacat cgaatatgag tatatgtgtt 10980
   cagttgagct agctgtcggt ctaaggcaga aaatgtggat ccacctgact tacgggagac 11040
   ccatacatgg gctagaaaca ccagaccctt tagagctctt gaggggaata tttatcgaag 11100
   gttcagaggt gtgcaagctt tgcaggtctg aaggagcaga ccccatctat acatggttct 11160
   atcttcctga ctctatagac ctggacacgc ttacaaacgg atgtccggct ataagaatcc 11220
   cctattttgg atcagccact gatgaaaggt cggaagccca actcgggtat gtaagaaatc 11280
   taagcaaacc cgcaaaggcg gccatccgga tagctatggt gtatacgtgg gcctacggga 11340
   ctgatgagat atcgtggatg gaagccgctc ttatagccca aacaagagct aatctgagct 11400
   tagagaatct aaagctgctg actcctgttt caacctccac taatctatct cataggttga 11460
   aagatacggc aacccagatg aagttctcta gtgcaacact agtccgtgca agtcggttca 11520
   taacaatatc aaatgataac atggcactca aagaagcagg ggagtcgaag gatactaatc 11580
   tcgtgtatca gcagattatg ctaactgggc taagcttgtt cgagttcaat atgagatata 11640
   agaaaggttc cttagggaag ccactgatat tgcacttaca tcttaataac gggtgctgta 11700
   taatggagtc cccacaggag gcgaatatcc ccccaaggtc cacattagat ttagagatta 11760
   cacaagagaa caataaattg atctatgatc ctgatccact caaggatgtg gaccttgagc 11820
   tatttagcaa ggtcagagat gttgtacaca cagttgacat gacttattgg tcagatgatg 11880
   aagttatcag agcaaccagt atctgtactg caatgacgat agctgataca atgtctcaat 11940
   tagatagaga caacttaaaa gagatgatcg cactagtaaa tgacgatgat gtcaacagct 12000
   tgattactga gtttatggtg attgatgttc ctttattttg ctcaacgttc gggggtattc 12060
   tagtcaatca gtttgcatac tcactctacg gcttaaacat cagaggaagg gaagaaatat 12120
   ggggacatgt agtccggatt cttaaagata cctcccacgc agttttaaaa gtcttatcta 12180
   atgctctatc tcatcccaaa atcttcaaac gattctggaa tgcaggtgtc gtggaacctg 12240
   tgtatgggcc taacctctca aatcaggata agatactctt ggccctctct gtctgtgaat 12300
   attctgtgga tctattcatg cacgattggc aagggggtgt accgcttgag atctttatct 12360
   gtgacaatga cccagatgtg gccgacatga ggaggtcctc tttcttggca agacatcttg 12420
   catacctatg cagcttggca gagatatcta gggatgggcc aagattagaa tcaatgaact 12480
   ctctagagag gctcgagtca ctaaagagtt acctggaact cacatttctt gatgacccgg 12540
   tactgaggta cagtcagttg actggcctag tcatcaaagt attcccatct actttgacct 12600
   atatccggaa gtcatctata aaagtgttaa ggacaagagg tataggagtc cctgaagtct 12660
   tagaagattg ggatcccgag gcagataatg cactgttaga tggtatcgcg gcagaaatac 12720
   aacagaatat tcctttggga catcagacta gagccccttt ttgggggttg agagtatcca 12780
   agtcacaggt actgcgtctc cgggggtaca aggagatcac aagaggtgag ataggcagat 12840
   caggtgttgg tctgacgtta ccattcgatg gaagatatct atctcaccag ctgaggctct 12900
   ttggcatcaa cagtactagc tgcttgaaag cacttgaact tacctaccta ttgagcccct 12960
   tagttgacga agataaagat aggctatatt taggggaagg agctggggcc atgctttcct 13020
   gttatgacgc tactcttggc ccatgcatca actattataa ctcaggggta tactcttgtg 13080
   atgtcaatgg gcagagagag ttaaatatat atcctgctga ggtggcacta gtgggaaaga 13140
   aattaaacaa tgttactagt ctgggtcaaa gagttaaagt gttattcaac gggaatcctg 13200
   gctcgacatg gattgggaat gatgagtgtg aggctttgat ttggaatgaa ttacagaata 13260
   gctcgatagg cctagtccac tgtgacatgg agggaggaga tcataaggat gatcaagttg 13320
   tactgcatga gcattacagt gtaatccgga tcgcgtatct ggtgggggat cgagacgttg 13380
   tgcttataag caagattgct cccaggctgg gcacggattg gaccaggcag ctcagcctat 13440
   atctgagata ctgggacgag gttaacctaa tagtgcttaa aacatctaac cctgcttcca 13500
   cagagatgta tctcctatcg aggcacccca aatctgacat tatagaggac agcaagacag 13560
   tgttagctag tctcctccct ttgtcaaaag aagatagcat caagatagaa aagtggatct 13620
   taatagagaa ggcaaaggct cacgaatggg ttactcggga attgagagaa ggaagctctt 13680
   catcagggat gcttagacct taccatcaag cactgcagac gtttggcttt gaaccaaact 13740
   tgtataaatt gagcagagat ttcttgtcca ccatgaacat agctgataca cacaactgca 13800
   tgatagcttt caacagggtt ttgaaggata caatcttcga atgggctaga ataactgagt 13860
   cagataaaag gcttaaacta actggtaagt atgacctgta tcctgtgaga gattcaggca 13920
   agttgaagac aatttctaga agacttgtgc tatcttggat atctttatct atgtccacaa 13980
   gattggtaac tgggtcattc cctgaccaga agtttgaagc aagacttcaa ttgggaatag 14040
   tttcattatc atcccgtgaa atcaggaacc tgagggttat cacaaaaact ttattagaca 14100
   ggtttgagga tattatacat agtataacgt atagattcct caccaaagaa ataaagattt 14160
   tgatgaagat tttaggggca gtcaagatgt tcggggccag gcaaaatgaa tacacgaccg 14220
   tgattgatga tggatcacta ggtgatatcg agccatatga cagctcgtaa taattagtcc 14280
   ctatcgtgca gaacgatcga agctccgcgg tacctggaag tcttggactt gtccatatga 14340
   caatagtaag aaaaacttac aagaagacaa gaaaatttaa aaggatacat atctcttaaa 14400
   ctcttgtctg gt 14412
<210> 5
   <211> 14412
   <212> RNA
   <213> artificial
<220>
   <223> a Sendai virus genomic RNA
<400> 5
   accagacaag aguuuaagag auauguaucc uuuuaaauuu ucuugucuuc uuguaaguuu 60
   uucuuacuau ugucauaugg acaaguccaa gacuuccagg uaccgcggag cuucgaucgu 120
   ucugcacgau agggacuaau uauuacgagc ugucauaugg cucgauauca ccuagugauc 180
   caucaucaau cacggucgug uauucauuuu gccuggcccc gaacaucuug acugccccua 240
   aaaucuucau caaaaucuuu auuucuuugg ugaggaaucu auacguuaua cuauguauaa 300
   uauccucaaa ccugucuaau aaaguuuuug ugauaacccu cagguuccug auuucacggg 360
   augauaauga aacuauuccc aauugaaguc uugcuucaaa cuucugguca gggaaugacc 420
   caguuaccaa ucuuguggac auagauaaag auauccaaga uagcacaagu cuucuagaaa 480
   uugucuucaa cuugccugaa ucucucacag gauacagguc auacuuacca guuaguuuaa 540
   gccuuuuauc ugacucaguu auucuagccc auucgaagau uguauccuuc aaaacccugu 600
   ugaaagcuau caugcaguug uguguaucag cuauguucau gguggacaag aaaucucugc 660
   ucaauuuaua caaguuuggu ucaaagccaa acgucugcag ugcuugaugg uaaggucuaa 720
   gcaucccuga ugaagagcuu ccuucucuca auucccgagu aacccauucg ugagccuuug 780
   ccuucucuau uaagauccac uuuucuaucu ugaugcuauc uucuuuugac aaagggagga 840
   gacuagcuaa cacugucuug cuguccucua uaaugucaga uuuggggugc cucgauagga 900
   gauacaucuc uguggaagca ggguuagaug uuuuaagcac uauuagguua accucguccc 960
   aguaucucag auauaggcug agcugccugg uccaauccgu gcccagccug ggagcaaucu 1020
   ugcuuauaag cacaacgucu cgauccccca ccagauacgc gauccggauu acacuguaau 1080
   gcucaugcag uacaacuuga ucauccuuau gaucuccucc cuccauguca caguggacua 1140
   ggccuaucga gcuauucugu aauucauucc aaaucaaagc cucacacuca ucauucccaa 1200
   uccaugucga gccaggauuc ccguugaaua acacuuuaac ucuuugaccc agacuaguaa 1260
   cauuguuuaa uuucuuuccc acuagugcca ccucagcagg auauauauuu aacucucucu 1320
   gcccauugac aucacaagag uauaccccug aguuauaaua guugaugcau gggccaagag 1380
   uagcgucaua acaggaaagc auggccccag cuccuucccc uaaauauagc cuaucuuuau 1440
   cuucgucaac uaaggggcuc aauagguagg uaaguucaag ugcuuucaag cagcuaguac 1500
   uguugaugcc aaagagccuc agcuggugag auagauaucu uccaucgaau gguaacguca 1560
   gaccaacacc ugaucugccu aucucaccuc uugugaucuc cuuguacccc cggagacgca 1620
   guaccuguga cuuggauacu cucaaccccc aaaaaggggc ucuagucuga ugucccaaag 1680
   gaauauucug uuguauuucu gccgcgauac caucuaacag ugcauuaucu gccucgggau 1740
   cccaaucuuc uaagacuuca gggacuccua uaccucuugu ccuuaacacu uuuauagaug 1800
   acuuccggau auaggucaaa guagauggga auacuuugau gacuaggcca gucaacugac 1860
   uguaccucag uaccggguca ucaagaaaug ugaguuccag guaacucuuu agugacucga 1920
   gccucucuag agaguucauu gauucuaauc uuggcccauc ccuagauauc ucugccaagc 1980
   ugcauaggua ugcaagaugu cuugccaaga aagaggaccu ccucaugucg gccacaucug 2040
   ggucauuguc acagauaaag aucucaagcg guacaccccc uugccaaucg ugcaugaaua 2100
   gauccacaga auauucacag acagagaggg ccaagaguau cuuauccuga uuugagaggu 2160
   uaggcccaua cacagguucc acgacaccug cauuccagaa ucguuugaag auuuugggau 2220
   gagauagagc auuagauaag acuuuuaaaa cugcguggga gguaucuuua agaauccgga 2280
   cuacaugucc ccauauuucu ucccuuccuc ugauguuuaa gccguagagu gaguaugcaa 2340
   acugauugac uagaauaccc ccgaacguug agcaaaauaa aggaacauca aucaccauaa 2400
   acucaguaau caagcuguug acaucaucgu cauuuacuag ugcgaucauc ucuuuuaagu 2460
   ugucucuauc uaauugagac auuguaucag cuaucgucau ugcaguacag auacugguug 2520
   cucugauaac uucaucaucu gaccaauaag ucaugucaac uguguguaca acaucucuga 2580
   ccuugcuaaa uagcucaagg uccacauccu ugaguggauc aggaucauag aucaauuuau 2640
   uguucucuug uguaaucucu aaaucuaaug uggaccuugg ggggauauuc gccuccugug 2700
   gggacuccau uauacagcac ccguuauuaa gauguaagug caauaucagu ggcuucccua 2760
   aggaaccuuu cuuauaucuc auauugaacu cgaacaagcu uagcccaguu agcauaaucu 2820
   gcugauacac gagauuagua uccuucgacu ccccugcuuc uuugagugcc auguuaucau 2880
   uugauauugu uaugaaccga cuugcacgga cuaguguugc acuagagaac uucaucuggg 2940
   uugccguauc uuucaaccua ugagauagau uaguggaggu ugaaacagga gucagcagcu 3000
   uuagauucuc uaagcucaga uuagcucuug uuugggcuau aagagcggcu uccauccacg 3060
   auaucucauc agucccguag gcccacguau acaccauagc uauccggaug gccgccuuug 3120
   cggguuugcu uagauuucuu acauacccga guugggcuuc cgaccuuuca ucaguggcug 3180
   auccaaaaua ggggauucuu auagccggac auccguuugu aagcgugucc aggucuauag 3240
   agucaggaag auagaaccau guauagaugg ggucugcucc uucagaccug caaagcuugc 3300
   acaccucuga accuucgaua aauauucccc ucaagagcuc uaaaggucuu gguguuucua 3360
   gcccauguau gggucucccg uaagucaggu ggauccacau uuucugccuu agaccgacag 3420
   cuagcucaac ugaacacaua uacucauauu cgauguuguc uuucaccggu uuccugagag 3480
   uucuagucag ugucucguac ugcaauagau cauaauugac aagccuccuc aauaucccau 3540
   augauaaucc uccuuuccua acgcuggcuc ucacuagaga cuuggucgua ucaagcaucc 3600
   cugcaaucgc cucccuaacu ccaguuaagg aauuacccag gaucucauga gccacucucg 3660
   gcaggaugac uuuccggucc auaaggaacg aggccagguu gagauccucu ucuccacuag 3720
   ucucggugaa gagaccagac aguagaggau ucggggauuc cugcagcaca gaucuagcag 3780
   ugauauucuu uauaaucgua guuauacucu gagaaugcgg gagguuacac gaauaagggu 3840
   cugaagccca aucuagaaaa cuagagucac cggguucuug auucaugacc cuguauaaua 3900
   ccugcuuguc uaacagaucc gcucugauga aucuuuugag aucagcuagg gcugcuacug 3960
   cggggucucc aauauuucua acaaagcauc uagauguaga cauguaguug aauccuccaa 4020
   cauuugcugg aaucaacacu gcacaucuca gccaauucuu acccuuaaag uauugaucuc 4080
   uuacggucgg gcugauaguu ggauuuauag ucaucccuag ugauaugcac accugcugac 4140
   aggucuuaua caacgcaaug caguagccua guauaggaga auacccauuu ucgauagcuu 4200
   uugcuaugga uguugagaug uucgaacaag cagaucuguu uucaucuacc agugucucgg 4260
   accagaauac acacuugguc aaggcuuuca ggcacugugg uaaaaucuuc ccaucauagu 4320
   auauccuuuu acuauagaca aacaucuugc uacuaaugau ggucucguuc aauuuuagcu 4380
   cgugcccuac aucaaacaug acgugucuua gagcaccgaa auauuuggug aucuccucau 4440
   agacaugauu uuucuucugc uuguaagucu gagcuacagg uacucuugau gucacggcua 4500
   uagcuugauu gucacccuga accauugcag agacccugac acccacucuc acagcugcua 4560
   gguggauugc acugauugag auuaaggucc acagcuucug gcaguaaccu ucuaugcccc 4620
   cccuaggauu auguaugaaa augccagagu cugcaugauc cuggaguugu cgaugcaucc 4680
   ggucggcgac uggacaguaa ggaucuccaa cauauauugu acaccuuuca aggacuggau 4740
   gcauccaguu aaagaagguc uugaagccaa auaucucguu gcaucucuga ccaaacaaug 4800
   caguacucuc aaaucuccag uuuaagcagu auuucuugag gucuguugug aggaagcaac 4860
   uuaacguuuc auagccgucu guugaugaau cuguugccuu gaauucaugu cuggaccucu 4920
   ucuuuucguc ccaguacccc ccagaguucu uauuuuccau gccuucguuu cucuucucug 4980
   augauuuaga guuauuguac acugaaucag uccuggggac gccugagaca gaaagaguag 5040
   ucaaucuuuu aaguaggucu aucucuccuu uaaccauccc auuuucgcug aauagcucuc 5100
   cuauuccuuu agccaguagu gucucugcca gcaccuguac ggcucgcauc uuauaaguca 5160
   uuuuugcgaa uagacgaccc ucuugcuuga ucucuuucuc uuugagacug uacgagaugu 5220
   ugaacuccuc gucuuucaac caaucuccug acuccacaua auugauaauu ucuucugggu 5280
   ugaaauucuc aucauuuaug aacacuucaa uaagccgccg ggucucuuca gacucugggg 5340
   cuuuauagua cagauuacua uccggguaua cagaguccca ugccuccuuc cugggggaua 5400
   gugcuuuguc uuucauauau auugugagau cuucaucuag uugugguucu auaaacuucc 5460
   gaaacuugaa gccuaugaaa cuuguauagu ugucuacagc acauucauaa gagauugccg 5520
   uauuggaccc uugagcguuc cuuaguucua gacacacgug aucagggaag ucacaggggg 5580
   gccacugucc gccaugccuc ucucuauacc cauuuaugau gauagugcaa aaaacugcau 5640
   gacacucgua uagggucuua agcuuuauug ccuuuugugc auacauaugg gcccuuaccu 5700
   ugucggcggc agugacagcc ucuaagcugg gguggccaaa uguccuaaag aaggaaaaga 5760
   ucucugcuuu cucaucaaua gagguuccau ggaaaauggc gaguaacgac uccacaauag 5820
   ugucugcuuc agcaucugug uacacgucuc uacuuguuaa aacagucugu agcucuguca 5880
   acacaugccu cauaaaugcc ccacguagag guauaacagg aucauuuagu uguaugagag 5940
   caagugauag gggcuccaau agugcgauga cauuguauau uuccucucca agacuugaga 6000
   agagggaauc cacuaguucc cauaauuccu caccuuugcu uguuauccca auggacuucu 6060
   uaucuagaug cccugcagca gacauauucc accuuccuuc uacaacauca caauacauca 6120
   agaccagcuc agggguuagg auauacccug ucaaugucaa cuuguucagu aucaugacaa 6180
   gaucuccgua uguuacuaga guguaugauu ugcauucuag gagguuaugu gaauuagagg 6240
   uaucgagggg uccccccggu cuggucuucu gcauccaccg caugucauau uugaugcuga 6300
   accaaguuag gaauggccua uaccaucuau uccugcugua cuuaucaguu aucuccggga 6360
   uggugccgau auccugcaac ggaucguacc ccucucuucc uucuauauug ccuaguugcu 6420
   ugaagauauu caaccaaaga uccuggaacc cacuagauaa cucccugguc agccgauccg 6480
   agaccgcgaa gacggaucgg auuuugucac auaucucugg uauaucaagc cuaaguaauu 6540
   ccugagagua gguuggguac gguucaaacg uguaucgguc uaaauccuuu auugugcguu 6600
   gaagagccuu acccagagac cugaucuuaa uuugacgggg ggacaauccu ccguuccuaa 6660
   uuuugugcuu uguaauauuu auuaugcugu cguccuucag ucuguagggc ugguucacau 6720
   cuaacaagac gugcaacugu gcuaucuucc cccugacuau gggagaguuc agguggcauu 6780
   cuggauagag uaugucagaa ggguuuuggg aggacuccug cccauccaug accuauggca 6840
   agcuucccau ucacccuggg uuuuucuuaa uacugugaga gacguaagag acugagagau 6900
   auuguggaug uucggagaug acucuagugu cagcaaggcc gacaagccug cuagucaguu 6960
   aaauuuaaga cucggccuug cauaauuuag ggaugcuagu cuuaaagagc aucggcugua 7020
   agguauucag gcucuucuga uugaucucga ugaugugaaa gcaguagccu uuaccaaaau 7080
   gcgugauaca cgaugucgug guauaugcag ccucuaauug aacauccuuu auccuuaaca 7140
   uauuuauaau guuaguagug uuagaauaca ugauuguugg guugacacgc gauguauugg 7200
   cauauagcgu gacgguagcg acguuagcug caucagggga caauggauaa gcaucagugu 7260
   auacgccuga uaugcauucc uucggacacu uauuguacca auugcacucu ucauuuccug 7320
   gucuagacaa ggcuucauga gguguccagu ugauagucaa aggguggcug acaucaagua 7380
   cuccuaucug caguugagag ugccagccug augaucuugu auagauguac acccgaucac 7440
   ccaauuuuaa uaaucuaccu uccgccccga gauaguuuug agugauugga augguuguga 7500
   cucuuaucuu uggccucucu gagagauagu cauugaccug gaugaucacg cugaccaccu 7560
   guuucccucc uagccaugua auuuucagag ccucauugca ugugucuugc gacaccuguu 7620
   ggcauccuug gguccuacau uuuguaucac ccugcagagg ggugguuagu ccaccauacc 7680
   caagaaauau caaugagccu ucuguugcaa ugccguugcc uacacugggg uauagugcag 7740
   agaacgggug aucaagaucu accucgcugu ugcgauaccg gugagacuua guucucccuu 7800
   ugagauccag gacaucaagg accagauccu caauaccauc acuagaguag ucgguucuuu 7860
   cgucuacagu cggcauggag caaagcugau aaccccuagu ccggguuguc accacagagc 7920
   augauuuccg auugucguug augucauaag ugugggacac uacgggguua agaucaggga 7980
   acauaucuga auugagugau auguacccua gcugcaggac cugauaugau uucccuaugu 8040
   cagcacaacc uuguguaaug agauuugaug aauaggcaua gauugccucg ccaauugaga 8100
   gugaagggag ccuaacacau ccagagaucg uuguagaacc agauaacaag cucggaccag 8160
   gcagcaauga gauuucagga ucugagcuaa gauacgguuc uccgacaggg caucuccaga 8220
   aacuaugugg cucaaguggg gcaauuccau cggcauggug gacugcgauc guacucucac 8280
   agugcugagu gagcucuugu cugcugcacg acuuaucaau caucuggaug acaucccugc 8340
   uguuuuuguu caacaagacu gggauuccgg uuugcacaga gcucugaaug uugacagccc 8400
   uugcuauaac cucuugccuu auuagacugg uaagugacuc uuucaccucc cugcugcuca 8460
   uguucaaugc cucuacaguc auugaguacu cuuucauacu auacccuugu cuagcagaaa 8520
   uuaugauaca gaugaucacu guggcaauug acaaagccca cugggugaau gagagaauca 8580
   gcaaccaugu gucggcuuua cuugaccucu cccaaccuga ugcugguuuu guggugcuac 8640
   cacuaggaga aguagaccag uacgagucac guuugccccu aucaccaucc augaucugug 8700
   cuuguuugag gcggccgcga ugaacuuuca cccuaaguuu uucuuacuac gguuagcuuu 8760
   ucauuuugau caucauguau gcuucuaugc aguugaugaa gaugucaaau ucauucaugg 8820
   ccuuguagac accuuggucu uggagcuuau uaaaaucacu cuucaccugc uccacugccu 8880
   ugcucuuauu uucacagggg agaaaucgau gacagcgccu cagccgcauc cugagggucu 8940
   ucagcuucuc acccagggaa uucaaaugcu ccuugauuuc ugggccaugc uucucugccu 9000
   ggggcaucac uucuaccagg uaaaacugga ucauuuccga uaaggcuugg caacccaagu 9060
   aacccuuaaa guccugcauu aaggagucgg uuagcaguau guuguccagc ugguccuuug 9120
   uuugaaagaa agucuucacc uggcugaagg caguccgcag cucuaggagc auguggcucu 9180
   ggccgacugg gaagugggug caguuauugu cuucccggcu guacuggccc cugcugaucc 9240
   ucaugccagu caguaagagc aggcagcaua gcagugcuga gccaggcauu gaacuuuggc 9300
   ggccgcuuaa uuaaccaagc acucacaagg gacuuuaucc cuaaguuuuu cuuauuuaag 9360
   acaaggagug acccgguggc uccgacugcc aggugguguc ugcuuggggc auugucgcag 9420
   gucucugaug ggugcacauu uacagcuuuc ugauccuucc gauguucuua gccacaacau 9480
   uaggguagua gcggaaauca cgagggaugg ccgguuggaa caccgcaucg acgccuguga 9540
   uuucuacaga ugccgcccaa aucaccaugu ucauaugggg auucacaucc auuaauggga 9600
   agcagacugc ccucuuccau gcgagcugac ucaugaaugu cuuagauagu gucccauuaa 9660
   ccugaacaug gaagcuuaua ccgccgauua acccaaguga gaaaaucagc cgcauucucu 9720
   caaucuugcu cuugcaguac ucaacagagu auaucuuccc gaccuuucuc cugaucaacc 9780
   cgaggugcac cauaaaauug agcuuuuucu ccccuugauc aucaaguacu gggaguaccc 9840
   ccuuuuguuc uguggagauc ccggucuuga gugucaccag uaaauuaaca gauauagagu 9900
   ugggcaaugc aaggucugca agggucuuug ggaucuugga uauggugauu gccccuagag 9960
   augucccauu gacaaacacc acucugaguc uuauguccuu guccacaggg aggcauuggg 10020
   gagcuagugc gaccuuguuu gcauuaaaua ucauucccug ucucagccug ccugaccaug 10080
   guaggagugg agcaccaauc gaauccacca uguauacgau caucucuccu gcucgaacag 10140
   cccuccucac cguaauucug agaucggugc aggccuuuaa gaguuccuga ucagucccgu 10200
   aguauuuggc cacaccuaug gguaacgacc cggagccgca uauugaguag cuggucggcu 10260
   cugucaaguc agauacgcuc ucuagauugg uuguuuguuu cggugucuca aagaaaccca 10320
   agagcaauaa aucuagguau cucacuccau guuuaggagg gucuccuacc uugacaaucc 10380
   ugaugugggg gauggcuuuc uuauccggac caguucucag aggcaggggc uccacaguac 10440
   cguuauccuc auaugagaac uuagggaauc uauagauauc ugccauugcg ccguguuagg 10500
   ugaaauuucu uucacccuaa guuuuucuua aucuuuacug gcugaugcug augguagauu 10560
   ggaucucucu augacugagg augguaggau gccucacccg ggaucuaguu ggucagugac 10620
   ucuauguccu cuucuacgag uuccaugacu gccuuaaccu cuuggucugu cuugcacuug 10680
   gauaaugauu ucacauaugc uacuuucuca gcucugcuua ggggacugcu cucuaugacg 10740
   agccugagag agugcauugu gggcuucucu uuggagggaa agagacguga ugcguuugag 10800
   gcccuggguu cugugucccu cucuugguac accggguugc ggaucucauc ucuaaauuca 10860
   uccucucgga uuagguccgg uuuguacuuc auaucuucua gggucuccau agauggguca 10920
   aaccugguag ccuuagucuu guucucuuuu gauuuugcaa aaacggaggg ggaccuugua 10980
   agggagucug uguugucagu cuugccaccu cuaucuguga ugauaugaag uguagauagg 11040
   uuggacauca gcaaugaguu cuguucuuuc ugauacucag agaaucucuu guaaauaucc 11100
   cggaaugauu ccacgcucuc uuggaucugu uugagcaguu guuuguucuc aucuaccuua 11160
   cgagcggaag auuucucggc agaaaggauc aggccgcaua cauugaaugu caucucugca 11220
   uaguuugcag acuuuagggc acgucuugca aacacauaac ucgcgucucg ggaugauucg 11280
   aauucuugag cagacuggau uacaccaaga cucgucaaca auguagccau cucuuucaua 11340
   gaugaugugu ucucuccuau gcccuuuuuu guugagucgg ucucuagacc cggguggauu  11400
   gggcggccgu uggcuggaca aucugagaca gagcggcucc cuauuggugg uuuccggucu  11460
   uugacccuga cggcgggggu guccccagag uugaugugcu cauccugugu agaugggggu 11520
   uuuccuggug gugacccugu gcuguuguaa cgauucagcg guggggaccg ggugccaggc 11580
   acgguugcug gaguaagagg uuuggaccca cuguugguag gucuucuuuu guuccuccgu 11640
   agcacagccu cuucaaguuc ggggcuagga aucaccagga ccccaguuac ucuugcacua 11700
   ugugagcugc caggcuccau gcuucuucca uuauuacuug cuccaccuuc uccuucauca 11760
   gguagggaug uacuuccucg uaccucuucu ggaaguccuu cagcuugguc uucuccccuc 11820
   uuaucagggu gcgcagccau cucucuguuu ucaucuucaa uaccugaucu cggauauccu 11880
   cucucauuug gaggauuuuc aaggauuccg gagucuccuc caucgcccag auccugagau 11940
   acagaguuug uaccaguucu ucccccaaag gcccggugua uauuuuguuu aucaagguuu 12000
   ccagcaugug cuucugccuc uggcuugcuu guucucccag agacucuacu cuccucaccu 12060
   gaucgauuau cuugggucga cgguguugag acuucuccuu cgcccucacu uuuggcucua 12120
   ugagcagagc cugguccuug gggaguguug augguguugu ggagccagcu ucuguccccu 12180
   ccgaugucag uugguucacu cgacaggaca gcaucgagga auccgauaac auccgagagc 12240
   gacucucguc cuccuggcgc cucccucuca acuucagaau cuucuuuaag aaugaaggca 12300
   ucuugaucca ugcgguaagu guagccgaag ccguggcugu cucgacugcu gggugugguc 12360
   ggugggguug gguguggccu ugccugagcc gaucggugga ugaacuuuca cccuaaguuu 12420
   uucuuacuac ggaucaagua ccuugaagcc ucguaugauc cuagauuccu ccuaucccag 12480
   cuacugcugc ggcaucguca ucuucgucau gaucgacacc guuauugcgg ccuucaucuc 12540
   cauggguugc agaauccucu ugccgucucu cugcgagucu cauggcuauu cuucucucua 12600
   ugucugauac auccucauca uugguuuccu ccucuaaccg uucagcccca uguaguguga 12660
   caaaguggcc accacucacc ugacgugccc aucuuucacc acuaucucca ccccaacccc 12720
   uagcguccug guccgcaugg gcuuuuguuu cuaggucgau gucggcauug ucuagagcua 12780
   ccucaauugc accaccgccu guugguuugu gguaagcacc auccccaccg gacaaguuug 12840
   ccagaugaug ucugagccuc cccuuggcug uauccgucac uccuaacuca ucuuccaagg 12900
   cacugcugau cuucgauuca gcauccuuug ccacggcuug uccuaguaag aacauuucca 12960
   uaucaaggua uguccucccu gugacguacu gcugcauugc cuuguucugu acgacggcga 13020
   cucccauggc guaacuccau agugcaggau aauugccugg agcaaauuca ccaugaacag 13080
   gguccuugag gauacagaua aagggagcuc uggggccuuu ugacagguag gugucuauga 13140
   ggcuucuaag cuuauuaaua ucgggccuca gguuugacaa cguuagagcu gccaucuuug 13200
   uuuccacccc auauuuaaua guguucauga aggaagccag cccugcaucu cggauguagu 13260
   ucccaacgau cuggauguuc uucucuaaug uggugagauc agaucuugca guauucauag 13320
   ucacaagggu cucaaccaug agagauacaa ggcuuugcug agaucucaua accgagccua 13380
   uccccucaac ugucucccca gugaaaacua aggcaccuuu cacggugccg ucuugucuga 13440
   acgccucuaa ccuguugaag aacccuuucc uuaagccggc gcugcuugug auggccuuca 13500
   ccagcacaau ccagacuugg acaauuauug cuccuaggca ugcaggauac ccauagauuu 13560
   gaaggagugu gucagggucu gcagcauccc guugacccug gaagaguggg cucuuguuga 13620
   ccauaggucc aaacagccau ucuguggucc ucucauauuc cauaucucuc gucuucacaa 13680
   ugaauccguc ugucuucguc cucuuagggu cuuucucuau guuguagauc acauauuuga 13740
   caucggcguu uacuccguuu guugucaagu acaauucugg acuacuguaa gccauggcaa 13800
   gcagagagac gaggaacccc ccucucugag agugcugcuu aucugugucc aaugagugag 13860
   cuaggaaggu aguugcaaug aauaacuugu cugcaucauc agucacacuu gggccuagua 13920
   cgaacacuga gacugugcuc cucuggccgg ggauaacagc accuccuccc gacuuauuaa 13980
   uacuuucgcu ccuccuagag cuaaauguau cgaaggugcu caacaacccg gccaucguga 14040
   agaucugcgg ccgcgaugaa cuuucacccu aaguuuuucu uacuacgguu aagucgcuau 14100
   gacaacaccg cccaccauga guccaaugau ugcaccuuug uuugaaccca caucuucugc 14160
   aaagaacacc aauuuuugau gaugaacuuc auauccugag ucaugucgga auucugcauc 14220
   aagcgcccga gccguccagg cggccagcag gagcagugcc aaaccgggca gcauaaguga 14280
   accuuggcgg ccgcgugaac uuuggcagca aagcaaaggg ucuggaaccu gcuccucagg 14340
   guggauacuu ugacccuaaa auccuguaua acuucauuac auaucccaua cauguuuuuu 14400
   cucuuguuug gu 14412
<210> 6
   <211> 9
   <212> RNA
   <213> Artificial
<220>
   <223> a start (S) sequence of minus strand RNA virus
<400> 6
   uuucacccu 9
<210> 7
   <211> 9
   <212> RNA
   <213> Artificial
<220>
   <223> a start (S) sequence of minus strand RNA virus
<400> 7
   uuugacccu 9
<210> 8
   <211> 9
   <212> RNA
   <213> Artificial
<220>
   <223> a start (S) sequence of minus strand RNA virus
<400> 8
   auucacccu 9
<210> 9
   <211> 9
   <212> DNA
   <213> Artificial
<220>
   <223> a start (S) sequence of minus strand RNA virus
<400> 9
   tttcaccct 9
<210> 10
   <211> 9
   <212> DNA
   <213> Artificial
<220>
   <223> a start (S) sequence of minus strand RNA virus
<400> 10
   tttgaccct 9
<210> 11
   <211> 9
   <212> DNA
   <213> Artificial
<220>
   <223> a start (S) sequence of minus strand RNA virus
<400> 11
   attcaccct 9
<210> 12
   <211> 15
   <212> DNA
   <213> Artificial
<220>
   <223> an end (E) sequence of minus strand RNA virus
<400> 12
   tttttcttac tacgg 15
<210> 13
   <211> 18
   <212> DNA
   <213> Artificial
<220>
   <223> a spacer sequence
<400> 13
   cggccgcaga tcttcacg 18
<210> 14
   <211> 18
   <212> DNA
   <213> Artificial
<220>
   <223> a spacer sequence
<400> 14
   atgcatgccg gcagatga 18
<210> 15
   <211> 18
   <212> DNA
   <213> Artificial
<220>
   <223> a primer for amplifing a Sendai virus genomic fragment
<400> 15
   gttgagtact gcaagagc 18
<210> 16
   <211> 42
   <212> DNA
   <213> Artificial
<220>
   <223> a primer for amplifing a Sendai virus genomic fragment
<400> 16
   tttgccggca tgcatgtttc ccaaggggag agttttgcaa cc 42
<210> 17
   <211> 18
   <212> DNA,
   <213> Artificial
<220>
   <223> a primer for amplifing a Sendai virus genomic fragment
<400> 17
   atgcatgccg gcagatga 18
<210> 18
   <211> 21
   <212> DNA
   <213> Artificial
<220>
   <223> a primer for amplifying a Sendai virus genomic fragment
<400> 18
   tgggtgaatg agagaatcag c 21
<210> 19
   <211> 50.
   <212> DNA
   <213> Artificial
<220>
   <223> An artificially synthesized primer sequence
<400> 19
   attgcggccg ccaaggttca cttatgctgc ccggtttggc actgctcctg 50
<210> 20
<211> 81
   <212> DNA
   <213> Artificial
<220>
   <223> An artificially synthesized primer sequence
<400> 20
   attgcggccg cgatgaactt tcaccctaag tttttcttac tacggttaag tcgctatgac 60
   aacaccgccc accatgagtc c 81
<210> 21
   <211> 248
   <212> DNA
   <213> Artificial
<220>
   <223> An artificially synthesized sequence
<400> 21 .
   gcggccgcca aggttcactt atgctgcccg gtttggcact gctcctgctg gccgcctgga 60
   cggctcgggc gcttgatgca gaattccgac atgactcagg atatgaagtt catcatcaaa 120
   aattggtgtt ctttgcagaa gatgtgggtt caaacaaagg tgcaatcatt ggactcatgg 180
   tgggcggtgt tgtcatagcg acttaaccgt agtaagaaaa acttagggtg aaagttcatc 240
   gcggccgc 248
<210> 22
   <211> 51
   <212> DNA
   <213> Artificial
   <220>
   <223> An artificially synthesized primer sequence
<400> 22
   acttgcggcc gccaaagttc aatgcctggc tcagcactgc tatgctgcct g 51
<210> 23
   <211> 79
   <212> DNA
   <213> Artificial
<220>
   <223> An artificially synthesized primer sequence
<400> 23
   atccgcggcc gcgatgaact ttcaccctaa gtttttctta ctacggttag cttttcattt 60
   tgatcatcat gtatgcttc 79
<210> 24
   <211> 596
   <212> DNA
   <213> Artificial
<220>
   <223> An artificially synthesized sequence
<400> 24
   gcggccgcca aagttcaatg cctggctcag cactgctatg ctgcctgctc ttactgactg 60
   gcatgaggat cagcaggggc cagtacagcc gggaagacaa taactgcacc cacttcccag 120
   tcggccagag ccacatgctc ctagagctgc ggactgcctt cagccaggtg aagactttct 180
   ttcaaacaaa ggaccagctg gacaacatac tgctaaccga ctccttaatg caggacttta 240
   agggttactt gggttgccaa gccttatcgg aaatgatcca gttttacctg gtagaagtga 300
   tgccccaggc agagaagcat ggcccagaaa tcaaggagca tttgaattcc ctgggtgaga 360
   agctgaagac cctcaggatg cggctgaggc gctgtcatcg atttctcccc tgtgaaaata 420
   agagcaaggc agtggagcag gtgaagagtg attttaataa gctccaagac caaggtgtct 480
   acaaggccat gaatgaattt gacatcttca tcaactgcat agaagcatac atgatgatca 540
   aaatgaaaag ctaaccgtag taagaaaaac ttagggtgaa agttcatcgc ggccgc 596
<210> 25
   <211> 35
   <212> DNA
   <213> Artificial
<220>
   <223> An artificially synthesized primer sequence
<400> 25
   aagcggccgc ctcaaacaag cacagatcat ggatg 35
<210> 26
   <211> 34
   <212> DNA
   <213> Artificial
<220>
   <223> An artificially synthesized primer sequence
<400> 26
   aggcggccgc ttaattaacc aagcactcac aagg 34
<210> 27
   <211> 43
   <212> PRT
   <213> Homo sapiens
<400> 27
   Asp Ala Glu Phe Arg His Asp Ser Gly Tyr Glu Val His His Gln Lys
   1 5 10 15
   Leu Val Phe Phe Ala Glu Asp Val Gly Ser Asn Lys Gly Ala Ile Ile
   20 25 30
   Gly Leu Met Val Gly Gly Val Val Ile Ala Thr
   35 40
<210> 28
   <211> 61
   <212> PRT
   <213> artificial
<220>
   <223> a polypeptide containing an A-beta fragmnet
<400> 28
   Met Leu Pro Gly Leu Ala Leu Leu Leu Leu Ala Ala Trp Thr Ala Arg
   1 5 10 15
   Ala Leu Asp Ala Glu Phe Arg His Asp Ser Gly Tyr Glu Val His His
   20 25 30
   Gln Lys Leu Val Phe Phe Ala Glu Asp Val Gly Ser Asn Lys Gly Ala
   35 40 45
   Ile Ile Gly Leu Met Val Gly Gly Val Val Ile Ala Thr
   50 55 60
<210> 29
   <211> 35
   <212> DNA
   <213> Artificial
<220>
   <223> An artificially synthesized primer sequence
<400> 29
   tgcttggtta attaagcttt cacctcaaac aagca 35
<210> 30
   <211> 35
   <212> DNA
   <213> Artificial
<220>
   <223> An artificially synthesized primer sequence
<400> 30
   tgcttggtta attaagcggc cgcctcaaac aagca 35